# EUROPEAN PATENT APPLICATION

(11) **EP 2 939 624 A2**
(43) Date of publication of application: **04.11.2015**
(21) Application number: 13869209.0
(22) Date of filing: 31.12.2013
(51) Int. Cl.: A61B 18/00, A01G 7/04, C02F 1/00

(54) **APPLICATIONS OF DRIFT AND SUSPENSION DEVICE**

(30) Priority: 31.12.2012 CN 201210590662
(71) Applicant: Lanzhou Jinfule Biotechnology Co., Ltd., Lanzhou, Gansu 730030 (CN); Kin Star International Limited, Road Town, Virgin Islands (VG); Spring Power Limited, Road Town, British Virgin Islands (VG); Jin Jin Pacifique Compagnie, 92160 Antony (FR)
(72) Inventor: JIN, Jifan, Lanzhou Gansu 730030 (CN)
(74) Representative: Sun, Yiming
(86) International application number: PCT/CN2013/001673
(87) International publication number: WO 2014/101303

(57) **Abstract**

Applications of a suspension device in human bodies, animals, and plants, which are specifically: A) applications of a magnetic suspension device in human bodies and animals; B) applications of a drift and suspension device in plantation of plants; and C) applications of a drift and suspension device in seawater/sewage treatment. Through the applications of the device, a "hypomagnetic" environment can be provided for human bodies, animals, and plants, and the human bodies, animals, and plants can grow healthily in the "hypomagnetic" environment.

## Description

### TECHNICAL FIELD

The present invention relates to the field of life engineering, and more particularly, relates to application of a drift and suspension device.

### BACKGROUND

### Magnetism and Life

Life can be the explained like this according to the existing theory: Life is a substance system, which is currently divided into two categories: one is the inorganic life such as, a photon, a computer and a star; and the other is an organic life such as a cell, an animal and a plant. The organic life is unique to the astral environment like the earth, uses water as the carrier, and has such capabilities as its own renewal, precision duplicate and moderate division. The organic life is the substance system which is irreversible but always keeps repeating or retaining these capabilities. According to human conventions, the organic life is referred to as the life. Common persons would find it simple to distinguish a living substance from a non-living substance. However, it is a hard problem to give a scientific definition for the life for thousands of years, and the problem has not been fully resolved now. In biology, a general scientific definition for the life given by the modern biology is like this: Life is a complex phenomenon represented by the living bodies, which covers self reproduction, growth and development, metabolism, genetic variation and response to stimuli. In molecular biology, life is a molecular system constituted by such substances as nucleic acids and proteins, and has the abilities of continuously breeding and reacting to the external world. Other definitions: Life is a comprehensive running form that the organism constituted by biological macromolecules such as the nucleic acids and proteins continuously conducts exchange of substance, information and energy.

The magnetic field is a basic field in the nature, and is a part of an electromagnetic field. The magnetic field is also a special substance which is intangible and invisible and has radiation features of wave particles. The magnetic fields exist around magnets. Two magnets interact with each other using a magnetic field as a medium. A particular substance exists in the current, moving electric charges, magnets or a surrounding space of changeable electric fields. The magnetism of the magnets originates from the current, and the current is the movement of charges. To sum up, the magnetic field is produced by moving electric charges or changing of the electric field.

The basic characteristic of the magnetic field is to apply an acting force to the moving charges. Specifically, the electricity-applied conductor suffers an acting force from the magnetic field in the magnetic field. The acting forces or torques of the magnetic field to the current, and of the magnetic field to the magnet are all derived from herein. According to the modern theory, the magnetic strength is a relativistic effect of an electric field force.

When an external magnetic field is applied to the substance, the interior of the magnetic substance is magnetized, and many tiny magnetic dipoles are produced. The magnetization strength measures the magnetization degree of the magnetized substance. The magnetic field of the magnetic substance can be calculated according to the magnetization strength of the magnetic substance. The energy input is desired to create a magnetic field. When the magnetic field is annihilation, the energy can be recycled. Therefore, the energy is regarded to store in the magnetic field.

### Type of Magnetic Field

A constant magnetic field is also referred to a static magnetic field; and an alternating magnetic field, a pulsating magnetic field, and a pulsed magnetic field belong to a dynamic magnetic field. The equal or almost equal magnetic field strength in each space of the magnetic field is referred to a homogeneous magnetic field; otherwise, the magnetic field is referred to an inhomogeneous magnetic field. The greater the distance away from the surface of the magnetic pole, the weaker the magnetic field, with the magnetic field strength varying in a gradient pattern.
1. The constant magnetic field refers to that strength and direction of the magnetic field remain unchangeable that is called a constant magnetic field or a static magnetic field, for example, the magnetic field is generated by ferromagnetic plates and the electromagnet with DC.
2. The alternating magnetic field refers to that strength and direction of the magnetic field are regularly changed in the magnetic field, such as the magnetic field generated by an industry frequency magnetic therapy machine and a heteropolar rotating magnetic therapy instrument.
3. The pulsating magnetic field refers to that magnetic field strength is regularly changed while the magnetic field direction is not changed, for example: the magnetic field generated by a homopolar rotating magnetotherapy through the pulsating DC electromagnet.
4. The pulsed magnetic field refers to that a blocking oscillator is used to generate intermittent pulse current, and the electrical current is input into the solenoid coil to generate a pulsed magnetic field in various shapes. The pulsed magnetic field is characterized by intermittent emergence of the magnetic field, and the change in frequency, waveform and peak can be adjusted as needed.

Due to the complexity of life, life has no an accurate definition. At present, we can only employ the complexity of life to define the life. Then, concerning recognition of the life, origin of the life, essence of the life and the like, discussion is given hereinafter.

We consider that the running of the life is totally constituted by three levels. The mental level: the existence of life as the main control (the interaction between different magnetic fields forms different but relatively stable structures, and the structures forms a relatively stable living body, then the corresponding pathways are formed under the action of the corresponding magnetic field, run on the basis of the structure). Energy level serves as substance running activities of the life; the biomagnetic field in the inner side of the life serves as a running path of life information (spiritual energy). These three levels are interdependent and mutually serve as a premise, and seamlessly form a complete life existing system. A life being, large to the whole life (higher plants and animals), small to a cell, lower organisms, and molecules and atoms all has the common characteristics of the above three levels. Taking the human bodies, animals or plants involved in the health field as integral living bodies, the external environment of these living bodies, such as, the universal magnetic field, the geomagnetic field, existence of the magnetic fields caused by various appliances and the like human factors, is called an external magnetic state of the living bodies. The relatively stable magnetic field structure composing the living body structure according to certain procedures and relatively stable magnetic pathway formed by the interaction of the magnetic field are called an internal magnetic state (specifically, as a complete living body , life structure is made of various molecules or atoms through interaction of each magnetic field according to certain procedures; the substance basis of living bodies is formed by the action between magnetic fields controlled by certain procedures, the action between magnetic fields forms a relatively stable magnetic field pathway, and the energy, substance and information of the living bodies are run through magnetic field pathway). The internal magnetic state of the living bodies forms various biomagnetic fields which ensure the existence of effective control in the spirit level of the life. The internal magnetic state is used as a relatively independent (or closed) biomagnetic field, to maintain a relatively stable internal magnetic state structure and a relatively stable and flow of the magnetic field pathway of the internal magnetic state, the internal magnetic state not only needs power supply from external magnetic state (the outside world), but also conducts substance exchange and information exchange with the external magnetic state (the external world), and the like. Meanwhile the biomagnetic field of the internal magnetic state is also affected by the magnetic field of the external magnetic state, that is, the action of the magnetic field of the external magnetic should be smooth on the basis of keeping the inherent magnetic field structure of the internal magnetic state.

Taking the human bodies, animals or plants involved in the health field as integral living bodies, a healthy and prosperous life and continuous running of the life are totally dependent on whether the biomagnetic field on the basis of the internal magnetic state is capable of normally and stably performing a field quantity operation, i.e., ensuring stable and smooth running of the internal magnetic state pathway. Each part of living body produces weak but the strength-varying bimagnetic field in different during life activities. At present, the most researchable magnetic field is human body magnetic field, and the magnetic flux density B of the human body is generally between 10-13-10-8 T, far below that of the earth's magnetic field (approximately 10-5T). The magnetic field B and frequency f of some human tissues, and the earth magnetic field and other environmental magnetic field B and frequency f are listed in Table 1.

**Table 1 Magnetic fields of human body tissues, ground magnetic field, and other environment magnetic fields B*, and frequencies f thereof**

| Magnetic field of human body tissue | | | Geomagnetic field and other environment magnetic field | | |
|---|---|---|---|---|---|
| Normal heart magnetic field | ≤10⁻¹⁰ | 0.1-0.4 | Basic geomagnetic field | ≤5 x 10⁻⁵ | 0 |
| Cardiac magnetic field | ≤5×10⁻¹¹ | 0 | Rock abnormal magnetic field | About 10⁻⁴ | 0 |
| Normal brain magnetic field (α rhythm) | ≤5×10⁻¹³ | Alter nating | Magnetic storm changeable magnetic field | 5 x 10⁻⁹-10⁻⁷ | 0.1-100 |
| Normal brain magnetic field (in sleep) | ≤5×10⁻¹² | Alter nating | Electromagnetic interference magnetic field in cities | 5 x 10⁻⁷ | 0.1-100 |
| Eye magnetic field | 10⁻¹³-10^{- 12} | | | | |
| Skeletal muscle magnetic field | ≤ 10⁻¹¹ | 1-100 | | | |
| Abdomen magnetic field | ≤ 10⁻¹⁰ | 0 | | | |
| Asbestos miner's lung magnetic field | 5 x 10⁻⁸ | 0 | | | |

| | | | | | |
|---|---|---|---|---|---|
| * Used unit of the magnetic field: 1GS=10-4T, 1γ=10-9T=InT. | | | | | |

The biomagnetic field of the internal magnetic state of living bodies can be detected using a variety of magnetic methods. For example, a 250 MHz NMR spectrometer is used to observe the NMR spectrum of the serum of normal people (36) and cancer patients (197), it can be found that a significant difference exists in the area ration Sα/Sβ between α spectrum line and β spectrum line(Table 2).

**Table 2 Sα/Sβ values of tumor patients, other patients, and normal people**

| Disease | Cases (people) | S_{α}/S_{β} | Disease | Cases (people) | S_{α}/S_{β} |
|---|---|---|---|---|---|
| Normal people (comparison) | 36 | 1.85±0.06 | Leukaemia | 4 | 2.36 |
| Cervical cancer | 23 | 2.53±0.14 | Ovarian cancer | 4 | 2.04 |
| Lung cancer | 21 | 2.52±0.12 | Fibrosarcoma | 2 | 2.17 |
| Stomach cancer | 19 | 2.25±0.10 | Thyroid cancer | 2 | 2.03 |
| Esophagus cancer | 16 | 2.35±0.11 | Cervicitis | 3 | 2.15 |
| Intestinal cancer | 14 | 2.40±0.17 | Hepatitis | 3 | 2.33 |
| Mammary cancer | 13 | 2.43±0.15 | Hysteromyoma | 3 | 3.19 |
| Liver cancer | 9 | 2.52±0.15 | Lymphadenopathy | 2 | 2.32 |

Another method for judging cancer lesion from the nuclear magnetic resonance (NMR) spectrum is to compare ratio between α spectrum line height h and the width Δf (h/Δf). From observing of line width Δf of α spectrum line of the NMR of 460 cancer patients' serum and spin - spin relaxation time T2 = 1/Δf, it can be seen that T2 values of normal people and cancer patients have a significant difference (Table 3) in the operating frequencies of 90 MHz and 250 MHz.

**Table 3 T2 values of serums α of normal people and cancer patients**

| Nuclear magnetic resonance frequency | 90 MHz | | | 250 MHz | | |
|---|---|---|---|---|---|---|
| Health condition | Normal people | Leukemia patient | Stomach cancer patient | Normal people | Leukemia patient | Stomach cancer patient |
| T₂ (average) (ms) | 82±4.7 | 112±13. 5 | 110 | 30 | 39.8 | 39.7 |

A 100 MHz pulsed nuclear magnetic resonance spectrometer with the superconducting magnetic field is used to conduct a comparison study between tumor tissues of 106 tumor patients and normal tissue of autopsy cases without disease on whole body, and it is found that proton spin - crystal lattice relaxation time T1 of all studied tumor tissues significantly increase over the corresponding normal tissues T1 (in Table 4). This method has been prepared as a supplementary method for diagnosis of malignant tumor. The NMR imaging method described above can also be applied to the diagnosis of certain diseases.

**Table 4 Comparison of spin - crystal lattice relaxation time T1 of tumor tissues and normal tissues**

| Tissue | Spin - crystal lattice relaxation time T1 | | Variation range of T₁ | |
|---|---|---|---|---|
| | Tumor tissue | Normal tissue | Tumor tissue | Normal tissue |
| Mammary gland | 1.080±0.08 | 0.367±0.079 | 0.644-1.561 | 0.281-0.676 |
| Skin | 1.017±0.108 | 0.616±0.019 | 0.830-1.236 | 0.539-0.696 |
| Muscle | 1.413±0.082 | 1.023±0.029 | 1.030-1.645 | 0.773-1.196 |
| Stomach | 1.233±0.109 | 0.765±0.075 | 1.025-1.387 | 0.455-1.083 |
| Intestine | 1.122±0.04 | 0.641±0.08 | 0.737-1.363 | 0.368-1.118 |
| Lung | 1.110±0.057 | 0.788±0.076 | 0.847-1.502 | 0.537-0.870 |
| Lymph | 1.004±0.056 | 0.720±0.076 | 0.563-1.305 | 0.363-0.876 |
| Bone | 1.027±0.152 | 0.554±0.027 | 0.763-1.623 | 0.351-0.686 |
| Bladder | 1.241±0.165 | 0.891±0.061 | 1.010-1.50 | 0.711-0.978 |

Therefore, when other technical methods such as a nuclear magnetic resonance or the like are used to detect and analyze the biomagnetic field of the normal tissue and pathologically changed tissue, significant difference results can be obtained. Specifically, abnormal running of the biomagnetic field of a patient can be clearly detected, and the abnormal presents that the running of the magnetic field is disorder, the magnetic field pathway of the internal magnetic state is hindered at different levels, and even seriously, blocked phenomenon emerges.

**Table 5 Magnetic fields of organs of human bodies**

| Magnetic fields of organs of human bodies | |
|---|---|
| Organ | Magnetic field strength |
| Lung | 10⁻⁷-10⁻⁹ |
| Abdomen | ≤10⁻¹⁰ |
| Heart | 10⁻¹⁰-10⁻¹¹ |
| Skeletal muscle | 10⁻¹¹ |
| Eye | 10⁻¹²-10⁻¹³ |
| Brain | 10⁻¹²-10⁻¹⁴ |

It can be seen from Table 5 that the biological magnetic field of human organs is far lower than the earth magnetic field, and cannot be detected. At present, a fluxgate magnetometer (gradiometer) is mainly used to detect, and the resolution reaches to 10-10-10-11 T; a superconducting quantum interference device (SQUID) is also used to detect, and the resolution reaches to 10-15 T.

It is analyzed that the biological magnetic field of such organs as lungs, hearts, brains, intestines which get pathological changes will apparently show different features with the biological magnetic field of normal healthy human organs. The biological magnetic field of the normal healthy human organs is relatively smooth, that is, the magnetic field pathway of an internal magnetic state is required to be smooth, stable and orderly during the normal running (each organ of human body also forms the corresponding internal magnetic state of the organ, and forms the corresponding magnetic field pathway of the internal magnetic state in an inner side of the organ; cells constituting organs have the corresponding internal magnetic state forming the cell, and forms the corresponding magnetic field pathway of the internal magnetic state in an inner side of the cell). Therefore, to ensure the smoothness, stability and orderliness of the biological magnetic field pathway of the internal magnetic state is a necessary condition of remaining healthy, normal running for the living bodies, such as, the human body, and animal and plant bodies. At the same time, the running of the internal magnetic state must keep a harmonious and supplementary dynamic state with the magnetic field of the external magnetic state (such as, the earth magnetic field, the universal magnetic field, and the surrounding environment). Specifically, that is the "Nature and Humanity" ideology in Chinese traditional philosophy thinking; but the internal magnetic state of such living bodies as human body, and animal and plant bodies takes a long time process of adapting and harmonizing with the external magnetic state (such as, the earth magnetic field, the universal magnetic field, and the surrounding environment); and now the internal magnetic state possesses a complete, supplementary, and harmonious life instinct with the external magnetic state (the meaning of each other is that the external magnetic state affects the internal magnetic state, then the internal magnetic state adjusts and restores itself, and gradually adapts and evolves, and finally forms life instinct with the external magnetic state, but meanwhile the internal magnetic state will change and affect to the external magnetic state for the purpose of existing). Complementary blending between the internal magnetic state and the external magnetic state is innate, but the mutual interference and damage between each other always exist such as: the interference and damage are realized as natural disaster, abrupt climate change, and environmental pollution; therefore, during the interaction of the internal magnetic state and the external magnetic state, the contradiction between each other from the confrontation to relative adaptation, transformation with each other and then to mutual tolerance, thus the living conditions of such living bodies as human body, animals and plants are formed; meanwhile there is also the interaction of biomagnetic field between a living body, for example, through the interaction of with climate, the human beings gradually adapt to climate via increasing clothing and heating; through changing the surrounding environment, the human beings affect the climate (for supplement to the external magnetic state, the external magnetic state is a relative concept to the internal magnetic state, and is also the magnetic state generating action with the internal magnetic state through a magnetic field; generally speaking, the external magnetic state is the factor affecting the internal magnetic state or the presence of living bodies, for example, the external magnetic state may be all factors: the earth magnetic field, the universal magnetic field, the solar radiation, the climate, the air, houses that act with the magnetic field). The influences of the external magnetic state to the internal magnetic state are as follows: 1. affecting the exchanges with substance, information and energy between the internal magnetic state and the external magnetic state, this kind of exchange is conducted under the spiritual control of the internal magnetic state; in this case, the magnetic field of the external magnetic state is relatively weak, such as, people acquire knowledge by reading, acquire information through mutual exchanging, acquire energy through diet, and the iron and steel gets magnetism after magnetization; 2. affecting the biomagnetic field (biomagnetic field path) of the internal magnetic state to reversible (recoverable), this kind of reversible (recoverable) process is conducted under the spiritual control of the internal magnetic state; in this case, the magnetic field of the external magnetic state is relatively strong; 3. or affecting the biomagnetic field (biomagnetic field path) of the internal magnetic state to irreversible (unrecoverable), but the influence can be restored by using a certain way and method, can not be restored simply by the strength of the internal magnetic state, and can be conducted under assistance of external force (hypermagnetic state) with the spiritual control of the internal magnetic state; in this case, the magnetic field of the external magnetic state is relatively strong; 4. or affecting the biomagnetic field (biomagnetic field path) of the internal magnetic state to irreversible (unrecoverable), and such another internal magnetic state is formed, that is, the former structure is changed; another substance appears in the spirit level; in this case, the magnetic field of the external magnetic state is very strong.

The above is only classification on the influence. Essentially, in the process of the interaction between the magnetic field of the internal magnetic state and the magnetic field of the external magnetic state, a variety of the above situations may happen or happen at the same time. For example, the celestial body that collided with the Galaxy 100 million years ago may be a smaller galaxy or a larger dark matter structure. Although scientists have no idea to confirm the culprit of the collision, they can assure you that the constellations with wrong position and motion will return to equilibrium within the next 100 million years. In this example, the Galaxy is in the internal magnetic state, while the celestial body that collided with the Galaxy is in the external magnetic state. After the collision occurs, a part of the Galaxy (the part's internal magnetic state) changes reversibly with a self-healing effect. The part can carry out self-healing under the spiritual control of the internal magnetic state of the whole Galaxy (The small individual galaxies that form the Galaxy have their stable positions and states, which are controlled spiritually by the internal magnetic state of the whole Galaxy). Another part of the Galaxy (the part's internal magnetic state) is affected irreversibly or cannot self-heal, and forms a new stable structure, i.e. a new internal magnetic state and a magnetic field pathway formed under the spiritual control of the internal magnetic state of the Galaxy. In the process of the collision, there is exchange of information, matter and energy between the internal and external magnetic states. For example, when plants or animals (the internal magnetic state) are infected by external bacteria or virus (the external magnetic state), if plants or animals (the internal magnetic state) have resistance or antibody, i.e. the biomagnetic field pathway of the internal magnetic state is very smooth and the biomagnetic field of the internal magnetic state is stronger than that of the external magnetic field, impact only happens on matter, energy and information between the internal magnetic state and the external magnetic state, the impact on the magnetic field pathway of the internal magnetic state is smaller (the bacteria or virus entering plants or animals is quickly killed by antibodies or immune cells, and excreted). Under the condition of the lower resistance or weaker antibody to the animal and plant (internal magnetic state), i.e. the biomagnetic field of the internal magnetic state is weaker than that of the external magnetic field, the biomagnetic field of the internal magnetic state is influenced by the biomagnetic field of the external magnetic state, causing the biomagnetic field of the internal magnetic state (biomagnetic field pathway) has a reversible (recoverable) impact, such as some pathological changes on plants and animals. However, the symptoms can self-recover and recuperate under the spiritual control of the internal magnetic state of plants and animals (the bacteria or virus entering the bodies of plants and animals will struggle with plants and animals for a while, and then is killed and excreted). Under the condition of the much lower resistance or much weaker antibody to the animal and plant (inner magnetic state), i.e. the biomagnetic field of the internal magnetic state is quite weaker than that of the external magnetic field, causing the biomagnetic field of the internal magnetic state (biomagnetic field pathway) is influenced irreversibly (unrecoverable), certain methods and ways should be used to restore the impact, such as drug treatment, food treatment and other physical and chemical treatment (those methods and ways assisting the internal magnetic state to self-recover can be called hypermagnetic state, which can not only help the internal magnetic state with no self-recovering ability due to the influence from the magnetic field of the external magnetic state recover, but also enable the internal magnetic state with self-recovering ability recover more easily). As for the recovery which cannot be realized purely by the internal magnetic state of plants and animals itself, it is done with the assistance of the spirit of the internal magnetic state as well as the external force (hypermagnetic state). For example, some infectious diseases can be healed with drug treatment. However, if the magnetic field of the external magnetic state of bacteria and virus is much stronger than the biomagnetic field of the internal magnetic field of plants and animals, the biomagnetic field pathway of the internal magnetic field of plants and animals will be totally separated, cut off and destroyed. In that way, the introduction of the hypermagnetic state will make no effort on the recovery.

The environment where the internal magnetic state of plants and animals exists is always under the influence of the magnetic field of the external magnetic state. It is reported that some kinds of bacteria have the ability to swim northward along the direction of the earth's magnetic field. The phenomenon can be called magnetotropism, which enables bacteria to feel the direction of the earth's magnetic field, so as to compensate for the lack of ability to perceive gravity. Scientists also find magnetotropistic particles in the pigeon's head and the bee's abdomen, which is related to the geomagnetic navigation mechanism. Experiment shows that ciliate in the magnetic field at about 0.14 mT will change its direction of movement in accordance with the direction of the magnetic field after a few seconds. A series of observations indicate that, as the relationship between the permeability of the biomembrane and the earth's magnetic field is ubiquitous, the physiological rhythms of organism (human) often coordinate and synchronize with the changes of the geomagnetic field.

Is human's physiological and pathological state also affected by the geomagnetic field? A statistic study on the relation between 67 strong magnetic storms and the mortality, morbidity and neurological disorder occurred in five years in a German city finds that human's physiological and pathological state has relatively relevance with the geomagnetic field. A one-year study on 43 volunteers in Puerto Rico in Central America finds that there was a temporal correlation between the blood pressure and the number of white blood cells of the volunteers and the fluctuations of the geomagnetic field in the region. Another statistic study on the number of patients with acute heart disease taken to major hospitals in two Indian cities and the daily activities of the geomagnetic field (KP Index) between 1967 and 1972 shows that human's physiological and pathological state has apparent relevance with the geomagnetic field. The study also shows that acute heart disease and the changes of the geomagnetic field may be affected by the solar wind (charged particles flowing from the sun).

From the above studies, we can conclude that the influence of the external magnetic state on the internal magnetic state is an objective existence all the time. Because of the objective existence and the variety of the influence, the influence on the internal magnetic state is various and diverse. The spirit of the internal magnetic state always regulates the internal magnetic state, and maintains its stability and the stable and regular operation of the biomagnetic field of the internal magnetic state. But if the influence is just normal influence from the external magnetic state, i.e. the influence of the external magnetic state does not exceed the scope of the internal magnetic state, or the threshold value of the influence of the external magnetic state is lower than that of the internal magnetic state, the influence of the external magnetic state on the internal magnetic state, under the spiritual control of the internal magnetic state, will enable the internal magnetic state to maintain a certain stable equilibrium. While the threshold value of the influence of the external magnetic state is higher than that of the internal magnetic state, the geomagnetic field will change in accordance with the influence of solar activities, and meanwhile, the magnetic pole of the geomagnetic field will become strong, weak or change direction over time. Besides, when the pollution of the environment where the internal magnetic state exists, various spiritual stress and unstable mood triggered by emergent events in life, such as fear, anxiety, worry and anger, are beyond a certain limit or the threshold value, great influence will take on the smoothness of the biomagnetic field pathway of the internal magnetic state. In that case, the entire structure of the internal magnetic state will be damaged, destroyed, changed, the smoothness of the biomagnetic field pathway of the internal magnetic state will also be separated, cut off and destroyed. And the spiritual control of the internal magnetic state will not enable troubled internal magnetic state (include the entire structure and the biomagnetic field pathway of the internal magnetic state) to recover structure and the smoothness of the biomagnetic field pathway. The influence of the external magnetic state on the internal magnetic state has a certain cumulative effect too. For example, if long-term fatigue and tiredness are not relieved through proper rest and recovery, the organism will age or age quickly. Although the influence of the external magnetic state on the internal magnetic state is lower than the threshold value of the magnetic field of the external magnetic state, if the speed and frequency of the influence exceed the speed and frequency of the spiritual control of the internal magnetic state, the entire structure of the internal magnetic state and the influence on the biomagnetic field pathway of the internal magnetic state will not recover in time, causing irreversible damage. In the process of interaction between the external magnetic state and the internal magnetic state, as time goes by and the external magnetic state has no recovering change, the internal magnetic state will keep on adapt itself to the external magnetic state under the spiritual regulation of the internal magnetic state after a certain period. The adaptation includes the structural change of the internal magnetic state influenced by the external magnetic state and adaptable change of the biomagnetic field pathway of the internal magnetic state under the spiritual regulation of the internal magnetic state. However, those changes are relative. If the speed and frequency of the influence of the external magnetic state return to the original state or are cancelled, the changes of the internal magnetic state will gradually recover to a relatively stable state under the spiritual regulation of the internal magnetic state. When the speed and frequency of the influence of the external magnetic state keep going, the changes of the internal magnetic state will happen based on the adaptation to the external magnetic state, enabling the structure of the internal magnetic state to change accordingly and irreversibly (or irrecoverably, including death) and enabling the biomagnetic field pathway of the internal magnetic state under the spiritual regulation of the internal magnetic state to change accordingly and irreversibly (or irrecoverably, including death). Besides maintaining its life's existence and continuation through self-regulation and recuperation on spirit level, the internal magnetic state needs to grow healthier, longevous and more vigorous. To realize these goals, spiritual self-regulation and recuperation of the organism is not enough, the assistance of the hypermagnetic state needs to be relied. We call the way and method in which the internal magnetic state does self-recovery hypermagnetic state, which can not only help some internal magnetic state unable to purely rely on itself to recover due to the influence by the magnetic field of the external magnetic state, to recover by itself, but also make the internal magnetic state enable to self-recover do self-recovering more easily. No matter which method or way of the hypermagnetic state to choose, including food treatment, medical treatment, physical treatment and psychological treatment, it is an influence of the biomagnetic field of the hypermagnetic state on the biomagnetic field of the internal magnetic state, or an influence to make the biomagnetic field pathway of the internal magnetic state smooth, so as to assist the spirit of the internal magnetic state to self-recover and recuperate.

As a basic unit cell for making up human bodies, animals and plants, its basic structure comprises cytomembrane, cytoplasm, cell nucleus, and the like., wherein main function of the cytomembrane is to create a stable state in a qualified sence, that is a stable and dynamic chemical component region to allow substance exchange and penetration in and out membrane through selective diffusion and transportation, effectively supplying, replenishing and eliminating partial wastes(metabolite), and the like, which is produced during energy transformation, energy required by cells is also produced therein, namely, operation exchange among biological magnetic energy, substances and information, biomembrane effectively controls operation and transmit rates of energy, substances and information in the internal biological magnetic field pathways, energetic magnetic state biological magnetic field can influence penetration capability of energy information(all kinds of ions) on biological membrane, and then it decides metabolism of cell biological magnetic field energy, in non-stop existence and development of cells, cells are constantly weakened and aged and their functions are decayed and then they are replaced by new cells, with operation and transmitting change of cytomembrane on energy, substances and information in the internal biological magnetic field, (such as gradually weakening).

As individual life (human bodies, animals and plants) grows and ages, cytoadherence among cells will take place, thus, space of intercellular substance among cells will be compressed, (wherein intercellular substance exists among cells, cells in the human tissue are impregnated therein. the intercellular substance supports, protects, bonds and nitrifies cells, takes part in the formation of microenvironment for cell survival. It is a liquid environment for human cell life and contains all of substances required by cellular metabolism. In the same way, the intercellular substance liquid also receives cellular metabolic products or unemployed substances. Interchange of material between cells and liquid is constantly performed: taking oxygen and nutriment, and eliminating wastes such as carbon dioxide). Cytomembranes among cells are bonded together, thus, pathways for operation and exchange of substances, energy and information between cells and intercellular substance are hindered, cut off and blocked, that is to say, bonding leads to hindering, cutting off and blocking of biological magnetic field pathways of internal magnetic state(cells), then resulting in hindering, cutting off and blocking of operation and exchange of substances, energy and information with external magnetic state(intercellular substance and other cells), thus, bonding among cytomembrane becomes tighter, hindering of biological magnetic field communication between the internal and external magnetic states becomes more and more serious, which causes lack on obtaining, using and circulating of energy that is supposed to be supplied and replenished, wastes produced from internal magnetic state energy transformation can not be sufficiently eliminated, thereby, accelerating cells ageing process, making human bodies, animals and plants fall ill, making circulation obstructed and damaged, life quality declined, reducing plant output, accelerating ageing, death, and the like.

To keep smooth and normal running of (internal magnetic state) biological magnetic field of cells during their life existence, hypomagnetic assistance is the must option. Hypomagnetic assistance to the internal magnetic state includes: (1) hypomagnetic in form of external magnetic state works on the internal magnetic state, (2) hypomagnetic in the form of substances, energy and information running in the internal magnetic state works on the internal magnetic state, and (3) hypomagnetic in the form of external magnetic state as well as substances, energy and information running in the internal magnetic state works on the internal magnetic state.

Hypomagnetic in the form of external magnetic state works on the internal magnetic state, mainly reflects as follows: putting a life body(internal magnetic state) from a changeable external magnetic state(various changeable environments during the life body running process) into another controllable external magnetic environment suitable for survival, growth, living, self-heal, and the like. of life body (internal magnetic state), which is widely researched by medical science field at present, such as various physical therapy methods and some assistant physical therapy methods from traditional Chinese medicine, physical therapy method is a method making artificial or natural physical factors work on human body to produce beneficial reactions, thereby to prevent and treat diseases, which is important content for rehabilitation therapy. Physical therapy can be divided into two kinds: artificial physical factors therapy and natural physical factors therapy.

Artificial physical factors therapy includes:
(1) Electrotherapy: comprises electrostatic therapy, direct current therapy, low frequency electrotherapy, medium frequency electrotherapy, high frequency electrotherapy, ultrahigh frequency electrotherapy, extra-high frequency electrotherapy, ion-introduction therapy, aeroionotherapy, hydroelectric bath therapy, radiofrequency therapy, and the like.
(2) Magnetotherapy: a method makes magnetic field work on a certain part or acupoint of human body to treat diseases, and comprises static magnetic field therapy, pulsed magnetic field therapy, low frequency magnetic field therapy, medium frequency magnetic field therapy, high frequency magnetic field therapy, and the like.
(3) Light therapy: a method uses sunlight or artificial light (infrared, ultraviolet ray, and the like.) for preventing and treating diseases to accelerate organism recovered, and comprises infrared therapy, visible light therapy, ultraviolet ray therapy, laser therapy, and the like.
(4) Ultrasound therapy: a method uses ultrasound for treating diseases, and comprises ultrasonic-static electrical theraphy and ultrasonic medicines penetration therapy.
(5) Conductive heat therapy: uses all kinds of heat sources as medium, makes heat directly conduct to human body to reach treatment, and comprises mud theraphy, paraffin wax theraphy, and jade theraphy.
(6) Yoga and Qigong theraphy
(7) Other theraphy, such as hydrotherapy, cryotherapy, kinesitherapy, cupping therapy, electronic biofeedback therapy, and the like.

Natural physical factors therapy comprises mineral spring, weather, air, sunlight and seawater therapy, and the like. Conventional natural physical factors include sunlight, air, seawater, mineral spring, high mountains, forest, and the like. When human body is in different natural environments, he receives comprehensive influences of different natural physical factors, to prevent diseases.

Chinese medicine physiotherapy, based on acupuncture, needling, moxibustion, medicine and somatic twisting, five supports of theory of traditional Chinese medicine, and guided by science of channels and collaterals, treats diseases of patients and makes full use of modern physical therapy facilities for partner treatment, yielding twice the result with half the effort.

Common features of the above-mentioned various physical therapy methods are: using all kinds of physical methods and ways to make the impaired organism healed by itself, make the damaged circulation function recovered, and make the hindered and blocked biological magnetic pathway of organism orderly and unblocked. Although using the above mentioned methods and ways reach remarkable effect, a large limitation on curing diseases, taking care of organism and prolong life still exists due to their working is on local part, which leads to the local part is recovered and its function is strengthened, while other parts (organ tissues) fail to gain the corresponding function strengthening, that is a cask effect on a whole; meanwhile, the interaction duration is long, during long period of time, change of organism itself and external influence on the organism are uncontrollable, as well as the key working point, and the curative effect fails to exhibit in a long working period under the circumstances of accumulative effect caused by rapid development of disease, acute onset, acute deadly infectious diseases and persistent pathogen; although, individual physical therapy works on the whole, the working process and key factors are failed to be effectively controlled.

Concerning the issues about how to make the treatment effect of physiotherapy more significant, more reliable and effective, and how to make the whole process more controllable, we need to be clear how various treatments functions.

The study has identified that a magnetic field having strength higher than 10 mT or an even ultrastrong magnetic field exerts its affects to most of living organisms, and when the strength, uniformity and action time of the magnetic field are different, the exerted effects vary. When the bacterium is placed in a uniform and constant magnetic field having strength greater than 1.4 T, it is found that such a magnetic field may inhibit the growth of the bacterium. The sea urchin egg is placed in a 10-14 T strong magnetic field of a superconducting magnet. Two hours later, it is observed that division thereof is subject to a notable delay. However, a uniform and constant magnetic field having strength higher than 0.125 T may promote the growth of oat. These examples show that under the action of the magnetic field having specific strength, different living organisms exhibit different effects. Since the different living organisms have different biomagnetic pathways in the biological internal magnetic state, when the external magnetic state of an externally applied magnetic field acts on the biomagnetic field in the internal magnetic field, a period of time later, if the biomagnetic field pathway in the internal magnetic state becomes more smooth and orderly, the living organisms exerts a promotion effect; otherwise, the living organism exerts an inhibition effect.

Fruit flies and mice are often used in biological tests. The fruit flies are raised for a long period of time in uniform and constant magnetic fields having different strength, and observation is conducted to see the distortions of the shape of the fruit flies caused by the magnetic fields. It is found that the distortions are not significant when the magnetic field strength is 0.01-0.15 T, whereas the distortion rate quickly increases when the magnetic field strength is increased to 0.3-0.4 T. This indicates that the biological effects of the magnetic fields have a critical magnetic field, which is referred to as a threshold field; and the effects are significant if the threshold field is exceeded. At this stage of life of fruit flies, the internal magnetic state and biomagnetic field pathway thereof have already been in a stable stage. Such a stable state is formed when the fruit flies are not in an external magnetic state environment with no externally applied magnetic field. When the fruit flies are raised for a long period of time in a uniform magnetic field, in its originally stable biomagnetic field (that is, the internal magnetic state structure and the biomagnetic field pathway), under the long-term action of the external magnetic field, the internal magnetic state structure and the biomagnetic field pathway may be subject to new stable structure and magnetic pathway. Therefore, significant distortions emerge. However, after the fruit fly eggs, larvae and pupae are placed in a strong magnetic field having strength of 10-14 T generated by a superconducting magnet for 1 to 2 hours for treatment, the growth and development, in, sex ratio and chromosome of the fruit flies are subject to no significant change. This indicates that the biological effects of magnetic fields are not only associated with the magnetic field strength, but also associated with the action time of the magnetic fields. At the stages of the eggs, pupae and larvae of fruit flies, the fruit flies have better magnetic field compatibility, and are simply intended to be compatible with the external magnetic state. Therefore, under such circumstances, the growth and development, sex ratio and chromosome are subjected to no significant change. However, if the fruit flies of different ages or instar pupal ages are placed in a non-uniform strong magnetic field having gradient of 0.9 T/mm and strength of 2.2 T, the fruit fly pupae at smaller ages die a few minutes later; and 10 minutes later, about 50% of the fruit fly pupae of different pupal ages become adults. However, even being adults, these fruit fly pupae never survive one hour. In addition, 5 to 10% adults are subject to severe abnormal wings, and shape distortion. If the fruit flies are placed in a non-uniform strong magnetic field having gradient of 0.7 T/cm and strength of 4.5 T, these fruit flies all die one hour later. These experiments show that non-uniformity degree of the magnetic field exerts significantly remarkable effects on the living organisms. Since the fruit flies are the magnetic field in such an external magnetic state, different positions of the bodies of the fruit flies are subject to different magnetic field strength, and even the same magnetic field pathway is subject to different strength of the external magnetic field. Therefore, orderliness and smoothness of the magnetic field pathways in different positions of the fruit fly bodies are not in synchronization, and the integrity of the living organisms is affected. As a result, results different from those in the magnetic fields may be present.

People, in a constant magnetic field of about 2T for 15 minutes, find no discomfort or injury effect. However, when people approach the strong magnetic field (about 1-2 T) of an accelerator, they fail to feel the direction only several minutes later, and such feeling disappears if they stays longer. When the people leave the strong magnetic field of the accelerator, they still have the feeling of finding no direction and walking unstably. This phenomenon indicates that a change in the magnetic field affects people's feeling in direction determination. Concerning how the direction determination is affected, when people are in a constant magnetic field, the biomagnetic pathways in the internal magnetic state of the human bodies are promoted in terms of orderliness and smoothness (such promotion needs to be achieved in the entire constant magnetic field, which will be described in detail hereinafter). However, when the people are in the strong magnetic field (about 1-2 T) of the accelerator, since the strong magnetic field of the accelerator is an alternating magnetic field, and the alternating magnetic field may affect the orderliness and smoothness of the biomagnetic field pathways in the internal magnetic state of the human brains, the biomagnetic field pathways in the internal magnetic state of the human brains are subject to blocking and isolation of information transmission. As a result, signals transmitted by the brain in the spirit level fail to be effectively and accurately transferred.

Qi Hao has employed an atomic force microscope (AFM) to observe changes of surface fineness of the cells treated with the static magnetic field (SMF). The results show that, with the magnetic prolonged exposure, the SMF may cause different degrees of damages or injuries on the surface of the tumor cell. Such damages or injuries may be mainly represented by a large number of depressions of different sizes on the cell membrane, and an increase of the number of depressions and the diameter with the increase of the exposure time, wherein the cells growing in a suspension manner are more sensitive to the magnetic field treatment than the cells growing in a wall-mounting manner.

When the mice transplanted with tumor are placed and raised in a non-uniform magnetic field having gradient of 0.1 T/cm and strength of 0.24-0.45 T, the tumor grows (measured by the area of the tumor) more slowly than the tumor not applied with the magnetic field; 15 days later, the tumor stops growing; 22 days later, the tumor starts shrinking. At the same time, the mice applied with no magnetic field die due to growth of the tumor. 27 days later, the tumor of the mice applied with the magnetic field completely disappears. This interesting experiment offers a possibility that the magnet therapy inhibits or event eliminate the tumor. After the tumor is transplanted into the mice, the tumor reproduces in the organism to form the biomagnetic field pathway in the internal magnetic state of the tumor. To survive in the organism, the tumor needs to overcome the immune system in the organism, and compete with the cells of the organism for the limited energy. The internal magnetic state biomagnetic field of the tumor interacts with the biomagnetic field of the organism of the mice, thereby forming a tumor internal magnetic state structure and biomagnetic field pathway that can survive and grow in the mice bodies and conduct conversion and exchange of the substance, energy and information under control of the spirit level. The tumor cells are characterized by absorbing energy from the external environment and unlimitedly divided, which is an internal magnetic state constantly forming new structures and biomagnetic field pathways. Therefore, the biomagnetic field is an instable biomagnetic field with no stable internal stage being generated, especially in case of malignant tumor. In addition, existence of the tumor in the mice body changes the normal structure and biomagnetic field pathway, and damages orderliness and stability of the biomagnetic field pathway in the mouse body. When the mice transplanted with tumor are placed and raised in a non-uniform magnetic field having gradient of 0.1 T/cm and strength of 0.24-0.45 T, under the action of the externally-applied magnetic field, the entire structure of the mice is repaired, and thus the internal magnetic state biomagnetic field of the mice recovers to be more orderly and smooth, whereas the biomagnetic field pathway of the tumor is inhibited. Therefore, in the initial stage of transplanting the tumor into the mice, the growth of the tumor becomes slower, and 15 days later the tumor is inhibited and stops growing; 22 days later, since the biomagnetic field pathway in the internal magnetic state in the mouse body recovers to be orderly and smooth, under double actions of the biomagnetic field of the organism and externally-applied magnetic field, 27 days layer, the tumor completely disappears.

For example, if the mice transplanted with tumor are placed in an alternating magnetic field (having a frequency of 12 Hz) having strength of 0.15-0.17 T, the growth of the tumor is inhibited. Even if the mice are taken away from the alternating magnetic field, the tumor cannot grow and develop. However, if the tumor grows to a tangible size, even if being applied with an alternating magnetic field or a constant magnetic field, the growth of the tumor cannot be inhibited. Since the repair of the internal magnetic state biomagnetic field pathway in the alternative magnetic field is not remarkable, but the effect of inhibiting the tumor is quite significant, to clarify the effect on the tumor, occurrence of the tumor needs to be described.

Four conditions need be accommodated:
Inflammation: caused by the cancer-causing factors to a part of cells in the organism.
   (2) Insufficient energy supply: The biomagnetic field pathway in the internal magnetic state in the cells in the inflammatory is severely blocked, the biomagnetic field in the inflammatory region is seriously damaged, and therefore the cells in the inflammatory area fail to conduct transmission and exchange of the substance, energy and information.
   (3) Bacteria environment in the inflammatory area: It is common that the bacterial coexist with the normal cells in the organism; for example, a scientific study has found that there are 1 million bacteria (having an average diameter of 0.5-5 µm) in the human body, 1000 different species, while the number of human cells (having an average diameter 10-20 µ η'l) is 40-60 trillion.
   (4) Transients: When the above three conditions are met and the role of external magnetic state of inflammatory cells in the region can not transfer and exchange of matter and energy information, resulting in areas of inflammation cells transient inflammation due to insufficient cellular energy supply area reached limit, and because the magnetic field of biological pathway is serious damage, control and feedback spiritual body cells can not be obtained within the area of mutual exchange, plus areas of inflammation bacteria living environment, as well as the external environment in which the external magnetic state biological magnetic field, causing the cells transient, transient abnormal cells to tumor cells, can not directly absorb energy and unlimited division in normal cells from the original way to become energy can directly access the ability to divide indefinitely and reproduction. (The role of bacteria in the transient process, a magnetic field is compatible cellular inflammation within the body region between the magnetic state in active bacterial extracellular magnetic state, according to the principle of magnetic compatibility, there will be areas of inflammation biomagnetic field within the magnetic state of the body cells will be active bacterial extracellular magnetic state of bio-magnetic field magnetization, and more and more bacterial cells biological external magnetic field tends to be the same as the body cells such areas of inflammation on having the bacterial cells characteristics, and to maintain the body cells in the process of the body portion of the magnetic field within the magnetic state of the biological pathway blocked, biomagnetic energy substances required information is cut off, so the cells can not get the body to provide energy material information, the role in inflammation , the bio-magnetic field in the region affected by body cells, bio-energy field become weaker, resulting in the cell's own structures and biological pathways not normally maintain the magnetic field, which would make the cells more vulnerable to the outer periphery of the magnetic state biomagnetic field. In the ambient bacterial environment, due to the bacterial outer magnetic field of biological state, the body cells in the region are more likely to be compatible with the bacterial outer magnetic field magnetic field generating biological state; 2. bacterial cells and cells of the organism conduct transmission and exchange of material and energy information transmission and exchange, because the body cells and bacteria in the magnetic state biomagnetic field in the region are compatible, the external magnetic field and bacterial biomagnetic field state in the region tends to be the same as the body cells, and therefore will be more likely among them The transmission of information and exchange of material and energy, as a magnetic field tends to be more compatible with the magnetic field after an organ transplant between identical couples, the exclusion does not occur, as between the body cells and bacterial cells were similar to human organs transplant, so that the magnetic state of the body cells in these structural changes occurred) transients produced by tumor cells under relatively favorable conditions, we will start to split value added, tumor cells have one to two, two to four It has been added to a common clinical tumor shape, then the role of mutual struggle between the biomagnetic field of the tumor and the biomagnetic field of the organism, thus further determining the direction the development of the tumor, diffusion, transfer or being restrained.

After the occurrence of the transients, further spreading and transfer of the tumor need to satisfy three conditions. Firstly, division is conducted in the region where the transients have occurred, to increase the number of tumor cells, and cause the tumor cells to spread around; when the surrounding energy is all absorbed by the tumor cells, the tumor cells are subject to proliferation and metastasis to accommodate its further demands on energy, just like the clock on the pasture, when the grass within a region on the pasture is eaten out, the flock need to find another region where grass is abundant. The proliferation needs to satisfy the following laws: The tumor cell which has the strongest biomagnetic field energy in all the tumor cells is subjected to proliferation first, and such tumor cell is called a "tumor stem cell". Metastasis of the tumor observes the adjacency principle. To be specific, the tumor is firstly subjected to metastasis to an organ or tissue closest to the pathway in the tumor region (for example, the stomach cancer is subjected to metastasis to liver, the spleen cancer is subjected to metastasis to intestine, and the lung cancer is subjected to metastasis to kidney). Selection of the pasture by the "tumor stem cell" complies with the three conditions in the transient process.

After the tumor is generated, orderliness and smoothness of the biomagnetic field pathway in the region of the organism are affected, thereby affecting the biomagnetic field pathway in the internal magnetic state in the organism. This results in reduction of the substance, energy and information transmitted over the biomagnetic field pathway in the internal magnetic state, reduction of the biomagnetic field flux in the biomagnetic field pathway in the internal magnetic state, reduction the energy of the internal magnetic state biomagnetic field; reduction of the substance, energy and information transported to various function organs, and reduction of the functionalities of the function organs. The reduction of the functionalities may also include insufficient energy supply of the central nervous system, such that the control on the other function organs in the spirit level of the central nervous system is weakened. In addition, the control on the tumor region is also weakened. This will result in that the biomagnetic pathway of the normal organism in the tumor region is blocked, the biomagnetic field flux in the biomagnetic field pathway is reduced, the energy transmitted to the region is also reduced, and accordingly the biomagnetic field in the region is weak. In this way, the region is simply subject to the impact caused by the tumor biomagnetic field and is simply intended to be compatible with the tumor biomagnetic field, such that the surrounding body cells becomes tumor cells via the transient process. Therefore, during the development of tumor, a part of tumor cells originate from division reproduction of the tumor cell, and a part of tumor cells originate from variation of the body cells in the transient process. The origin of the cancer cells vary with the development stage of the cancer.

Herein it is also important to make it clear that in the initial transient process, when the cells of the organism produce tumor cells, if the organism leaves the environment having inflammation (cancer)-causing factors such that the condition for the birth of the tumor cell is damaged. In this case, origins of the tumor cells in this region are similar, and the tumor cells are mainly formed by tumor cells produced by self-division and reproduction of the tumor cell generated in the transient process. While the organism leaves the inflammation-causing factors, in the course of mutual action and struggle between the internal magnetic state biomagnetic field of the organism and the external magnetic state biomagnetic field of the tumor, a hypermagnetic state biomagnetic field is applied, for assisting the internal magnetic state to make the biomagnetic field pathway in the internal magnetic state more orderly and smooth via repairing and dredging, enhance the biomagnetic field flux in the biomagnetic field pathway, and increase the supply of energy to the portions of the organism. In this way, more substance, energy and information may be transmitted and exchanged, thereby maintaining stability of the biomagnetic field of the organism, and orderliness and smoothness of the biomagnetic field pathway, and further inhibiting the effects caused by the external magnetic state biomagnetic field of the tumor to the internal magnetic state biomagnetic field of the organism, i.e., compatible with the internal magnetic state magnetic field of the organism. In this way, further development of the tumor is restrained. Further, while the organism leaves the inflammation-causing factors, the organism is in a youth state (stage), the internal magnetic state biomagnetic field of the organism is in an energy prosperous state, and orderliness and smoothness of the biomagnetic field pathway are in an optional state. Therefore, the tumor generated by the transients causes relatively less effects on the biomagnetic field of the organism, and further development of the tumor is restrained. With respect to the hypermagnetic state assisting the internal magnetic state, the energy in the biomagnetic field in the internal magnetic state of the organism is enhanced (such that the biomagnetic field flux in the internal magnetic state biomagnetic field pathway is increased), such that the internal magnetic state stays in a dominant role in the course of interaction and struggle with the external magnetic state biomagnetic field of the tumor, thereby inhibiting further development of the tumor. If the tumor is diagnosed in its early stage, the tumor may be quickly controlled and effectively treated.

The hypermagnetic state herein includes:
the portion assisting the organism to overall repair and dredge the biomagnetic field pathway in the internal magnetic state. This portion further comprises: the hypermagnetic state acting on the exterior of the organism so as to dredge and repair the internal magnetic state of the organism, for example, a human body suspension device to be described hereinafter; substances acting on the interior of the organism so as to enter the biomagnetic field pathway in the internal magnetic state of the organism via intake to repair and dredge the blocked and damaged biomagnetic field pathway in the internal magnetic state, and enhance the biomagnetic field flux in the biomagnetic field pathway. This further recovers the supply of the energy-containing substance to various portions of the organism, for example, drugs. (Note the symptoms reflected by the human body during the entire process, for example, symptoms from various symptoms caused by the tumor to increased food appetite and balanced weight approaching the standard weight.)

The hypermagnetic state assist the dredging of the biomagnetic field pathway in the internal magnetic state of the local tissues, organs, and the systems of the organism, and repair and dredge the blocked positions in the biomagnetic field pathway in the internal magnetic state by means of the effect of the hypermagnetic state biomagnetic field, such that the biomagnetic field pathway in the internal magnetic state becomes more orderly and smoother, and the biomagnetic field flux in the biomagnetic field pathway is increased, thereby further recovering the biomagnetic field pathways of various parts of the organism. In this case, the control and feedback effects of the central nervous system in the spirit level on various tissues and organs of the organism are enhanced, and the energy-containing substance is orderly and smoothly supplied to the internal magnetic state biomagnetic field, thereby recovering the supply of energy-containing substance to various parts of the organism.

The two processes of overall repairing and dredging of the organism and partial assistance for the organism are subject to no sequential limitation. These two processes may be separately performed, or may be simultaneously conducted, which is determined according to the blockage of the biomagnetic field pathway in the internal magnetic state of the organism. If it is desired to firstly repair and dredge the partial blocked biomagnetic field pathway in the internal magnetic state of the organism, and then repair and dredge the entire biomagnetic field pathway in internal magnetic state of the organism by using the hypermagnetic state, so as to the entire biomagnetic field pathway in the internal magnetic state of the organism is orderly and smooth, thereby further recovering the energy-containing substance supply to various parts of the organism. In this way, the diseases of the organism are completely and thoroughly cured, ageing is prevented and delayed, and the life quality is improved. For example, sometimes, it is desired to firstly repair and dredge the entire biomagnetic field pathway in the internal magnetic state of the organism, increase the biomagnetic field flux in the biomagnetic field pathway, and then transfer more energy to partial biomagnetic field pathway in the internal magnetic field of the organism under control of the central nervous system, so as to enhance the effects caused by the internal magnetic state biomagnetic field of the cells, tissues and organs in the region onto the external magnetic state. In this way, the internal magnetic state dominates in the interaction between the internal magnetic state and the external magnetic state, and overcomes the effects caused by the external magnetic state, thereby thoroughly curing the diseases of the organism, preventing and delaying ageing, and improving the life quality.
(Note that after the entire regulation, dredging and repairing, under control of the central nervous system, partial blockage is dredged and repaired, finally achieving the entire repairing; the blockage is firstly partially dredged and repaired and then the entire pathway of the organism is dredged and becomes orderly and smooth; finally, regulation and control of the whole organism are completed under control of the central nervous system.)

For example, wheat is treated in magnetic fields having different strength, and then the wheat seeds are sown, with results as listed in Table 6. As compared with the wheat seeds which are not treated in the magnetic field, the treated wheat seeds gain increase of yield. In addition to the yield increase (2.6%) of the wheat seeds treated in the magnetic field having specific strength, the wheat seeds treated in the other magnetic fields having different strength all gain the yield increase of 20% or higher, and the effects caused by the magnetic fields are remarkable.

**Table 6 Yield increase of the wheat treated in the magnetic field**

| Magnetic field strength (oersted) | Yield of the unit area (kg/Mu) | Yield increase (%) |
|---|---|---|
| 0 (control group) | 211.4 | / |
| 500 | 257.1 | 21.7 |
| 2000 | 253.7 | 20.0 |
| 5000 | 216.9 | 2.6 |
| 9000 | 261.1 | 23.5 |

In another experiment, after wheat seedlings are treated in a constant magnetic field having strength of 0.01 T, as compared against the control group not subjected to treatment in the magnetic field, the treated wheat seedlings have the advantages of high germination more tillers, growing well, large heads, with an average yield increase of about 8.8%. Barley seeds treated in a constant magnetic field having strength of 0.01 T gains 23.1% yield increase over the control group not subjected to treatment in the magnetic field. Barley seedlings grow in the magnetic field having strength of 0.12 T, with the seedlings and roots growing faster than the control group not subjected to the magnetic field. This indicates that the magnetic field promotes the growth. In the experiment where the seeds are treated in the magnetic field and applied with magnetic fertilizer, yield increase of the rape reaches 18.6%.

Since in the experiment, the wheat seeds are treated in the magnetic fields having different strength, the entire internal magnetic state biomagnetic field of the wheat is under assistance of the hypermagnetic states of the magnetic fields having different strength, and thus the entire biomagnetic field pathway in the internal magnetic state of the wheat is dredged and repaired. This enhances the biomagnetic field flux in the biomagnetic field pathway in the internal magnetic state of the wheat, and further increases supply of the energy-containing substances to various parts of the wheat, thereby achieving the effects of yield increase of the wheat.

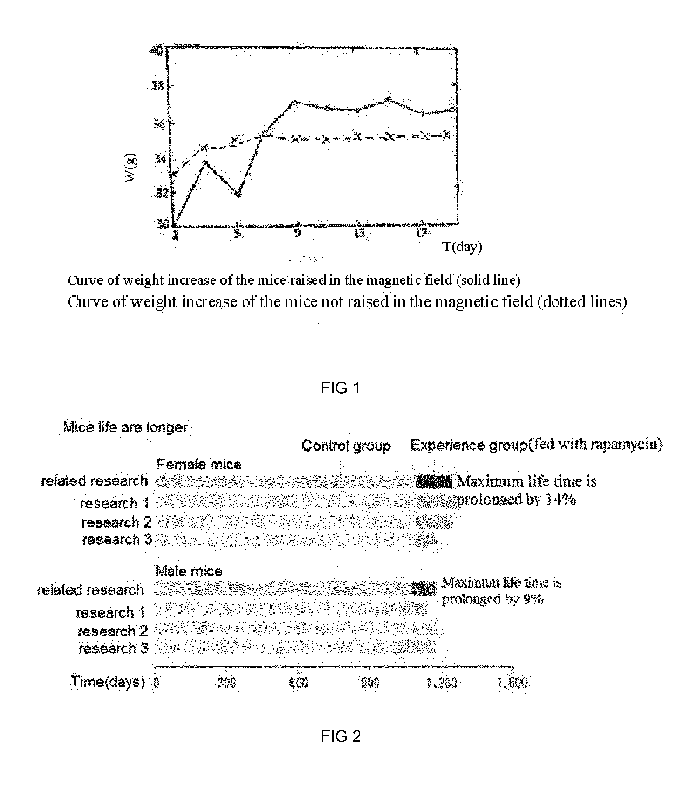

For example, chicken are raised in the magnetic field, and the weights of these chicken are double-folded over the chicken in the control group (the chicken are raised in no magnetic field); when an operation is made on the chicken, the chicken raised in the magnetic field recover faster than the control group in which the chicken are raised without the magnetic field. In an experiment where white mice are raised in the magnetic field, 14 white mice having an average weight of 32 g are randomly grouped into two groups, and are separately placed into two wooden mouse cabinets (having a dimension of 26 x 14 x 14 cm) for artificial feeding. A permanent magnet is disposed on each of two sides of one mouse cabinet. The magnetic field strength on the surface of the magnet is 0.15 T. The magnetic field strength at the center of the mouse cabinet is 0.025 T. No permanent magnet is applied to the other mouse cabinet, which serves as a control group. The experiment shows that in the first several days, the white mice raised in the magnetic field are relatively nervous, with ears standing up, with towering hair, enlarged pupil, and wet hair; and these mice lie in a group, and are tend to be frightened in case of light stimulus. The white mice in the control group have no such abnormalities, and have normal activities. Four days later, such differences gradually disappear. Within one month, a change of the average weight of these two groups is as illustrated in FIG. 1. The weights of the mice in the group subjected to treatment in the magnetic field increase quickly. Two months later, the average weight of the group treated in the magnetic field further increases, whereas the average weight of the control group decreases. It is obvious that the magnetic field exerts some effects.

Since the chicken in the experiment are raised in the magnetic field, the weights of the chicken increase remarkably and the wounds of these chicken are healed quickly, and the weights of the white mice raised in the magnetic field increase remarkably. These all indicate that the hypermagnetic state exerts significant effects on the growth and development of the animals or plants.

Magnetized water refers to water or an aqueous solution (hereinafter referred to as water) that flows vertically at a suitable speed through a magnetic field having suitable strength, i.e., the water flow traverses (cuts) a magnetic line. In a broad sense, the magnetized water also comprises water flows through the magnetic field at a very low speed, and non-flowing water in the magnetic field. Nevertheless, the magnetized water in the narrow sense and the magnetized water in the broad sense embody different symptoms and effects. Many scientific experiments, production practice, and other experiments concerning the magnetized water and its application show that the magnetized water exhibits some symptoms and effects that worth attention, some of which have been applied in practice. In agriculture, the magnetized water may be used for seed soaking, seed breeding, and irrigation. If the rice seeds are soaked in the magnetized water, the seeds sprout early and synchronously, and the yield may be improved. If the magnetized water is used for irrigation of the rice seedling bed and the field, the yield may also be increased. Table 12 lists the thousand-grain weight, yield per Mu, and yield increase as compared against the control rice bed where no magnetized water is applied under circumstances where magnetized water is used for seed soaking, irrigation of the seedling bed and the field. As seen from Table 12, the yield increase generally reaches about 10-20%. The "Hongmeizao" rice seeds soaked with magnetized water are treated in the magnetic fields having strength of 0.03 T, 0.05 T, and 1 T, the sprouting rates of the rice seeds treated in these three magnetic fields are respectively 95%, 85%, and 80%. On the contrary, the rice seeds soaked with common water (the control group) have a sprouting rate of only 80%. For example, the watermelon seeds soaked with magnetized water are treated in a constant magnetic field having strength of 0.055 T, the sprouting rate reaches 81%, whereas the sprouting rate of the watermelon seeds soaked with common water is only 50%. It can be seen from these experiment results that the magnetized water exerts remarkable effects on sprouting promotion by means of seed soaking.

**Table 7 Yield increase of the rice applied with magnetized water**

| Rice species | First experiment | | | Second experiment | | |
|---|---|---|---|---|---|---|
| | Thousand-grain weight (g) | Yield per Mu (kg/Mu) | Yield increase (%) | Thousand -grain weight (g) | Yield per Mu (kg/Mu) | Yield increase (%) |
| Control group (with no magnetized water applied) | 28.3 | 320.9 | / | 28.5 | 365.0 | / |
| Seed soaking with magnetized water | 30.0 | 360.9 | 12.5 | / | / | / |
| Field irrigation with magnetized water | / | / | / | 29.5 | 393.8 | 7.7 |
| Seed soaking with magnetized water, and seedling bed irrigation with magnetized water | 29.2 | 347.5 | 8.3 | 30.3 | 406.0 | 11.2 |
| Seedling bend and field irrigation with magnetized water | 29.9 | 360.8 | 12.4 | 30.5 | 431.0 | 18.1 |
| Seed soaking with magnetized water, and seedling bed and field irrigation with magnetized water | 32.9 | 394.7 | 23.0 | 33.4 | 444.1 | 21.7 |

The magnetized water also exerts remarkable yield increase effects when being applied in seed soaking and irrigation of the vegetable (Table 8).

**Table 8 Yield increase of the vegetable applied with magnetized water**

| Treatment with magnetized water | Vegetable species | Magnetic field strength (T) | Experiment area (Mu) | Yield increase (%) |
|---|---|---|---|---|
| Seed soaking with magnetized water | Radish | 0.2 | 8 | 5.4 |
| | Carrot | 0.3 | 5 | 15.3 |
| | Bok choy | 0.3 | 7 | 12.7 |
| Irrigation with magnetized water | Radish | 0.25 | 3 | 27.7 |
| | Tomato | 0.25 | 7 | 25.1 |
| | Garlic | 0.25 | 7 | 19.3 |
| Seed soaking and irrigation with magnetized water | Radish | 0.25 | 2 | 35.5 |
| | String bean | 0.25 | 2 | 15.1 |
| | Cowpe a | 0.25 | 3 | 15.7 |

The experiment results show that treatment with magnetized water increase the yield of spinach, garlic chives, cabbage, bamboo shoots, celery, clinatro, soybean, cucumber, pepper and the like, with the general yield increase of about 10-30%, as high as over 50% (for example, cucumber). In addition, some experiment results further show that when the seeds are soaked with magnetized water for 5 hours, the yield of the sunflower increases by 21%, and the yield of the sugarbeet increases by 40%, and the scallion, tomato and carrot grow more quickly and the yields thereof increase.. Therefore, the magnetized water not only exerts significant yield increase effects for the crops, but also promotes reproduction of the favorable microorganisms in the soil. For example, gaseous rhizobium, azotobacter vinelandii, and Inorganic phosphorus bacteria are respectively cultivated by using magnetized water treated in a 0.2 T constant magnetic field and by using ordinary water (the control group); through 12 days' cultivation, the yield increase of the gaseous rhizobium, azotobacter vinelandii, and Inorganic phosphorus bacteria in the experiment group are respectively 443%, 222%, and 90% as compared against the control group. The experiment result shows that the magnetized water is capable of promoting activities, growth and reproduction of the microorganisms in the soil, which lays a foundation for the development of bacteria fertilizer. Generally speaking, many experiments show that the effects exerted by the magnetized water onto the crops are reflected in: promoting sprouting of the seeds, growth of the roots, water and fertilizer absorption ability, early tiller; advancing the fruit-bearing period; increasing grain quantity and thousand-grain weight; enhancing resistance to coldness and diseases of the crops; and increasing the sugar content of the vegetable.

In the aspect of animal raising, in an experiment, magnetic water is used for raising fishes, when no food is fed to the fishes, the life time of the fishes raised in the magnetized water is one or two times longer than the fishes raised in ordinary water. In this experiment, the fishes in the experiment group have life time one or 2 times longer than that of the control group due to the following reasons. First, under the effects of the hypermagnetic state biomagnetic field of the magnetized water, the biomagnetic field pathway in the internal magnetic state of the fishes in the experiment is further dredged and repaired, and the biomagnetic field flux in the biomagnetic field pathway in the internal magnetic state of the fishes in the experiment group is enhanced. In this way, the biomagnetic field pathway in the internal magnetic state of the fishes is more orderly and smoother, such that utilization rate of the energy and substance information in the organisms of the fishes are higher. In another aspect, in the experiment, no food is fed to the fishes. Therefore, in the life of the fishes, since the fishes take less energy-containing substances, the organisms of the fishes maintain normal life activities of the fishes by using the stored energy. With the consumption of the energy-containing substances stored in the organisms, the supply of the energy-containing substances is insufficient, resulting in exhaustion and death of the organisms. However, in the experiment group where the fishes are raised in the magnetized water, since the biomagnetic field pathway in the internal magnetic state of the organism is more orderly and smoother, the process from exhaustion to death of the fishes in the experiment group is prolonged against the control group. Second, with respect to various bacteria microorganisms and organisms such as alga and the like growing in the magnetic water, under the effects of the hypermagnetic state of the magnetized water, growth and reproduction of these organisms are promoted, thereby providing some food for the fishes in the experiment group, and thus prolonging the life time of the fishes in the experiment group. Concerning this aspect, designers of the experiment may conduct no analysis and research. However, this is a main objective factor.

### Ageing and Disease

In the study of the life science, they are common issues on how to prolong the life time and prevent ageing, to inhibit diseases, and to improve the life quality. Among these issues, preventing ageing comes first. This is a critical issue. At present, ageing is irreversible. However, by means of various technologies, ageing may be delayed, and ageing is not constantly unchangeable. No limitation is imposed on the life time of the human beings, and in fact, it is common that many people can live to their old age of 80 or 90. If we understand the biological principle and try to change the biological principle in a specific manner, theoretically we can live to the very old age of 200.

From the perspective of biology, ageing is a natural course with advancement of time, and which is a complicated natural phenomenon and reflected by retrogression of structure, functional decline, degradation of adaptability and resistibility. In the physiology, ageing is regarded as an individual growth and development course from an oosperm to the old age. In the pathology, ageing is result out of the accumulation of stress and strain, injury and infection, immune reaction degradation, malnutrition, dysbolism, ignorance, and drug abuse. In addition, from the perspective of the sociology, ageing means that an individual loses interest on fresh things, is not restrained in reality, and starts remember and cherish the past. From the perspective of Chinese traditional medicine, Suwen Shanggu Tianzhenlun (a collection of commonly asked questions resolved using the ancient theory which valuing the spirit given by the heaven) has discussed in detail that women grow, develop and age at a base of seven and men grow, develop and age at a base of eight in a curve reflecting senescence of Kidney's Qi; and clearly points out that the birth, growth, getting stronger, ageing, and death of the organism are regulated by the Qi in the kidney. To conclude, "kidney" plays a dominant role in the essential factors causing ageing.

The current study considers that ageing is a result collaboratively caused by such factors as cell degradation, DNA degradation, diet and spirit, ageing gene activity and the like. However, till now, no uniform ageing theory has been formulated.

Concerning the drive force of ageing, i.e., what drives the ageing, ageing needs to be discussed from two aspects? One aspect focuses on becoming old (which is a time concept, the degree of oldness is determined by decline and degradation, i.e., weakening of energy of the organism, which is duration of the course from strong to weak and finally becoming insufficient to support life activities). Decline or degradation means that the utilization and absorption of the normal substance energy by the cells are affected due to blocked and unsmooth supply; acquisition and exchange of substance, energy and information, and thus information exchange with the external magnetic state in the spirit level are restricted. As a result, the stable structures of the cells are changed and the normal functions are weakened, the acquisition and utilization of the energy-containing substances are weakened. To sum up, the decline or degradation is a process of energy reduction, from strong to weak. The fundamental reason of the ageing is that the orderly and smooth hindered vivo magnetic state biomagnetic path, in the spirit level of internal control magnetic state of the material energy information biomagnetic transport path running error and failure, resulting in an error and control changes to failure at the region within the magnetic field outside the magnetic state of the biological condition of biological magnetic field, on the one hand due to the effect of regulation under control, coordination, assist in the spiritual, material information to mobilize the energy within the body due to the magnetic state biomagnetic path orderly and smooth blocked, we can not effectively reach the inner magnetic state, reduced by the internal magnetic state biomagnetic path of biomagnetic field flux path, causing the material to reach the energy within the magnetic state of lack of information (e.g., the internal magnetic state of their own needs 1000 KJ energy, but due to the magnetic field within the magnetic state of the biological pathway blocked by a magnetic field within the magnetic state of biological pathways, only 500 KJ energy is provided), so that the effect of the magnetic field outside the magnetic state of the biological state under the influence of the generated energy is insufficient due to lack of material information to make changes without recovery, thus forming an irreversible change in terminal cancer, the tumor the role of the cell, resulting in the blockage of the biomagnetic field pathways in the internal magnetic state biomagnetic of the organism suffers from damage, thereby greatly reducing the biomagnetic field flux in the biomagnetic field pathway, and blocking the transmission and exchange of the substance, energy and information. When the energy-containing substances of the organism are excessively consumed, the organism is subjected to severe thinning and dehydration. Although the patient may be injected with a solution containing a large number of nutritious substances, energy and drugs (gamma globulin, globulin, ovalbumin, ATP, nutrient, and chemotherapeutics), since the biomagnetic field pathway in the internal magnetic state is damaged, the nutritious energy-containing substances that are remarkably effective in normal organisms cannot be transported to the target location (wherein these nutritious substances and energy function). As a result, the organism fails to effectively be supplemented with energy, nutrients and moisture, and the drugs fail to be transported to the target location to function. Consequently, the assistance effect on the organism and fragmentation effect to the tumor cannot be brought into full play.

In another aspect, the orderliness and smoothness of the biomagnetic field pathway in the internal magnetic state are affected, and thus exchange of substance, energy and information between the internal magnetic state, the external magnetic state and the spirit level. The information fed back by the internal magnetic state to the spirit level cannot accurately reflect the degree of the variation of the internal magnetic state under effects of the external magnetic state biomagnetic field (for example, 100 cells in the region of the organism are subjected to infection; however, since the orderliness and smoothness of the biomagnetic field pathway in the internal magnetic state are damaged, and thus only 50 cells correctly feed back the infection information to the spirit level). As a result, the feedback information is incorrect, and thus under the control of the spirit level, substance, energy and information desired by the internal magnetic state biomagnetic field fails to be sufficiently and accurately to the destination. In this way, the effects exerted by the internal magnetic state biomagnetic field under the effects of the external magnetic state fail cause any variation or restoration due to insufficient energy and lack of the substance information. Therefore, irreversible variations are formed. In the later period of cancer, due to the effects of the tumor cells, the biomagnetic field pathway in the internal magnetic state of the organism is damaged and destroyed, and the information fed back by the normal cells of the organism cannot accurately reflect variations and variation degree during the course of interaction and struggle between the normal cells and the tumor cells. The cancer patient in this period may suffer from various pains. Although there are many analgesic drugs available (for example, morphine, thorazine, and atropine) for application in the organism, since the biomagnetic field pathway in the internal magnetic state is damaged and destroyed and the information fed back by the normal cells of the organism to the spirit level cannot accurately reflect variations and variation degree during the course of interaction and struggle between the normal cells and the tumor cells, the drugs exerting remarkable effects in the normal organism cannot be transported to the target location (where the drugs function). As a result, the pains of the patient cannot be effectively relieved.

From the perspective of the cell level, intercellular substances are present between cells, and the cells in the human body tissues are all immersed in the intercellular substance fluids. The intercellular substance bears the cells, protects the cells, interconnects the cells, and provides nutrients for the cells; and participates to constitute the micro-environment where the cells live. The intercellular substance is a liquid environment where the cells of the human bodies live. The intercellular substance fluid contains all substances needed for the metabolism of the cells. Likewise, the intercellular substance fluid also receives the metabolism products of the cells, or the substances that are not used. The cells uninterruptedly conduct substance exchange with the liquid, absorb oxygen and nutrients, and discharge such wastage as carbon dioxide.

With the advancement of the life activities, the biomagnetic field pathway in the internal magnetic state of the organism is blocked, and transmission and exchange of substance, energy and information between the intercellular substances and the cells, between the cells, between the intercellular substances and the vascular lymphatic vessels are blocked and isolated. Therefore, the transmission and exchange of substance, energy and information among the cells, the intercellular substances, and the biomagnetic field pathways of the entire organism are affected. For example, the collagen molecules forming the intercellular substance are subjected to crosslinking bonds therebetween, as the biomagnetic field pathway in the internal magnetic state of the organism is blocked, transmission and exchange of substance, energy and information between the intercellular substances and the cells, between the cells, between the intercellular substances and the vascular lymphatic vessels are blocked and isolated. In this way, the crosslinking between the collagen molecules increases sharply. With the increase of the crosslinking, the water absorption of the collagen fibers is reduced, and the fibers lose tenacity, tending to be stiff, which is unfavorable to activities of the tissues. The elastin is a major component of the elastic fiber, and may be subjected to crosslinking as the entire biomagnetic field pathway of the organism is blocked and isolated. Fiber breakage, fiber tendering, yellow darkening in appearance may occur. As such, the buffer device between the cells is degraded in terms of functionality. In the movement of the organism, due to degradation of the buffer function of the intercellular substance between the cells, the cells are subjected to direct collision, thereby destroying the skeletal frame structure of the cells, and abnormal cytoadherence between the cells is present, and hence more cells are subjected to such abnormal cytoadherence. This destroys and affects the orderliness and smoothness of the biomagnetic field pathways between the cells and the intercellular substances, and between the cells, and reduces the biomagnetic field flux in the biomagnetic field pathway. Subsequently, the cells in the region where the abnormal cytoadherence are subjected to insufficient supply of the biomagnetic field substances and energy, and unsmooth information exchange, such that the biomagnetic fields of the cells in this region are weakened, and the functions thereof are degraded. In this way, the decline starts. If such a process occurs and is not restricted or inhibited, it is common that we feel degradation and decline of our bodies, and functionalities of our bodies (the functionality does not cover the brain's thinking function).

Described above is the abnormal cytoadherence occurred between the cells due to variations of the intercellular substance. It is also possible that, the variation of the intercellular substance may be firstly caused due to the cells, and then the above described outcome occurs due to the variation of the intercellular substance.

Intercellular substance: An intercellular substance exists between a cell and another cell, and cells in the human body tissues are all immersed in a intercellular substance fluid. The intercellular substance bears the cells, protects the cells, interconnects the cells, and provides nutrients for the cells; and participates to constitute the micro-environment where the cells live. The intercellular substance is a fluid environment where the cells of the human bodies live. The intercellular substance fluid contains all substances needed for the metabolism of the cells. Likewise, the intercellular substance fluid also receives the metabolism products of the cells, or the substances that are not used. The cells uninterruptedly conduct substance exchange with the liquid, absorb oxygen and nutrients, and discharge such wastage as carbon dioxide.

Analysis is made to the power (vigor) of the life to investigate the course in which the power changes from strong to weak and from weak to strong and then to weak again. (Note: In the ageing group, there are a large proportion of ageing living organisms whose bodies are weakened but their spirit such as thinking remains unchanged; however, for example, during the cultivation of chicken, some chickens' reproductive organs are not well developed, or even not developed, and thus their life time is short; with respect to plants, by means of passively reception of different stimuli and effects of different environments in various development stages in the static state, the plants become active. In the 90th year of the nervous system of a human being, the brain weight of the human being is reduced by 10 to 20% over the 20th year of the human being. The brain weight reduction is mainly caused by loss of the neural cells. Such loss is subject to regional specificity, for example, different degrees of reduction of cells in the different regions of the brain. At the age of 10, the neural cells are reproduced to their maximum value, and then no cell division occurs; and after 20 years old, the cell loss starts. However, the cell quantity in the entire brain is very large, and death or loss of some cells may not cause severe impacts. In addition, people have less knowledge about the memory machine. Therefore, memory degradation is not necessarily caused by cell loss.)

With respect to the base membrane, it is known that the base membrane is thickened in case of ageing, and the major component of the membrane is also the collagen, and other components thereof are glycoprotein and carbohydrate. However, it is not clear on how these molecules change to cause the thickening of the membrane. In addition, extracellular substances include blood and lymph. These substances are always in a running state, and are constantly updated, and therefore it is hard to determine the indicators of ageing.

Rapamycin may regulate the ageing process of the mice and other mammal animals. The kernel element of this mechanism is the target of rapamycin (TOR, the target protein of rapamycin), and the gene coding the protein (TOR gene). The TOR protein is a focus mostly concerned in the current geratology and pharmacy, because more and more animal clinical experiments indicate that inhibiting the activity of the TOR protein (that is, mTOR) in the cells of the mammal animal reduces risks of occurrence of most ageing-related diseases, for example, cancer, Alzheimer's disease, Parkinson's disease, neurodegenerative disease, type II diabetes, osteoporosis, macular degeneration, and the like. That is to say, if an effective drug is discovered which is capable of effectively inhibits the activity of the mTOR protein, the ageing of the human beings can be delayed, just like the effects exerted by rapamycin in the mice. This plays an extremely great significance in the preventative medicine. FIG. 2 schematically illustrates three experiments wherein the maximum life time of the mice may be prolonged by 9% to 14% with rapamycin. (Note: The maximum life time refers to an average life time of 10% oldest individuals in a group.) In the nutrition sensing stage of the juvenile period, the mTOR protein functions mainly in the form of a complex body of the mTORC1. When the nutrients are sufficiently provided, insulin and related growth factors are greatly produced and the mTORC1 is activated, thereby promoting synthesis of the cell components (mainly protein and fat), enhancing the cell growth and reproduction, and meanwhile inhibiting autophagy (which may degrade damaged mitochondria and molecules). When the nutrients are insufficient, the activity of the mTORC1 is inhibited and the cells stop reproduction and turn to self protection, thereby enhancing autophagy and providing energy and raw materials for cell renewal and energy system reestablishment.

As a destroyer of the old age, in adulthood, if the mTORC1 maintains it activity, the protein may be excessively synthesized, resulting in aggregation of proteins and abnormal reproduction of some cells (for example, reproduction of the smooth muscle cells may result in atherosclerosis, increase of osteoclast may result in bone damages), cell function damage (for example, reduction of the sensitivity of the cells to the insulin may result in diabetes), induction of cell ageing (the cells stop division and grow abnormally, which causes toxicity to the adjacent cells). Activation of the mTORC1 may inhibit the autophagy effect, and consequently the abnormal proteins and functionless mitochondria aggregates in the cells). When the activity of the mTORC1 is inhibited, the above course may be interrupted, which facilitate delaying of ageing.

During the study on the TOR protein, why does not mTORC1 maintains activity in the adulthood, and why does the maintained activity result in excessive synthesis of the proteins such that the abnormal proteins aggregate? One condition is the intake of sufficient food. In the adulthood, assimilation and dissimilation in the organism reach a dynamic balance, and reproduction and death in the organism also reach a dynamic balance. In this period, the biomagnetic field pathway in the internal magnetic state of the organism is subjected to the impacts of the biomagnetic field pathway in the external magnetic state (including the impact of the environment where the organism lives in a long period of time, impact of the ever-changing magnetic field, the impact of the motion, and the like), such that the orderliness and smoothness of the biomagnetic field pathway in the internal magnetic state is inhibited, and the biomagnetic field flux in the biomagnetic field pathway in the internal magnetic state is reduced. This weakens the regulation on the entire internal magnetic state by the central nervous system via the internal magnetic state biomagnetic field of the organism in the spirit level, and breaks the original balance of the organism (the original balance includes a balance between assimilation and dissimilation via interaction of the assimilation and dissimilation in the course of growth and development of the organism, and a balance between death and reproduction of cells via gaming between cell death and cell reproduction in the course of growth and development of the organism). This balance is broken due to the following reasons. In one aspect, out-of-control of the nervous system may result in excessive synthesis of proteins. In another aspect, insufficient utilization of the proteins may result in aggregation of proteins, and the organism cannot or has no ability to utilize the excessively synthesized proteins and aggregated proteins. As such, the proteins are utilized and absorbed by the parasitic fungus, bacteria and viruses in the organism. Therefore, the balance between the normal cells and the parasitic fungus, bacteria and viruses in the organism is broken, and thus the organism needs to employ more substance, energy and information to build a new balance with the fungus, bacteria and viruses. In this way, the probability of such diseases as tumor may be increased. Due to excessive synthesis of proteins and aggregation of abnormal proteins, and due to the fact that abnormal reproduction of some cells result in blockage of the normal biomagnetic field pathway, the structure of the biomagnetic field pathway in the internal magnetic state is changed. Therefore, the regulation function of the nervous system in this region fails to be effected, and thus the internal magnetic state biomagnetic field is out of control, which further affects the orderliness and smoothness of the entire biomagnetic field pathway of the organism, such that transmission and exchange of substance, energy and information in this region is blocked. This affects supply of the substance and energy and exchange of information in this region, for example, interaction between the surrounding external magnetic state and the spirit level of the nervous system, which finally resulting in insufficient supply of the cell energy and invalidation of control of the spirit level in the nervous system in this region. Consequently, the structures of the cells in this region are changed, and thus inheritance of the normal functions and information of the cells is affected. Therefore, as described above, as long as an effective drug is discovered which is capable of effectively inhibit the activity of the mTOR protein, the ageing process of the human beings may be delayed after adulthood, and risks of occurrence of most ageing-related diseases, for example, cancer, Alzheimer's disease, Parkinson's disease, neurodegenerative disease, type II diabetes, osteoporosis, macular degeneration, and the like may be reduced. This may be feasible in human bodies. Although at present it has been identified that rapamycin may regulate the ageing process of the mice and other mammal animals, side-effects (inhibiting the activity of the TOR protein) of rapamycin determine that it cannot be applied to human beings. Therefore, the scientists desire to develop a drug which is capable of safely and effectively inhibiting the activity of the mTOR protein, thus to delay the ageing progress of the human beings. However, until now, such an effective drug causing no side-effect like rapamycin has not been developed. Even if a substitution drug is found, it has no essential significant as compared against the present invention (theory, process, device, and technology). Assume that rapamycin is injected and then functions in the human body, according to the experiment results obtained in the mice, the maximum life time is respectively prolonged by 9% and 14%. To be specific, the life time of the human being may be prolonged from the current 73.4 years to 83.7 years. This signifies that we would need to use rapamycin from the age of 50 to the age of 83 uninterruptedly to prolong our life. During this course, people would be ready anytime to fight against old age-related diseases such as cancer. However, this is a way scarifying the life quality but only prolonging the life course, which goes beyond our pursuits of long life, free of diseases, and improved life quality. Actual ageing delaying lies in prolonging the stage of the initial balance, and making the initial balance prolonged as much as possible. To this end, orderliness and smoothness of the biomagnetic field pathway in the internal magnetic state of the organism shall be ensured, stability in the spirit level needs to be guaranteed (for example, happiness, joy, and good mood), and normal supply of the substance, energy and information is assured, instead of reducing the substance, energy and information entering the pathway by inhibiting the activity of the mTOR protein when the entire biomagnetic field pathway in the internal magnetic state of the organism. This maintains a balance matching with the blocked biomagnetic field in the internal magnetic state, reduces the occurrence of the ageing diseases such as tumor, and meanwhile inhibits assimilation and cell production in the organism and maintains the normal structure and functionality of the organism. Therefore, this is a negative way which surrenders to cancer-causing factors.

In the life course, the TOR protein reflects double functions, i.e., when a living organism is in its juvenile stage, the TOR protein is capable of sensing nutrition changes and regulating growth and development; in the adulthood, if the activity of the TOR protein is maintained, the TOR protein may damages the cell functionality and cause damages to the organism. It can be seen from the double functions of the TOR protein that the TOR protein implements consistent functions in the life course of the living organism. To be specific, in the juvenile stage and the adulthood, the function is that the TOR protein senses the nutrition changes. When the food is sufficient, the activity of the TOR gene increases, such that the more proteins are generated in the cells, and cell division starts. Since in the adulthood the TOR protein may damage the cell functionality and cause damages to the organism, if the activity of the TOR protein is maintained. The main reason is that the living organism grows to an adult. Therefore, only by means of regulation and assistance of the hypermagnetic state, the biomagnetic field pathway in the internal magnetic state of the organism can be dredged, orderliness and smoothness of the biomagnetic field pathway in the internal magnetic state can be maintained, stable running of the biomagnetic field flux in the biomagnetic field pathway in the internal magnetic state is ensured, and the effective control by the central nervous system in the spirit level is achieved. As such, the proteins produced upon activation of the TOR protein, thus excessive synthesis of proteins may not occur, and the aggregation of abnormal proteins may not be present. Since the control in the spirit level is effectively enhanced, division and reproduction of the cells may go on under the effective control in the spirit level. Therefore, even if the TOR protein is activated, abnormal reproduction of some cells may not take place in the organism (for example, reproduction of the smooth muscle cells may result in atherosclerosis, increase of osteoclast may result in bone damages). Orderly and smooth biomagnetic field pathway in the internal magnetic state ensures smooth transmission and exchange of the substance, energy and information between the cells, and between the cells and the energy-containing substances, and thus sufficient energy-containing substances are provided for the cells. As such information exchange between the cells and the external magnetic stage biomagnetic field and the spirit level is smooth, such that normal running of the normal functions and structures of the cell are maintained. Therefore, it is impossible that functionality of the cells is damaged due to the activation of the TOR protein (for example, reduction of the sensitivity of the cells to the insulin results in diabetes). The process of such cell senescence (the cells stop division and grow abnormally, which causes toxicity to the adjacent cells) is greatly prolonged (which may be prolonged for several times of the original process). Such cell senescence may occur when the TOR protein is subjected to function changes (attenuation). In other words, the TOR protein ensures long life of the living body.

Restriction of the TOR protein requires a powerful, orderly and stable living organism in the course of growth and development matching the TOR protein. People are in a trinity biomagnetic field at the same time, place and environment, and spirit and energy theory may be completely used to create and maintain a basic platform desired by a living organism. On such a platform, senescence and exhaustion of the living organism are impossible, because the biomagnetic field is smooth and (magnetic reluctance) may not be caused. Therefore, an energy system running on the biomagnetic field platform is capable of completely ensuring smoothness (causing no biomagnetic reluctance) of effective energy supply, and ensuring effective absorption, conversion, distribution, utilization and discharge of the energy, i.e., magnetic flux. As such, the nervous system (cranial nerves, small intestine nerves, and all the nerves of the body) that receives sufficient energy supply is capable of effectively commanding and coordinating various units of the living organism, further the cells and the parasitic flora of the living organism. This ensures more effectively compatibility and operation. During the operation, suitable and accurate compatibility, matching, and complementation between the TOR protein, various proteins, and various enzymes are achieved. As such, the life of the human beings, animals and plants may be greatly prolonged, and stability of such living organisms may be maintained for a relatively long period of time. Even cell inversion is achieved, such that inversion of the whole living body is achieved. In such a state, it is impossible that the energy generated due to excessive activation of the TORC1 protein is not absorbed. People may not suffer from various diseases after taking the TORC1. In addition, the protein may not be excessively synthesized, the abnormal proteins may not be aggregated, and abnormal reproduction of the cells may not occur (for example, reproduction of the smooth muscle cells may result in atherosclerosis, increase of osteoclast may result in bone damages). Cell function damage (for example, reduction of the sensitivity of the cells to the insulin may result in diabetes), and induction of cell ageing (the cells stop division and grow abnormally, which causes toxicity to the adjacent cells) may not be caused. Activation of the mTORC1 may inhibit the autophagy effect, and consequently the abnormal proteins and functionless mitochondria aggregates in the cells). When the activity of the mTORC1 is inhibited, the above course may be interrupted, which facilitate delaying of ageing.

In such a state, it is impossible that the energy generated due to excessive activation of the TORC1 protein is not absorbed. People may not suffer from various diseases after taking the TORC1. Only in this state, the energy generated by activation of the living organism is all completely converted and absorbed, atherosclerosis caused by reproduction of the smooth muscle cells may not occur, bone damages caused by increase of osteoclast may not occur, diabetes caused by reduction of the sensitivity of the cells to the insulin may not occur, the cells may not stop division or grow abnormally, which causes no toxicity to the adjacent cells.

Since the TOP protein generates excessive energy, normal operation of the TOR protein may be suppressed. Therefore, such methods as diet restriction, vegetarian diets, peinotherapy, and breatharianism are used to suppress activation and normal operation of the TOR protein, prevent such diseases as fat accumulation, and delay ageing of the living organism by about 10%. In this way, the survival time in the relatively balanced weak magnetic state (the life energy is relatively weak) is prolonged by 10%.

At present, by means of diet restriction, vegetarian diets, peinotherapy, and breatharianism, the effect on preventing occurrence of the above diseases such as fat accumulation is still not ideal. For example, with respect to cancer patients, no cases where the cancer is cured by means of reducing energy supply, i.e., peinotherapy are available. Activation and normal operation of the TOR protein result in that the TOR protein fails to be better compatible with damages from exhaustion and weakness, thereby failing to achieve an effective matching. Various diseases may be caused after taking the TOR protein, and thus the life time of the living organism is shortened. All the above is caused by the magnetic reluctance of the biomagnetic field of the living organism. For example, with respect to the mouse, bat and crow, they have similar vital signs, including blood flow and qualities of various visceral organs including the heart, but the life time of these living organisms is different. For example, the life time of the mouse is 2.5 years, the life time of the crow is from 7 to 10 years, and the life time of the bat is from 15 to 20 years. This is because the mouse is always in spiritual pressure of panic and anxiety, and needs to seek food outside, with ever changing external magnetic environments; and therefore, the magnetic reluctance of the internal magnetic state magnetic field is increased, and the magnetic flux (energy supply) is reduced, which results in that the nerve cell energy fails to be supplied effectively. As such, regulation, commanding and matching of the normal operation of the energy of the biomagnetic field are not sufficient or effective, causing blocking and disorderliness. As a result, the life time of the mouse is far less that of the bat. The crow suffers a pressure much less than that of the mouse and is subjected to interference of the external magnetic field much greater than that of the bat. Therefore, the life time of the crow is between that of the mouse and that of the bat. The bat neither suffers from the pressure not suffers from dangers, and is free of the interference of the external magnetic state since the bat hides in the day and comes out at night, and lives in the cave. Therefore, the biomagnetic field of the bat is smooth, and complies with the trinity theory of the biomagnetic field, spirit control and energy supply. In this way, the life time of the bat is the longest among the three.

Under assistance of the device and magnetic flux based on the trinity theory of the biomagnetic field, neural regulation and control, and energy supply (hypermagnetic state), the life time of the mouse would be longer than that of the bat. In practice, according analysis of the current studies, we consider that the effect of the activation of the TOR protein is compatible, interactive and matched with the normal life of the organism (300 to 500 years for human beings). It is the spiritual pressure (happiness, anger, sadness, and joy), over-fatigue (declination), diseases (declination), and interference of the external magnetic field that breaks such matched balance and thus causes the blocking of the magnetic field of the living organism, thereby affecting the supply of the magnetic flux, resulting energy insufficiency of the neural magnetic field, and causing disorderliness of the regulation, commanding and coordination and increase of invalid frequencies (including imbalance of the TOR protein energy and metabolism). This causes senility before ageing, and a failure to reach an expected and theoretical life time (less than 15% of the human brain cells are used in the life and the usage of the corresponding neural network fails to reach 15%). Actually, as long as the magnetic flux (energy) of the biomagnetic field in the internal magnetic state is effectively called, the magnetic reluctance of the biomagnetic field is broken, and the biomagnetic field pathway is maintained smooth, the energy of the biomagnetic field can be sufficiently, effectively and orderly supplied, and thus sufficiency energy is supplied to the neural system of the life organism for commanding and regulation, thereby effectively coordinating suitable operation of the biomagnetic field of the living organism under balance of strong magnetic state. Such call is a method for self-treatment and curing by means of overcoming the interference of the external magnetic state, and may have a plurality of manners, including, psychological hint, hypnotherapy, praying to Buddha for help, divination, praying in the church, Qigong, Yoga, and the like. By means of the above manners, the neural energy (mind) is centralized, the biomagnetic field is called, the magnetic reluctance is overcome, and the magnetic field operates effectively. That is, if you do things with your heart, that will come true one day, which reflects the relationship between spirit and energy. As reported by China CCTV, late-stage cancer patients (liver cancer) are self-cured by means of sculpturing (Shanxi sculptor xxx), painting (Beijing xxx), photographing (photographer xxx), and dancing (dancer xxx). All this indicates that sculpturing, painting, photographing, and dancing are their Buddha, God, and psychological hint in their minds, and therefore they are successful in treating their diseases. Especially, after knowing that he suffers from the hepatitis B and C viruses, the great painter Wu xx abandons treatment and goes out to devote himself into painting. One photo reflects his devotion to the sketching, wherein flies and mosquitoes land on his back when he is painting, but Mr. Wu is not aware about it at all. In this case, the beauty of the mountain is the Buddha and God for Mr. Wu, and he aggregates his spiritual system by means of magnetic field, and calls the convention energy of the biomagnetic field to gather all the energy to break the magnetic reluctance of the biomagnetic field. Day after day, the absolute magnetic reluctance becomes the relative magnetic reluctance, and finally the relative magnetic reluctance is eliminated, thereby achieving complete smoothness of the biomagnetic field. Mr. Wu returns to Beijing and finds through examination that his hepatitis has been completely cured. In addition, with the mind of praying to Buddha for help, many diseases that are hard to cure have been cured. Even if a person has no ability to call the biological energy in the internal magnetic state to break the magnetic reluctance of the biomagnetic field, by means of our trinity platform, the biomagnetic field can still be effectively and property smoothened, such that the neural system commands and regulates suitable supply, absorption, distribution, usage, and discharge of the desired energy.

"Ageing" is continuity in time, a time dimension for measuring the life existence, and a duration from the birth through such stable life structure to another living organism (during this duration, some parts constituting the organism are simply subjected to such inevitable factors as fatigue, damage, and consumption). The British Daily Mail reports that the bake moles can survive 30 years, about 10 times of the life time of the other mice. The moles may not be subjected to ageing, and maintain their reproduction capability before death. In addition, the brain tissues are not degraded with the increase of their ages. The bake moles have some special characteristics. Scientists are confused about such characteristics. Such moles are not only cold blood mammals, but also are capable of resisting cancer, taking toxic plants and adapting to the extremely high temperature environment. However, the moles have poor eyesight, and live underground in most of the life. The moles have special disease resistance capabilities, particularly, resistance to the cancer. The bake moles have a long life and live in the underground environment having low oxygen concentration and high carbon dioxide concentration, and have super immunity to the cancer. Therefore, at present, the moles provide a basis for research in cancer prevention and treatment, ageing prevention, and disease treatment.

All the above characteristics possessed by the moles are determined by the environment of the internal magnetic state and the structure of the internal magnetic state of the moles. To sum up, the moles have a stable living environment, a small activity range, and less natural enemies; the energy of the biomagnetic field in the internal magnetic state is great, an effect is caused by the interaction between the biomagnetic field in the internal magnetic state and the biomagnetic in the external magnetic field. By means of regulation, commanding and control in the spirit level, the biomagnetic field in the internal magnetic state can recover simply and become smooth, such that the characteristics of cancer resistance and long life time of the moles can be achieved.

### Sentiment and Health

Sentiment is a generic concept of a series of subjective recognition and experience, and is a psychological and physiological state generated collaboratively by a plurality of feelings, ideas and behaviors.

In people's life, things do not always turn out that way, sometimes frustrations or confusions. Setbacks in the career, conflicts in the family, and collisions in the interpersonal relationship are very common for people. Without counseling and guidance, the internal conflicts of the people may push them into such psychological issues as depression, panic, anxiety, and distress, and thus psychological barriers are caused and relevant imprint may be caused on the people. In addition, imbalance between different types of emotional activities may exert great impacts on the mental and physical heath of the people. Optimistic and happy psychological moods effectively enhance the mental and physical heath. On the contrary, pessimistic and unhappy psychological moods cause damages to the metal and physical health, thereby inducing diseases.

Huangdi's Internal Classic of the Chinese traditional medicine says that "Five depots generate five Qis, thereby generating happiness, anger, sadness, worry, and fear. Therefore, the happiness and anger damage Qi, hotness and coldness damage the form; extreme anger damages Yin, and extreme happiness damages Yang."

The scientists have proven that pessimistic activities of the brain is directly associated with the weakening of the immunity. If a person is always thinking about the unhappy things, there is a high probability that he or she suffers from diseases. This is a conclusion reached by the scientists in University of Wisconsin in the United States. Sentiment exerts a very important impact on the tissue system of an organism, and a person's sentiment may determine his or her health condition. We learn about the tissue of the organism through the brain, and the emotion is closely associated with the health of the people.

The study indicates that people whose emotion is unstable is not healthier than people who have a strong responsibility. Empirical openness generally refers to more extensive tolerance in the aspect of recognition, and easy acceptation of new view points. People who have such personality characteristics generally have a long life time. This is mainly because they are more creative, and the creative thinking mode helps to deal with the psychological pressure and maintain the health of the brain. Creativeness helps to maintain the health. A possible reason for such conclusion is that creativeness associates many neural networks in the human brain.

Nicolas Tourneau, from the Medicine Center of the University of Rochester, says that "Those who are creative generally deem the pressure as challenges rather than barriers, and hence they will actively overcome the pressure." Although research has been made to those who are naturally frank, i.e., those who have the personality of empirical openness, the results also show that more creative thinking relives the psychological pressure, exercises the brain, and improves the health level of the bodies.

According to the study made by Zhu Qiuling, the mental factors play an important role in the occurrence, development and prognosis of the cardiovascular disease, and maintaining good mental state is an effective way to prevent the cardiovascular disease, which not only complies with the theory that Qing and Zhi cause diseases, but also coincides with the biological-psychological-social medicine mode in the modern medicine.

Zhang Yulan and Wang Zongrong reveal that any activities including recreation and sports, and artistic appreciation can promote mode stabilization of the patient, elimination of loneliness, and improvement of symptom; and further help the patient to enhance the social adaptability and return to the social life.

Li Fangxia's study reveals that when a person is in a bad mood, in poor spirit, and do not want eat anything, even if he or she eats something, he or she may feel distention in the stomach, sometimes feel slightly aching. Some person will have such symptoms as dizziness and insomnia. In a good mode, a person have good appetite. Accordingly, changes of the mood directly affect variations of functions of the organs of the human body, most obviously the stomach and intestine. In practice, variations of the functions of the stomach and intestine are like a mirror which reflects changes of a person's mood.

Zhang Ruiqi reveals that anger is a tense mood generated when a person encounters frustration, and covers non-satisfaction, anger, exasperation, resentment, irritation, and rage. If a person often gets angry without any cause, people around the person would feel that the person is not reasonable and not mature. Getting angry not only affects interpersonal relationship, but also causes damages to the health, event causing diseases. This is because that the autonomic nerves of a person become excited when he or she is getting angry, such that the heart rate and blood pressure thereof increase. Therefore, those who always getting angry are easily subjected to high blood pressure, coronary disease, and malfunction of human immunity.

Studies made by Zhuang Qianling and Guo Guifang reveal that anxiety helps to identify the early-stage diabetes, which, is however, unfavorable to the subsequent life quality management. Therefore, anxiety causes two-way impacts to the diabetes patients. During nursing of the diabetes patient, observation and analysis on the mental and psychological factors of the patient needs to be conducted, to provide a basis for early identification of the diabetes. In addition, corresponding preventative measures shall be taken, to facilitate management of the life quality of the patient in anxiety.

Yang Kun and Li Zunqing et al. have studied that patients with chronic obstructive pulmonary disease are subjected to some mood disorders, and decline of life quality. The nursing workers shall timely apply psychological intervention to eliminate the mood disorders and improve the life quality. The nursing workers make family interview and nursing intervention, which are favorable to recovery of the mental and physical health of the patient.

With respect to the issues on how the mood affects the health and what the mechanism of such affection is, we have conducted many experiments to reveal how the mood affects the health. It has been identified that a positive, smooth and happy mode is favorable to treatment of a disease and recovery of the health, and improvement of the immunity; and a negative, anxious and bad mode affects the mental and physical health and increases the incidence of the diseases. As seen from the above examples, the positive factors achieve the effect of smoothening, and the negative factors cause blocking.

According to the life operation law of a normal living organism, maintaining orderliness and smoothness of the biomagnetic field pathway in the internal magnetic state of the organism, maintaining effective and accurate control in the spirit level, and providing supply of the energy and substance information desired by the life activities of the living organism are mandatory factors for keeping the health of the organism.

A mood is generated during the interaction between the internal magnetic state and the external magnetic state of an organism, and is a judgment before the interaction, a feeling during the interaction, and an assessment after the interaction. After moods are generated, different modes cause different degrees of impacts to the spirit level of the organism. Due to different degrees of impacts caused to the spirit level, different impacts are caused to the biomagnetic field in the internal magnetic state of the living organism. For example, with respect to getting extremely angry, to maintain the life activity of getting angry, the living organism will call more related energy and information thorns promoting the anger. In addition, the living organism also needs to call more energy to ensure operation of the life activity of getting angry in the biomagnetic field pathway in the internal magnetic state in the spirit level, which result in that more energy and substance information needs to be called in the spirit level of the living organism in the internal magnetic state to ensure the life activity of getting angry. In this case, the spirit level applies the main regulation and control functions to the process of getting angry, which, however, reduces inhibition on the other parts of the living organism. Moreover, the internal magnetic state in the spirit level needs to call more energy and substance information, and as a result, the other parts of the living organism which participate the calling process is burdened. In this way, the coordination and regulation capabilities of the living organism are lowered, and thus the living organism is more simply subjected to impacts of the biomagnetic field in the external magnetic state. In other words, getting excessively angry is caused because during the interaction between the internal magnetic state and the external magnetic state, the biomagnetic field pathway in the internal magnetic state is blocked, and no exchange is achieved between the control information and the feedback information. Since the exchange and transmission of the information is severely blocked, more exchanges of the information are subjected to imbalance. Therefore, in the spirit level, more energy and substance information need to be called to smoothen the blocked magnetic field pathway in the region, such that the control function of the spirit level over the other parts is lowered. Accordingly, the living organism is more simply subjected to the impacts caused by the biomagnetic field in the external magnetic state. However, properly getting angry makes the magnetic field pathway in the internal magnetic state in the spirit level more smooth and orderly. In the process of the interaction between the internal magnetic state and the external magnetic state, since some factors may cause the magnetic field pathway in the internal magnetic state in the spirit level to be blocked, release of motion is needed by means of getting angry. In this case, getting angry is an important factor (hypermagnetic state) for self-recovery of the biomagnetic field pathway in the internal magnetic state in the spirit level. Similarly, excessive happiness and suitable happiness have the same effects.

Therefore, variation of the mood within the normal range is good to health, as s long as it is favorable to self-recovery of the biomagnetic field pathway in the internal magnetic state in the spirit level, as long as the caused result is hypermagnetic, and as long as the blocked magnetic field pathway is smoothened. To sum up, the mood needs to be properly managed, without letting the mood develop as it goes on, and without restricting normal presentation of the mood.

In addition, it should be noted that out-of-control of the mood is also a condition to trigger the pathological changes and ageing of the living organism.

Transient refers to occasional accident that is inevitable in the course that a primary life evolves to a eucaryotic life. The gender of a living organism is determined at the instant of combination of sperm and ovum, and the individual differences between the living organisms are determined upon combination of sperm and ovum cells. Under certain conditions, normal cells tend to develop towards tumor cells. Transient also involves flashing of the inspiration in the brain, accumulation of energy, and quantitative changes to qualitative changes.

### Variation

As we all known, the gene controls the shape and property. However, it needs to further explore what controls the gene. Maintaining stability of the gene maintains uniformity of the species and differences of the individuals.

Variations refer to differences of the offspring and their parents. The difference between individuals of the offspring is an attribute of the living organism. Twins refer to the case where an ovoviviparous animal gives birth to two individuals in one pregnancy. The twins generally involve identical twins and non-identical twins. The identical twins refer to that two fetuses develop in the same fertilized egg, and the non-identical twins refer to that two fetuses develop in different fertilized eggs.

The following part describes in detail why the offspring developed by means of combination of an egg from the same female parent and a sperm from the same male parent have differences. Using the twins as an example, the identical twins have fewer differences than the non-identical twins do. This involves the transient process that has been described above. The identical twins are produced in the transient course, whereas the non-identical twins are respectively produced in two independent transient courses.

Genetic stability is a basis that one species distinct from another species. Such stability is achieved by interaction between the internal magnetic state and the external magnetic state in the development course of a species. The internal magnetic state acquires from the external magnetic state substance, energy and information desired to overcome the external magnetic state. In this course, an internal magnetic state having a structure and biomagnetic field pathway that is not subjected to impacts of the external magnetic field and capable of recovering by means of self-regulation is gradually formed in this course. During the interaction between the internal magnetic state and the external magnetic state of various species, in one aspect, the stable structure and orderly and smooth biomagnetic field pathway of the living organism are maintained by means of self-regulation and recovery (in the spirit level). In another aspect, normal life activities are maintained by means of adaptation to the external magnetic state. The external magnetic state is a factor that is ever changing in the development course of the living organism, and in addition the external magnetic state is indispensable for the internal magnetic state. Therefore, the internal magnetic state, in the stable structure and orderly and smooth biomagnetic field pathway, changes with the change of the external magnetic state. Such change is to better complete the life process, and to make the biomagnetic field pathway upon the effect of the external magnetic state more orderly and smoother. Therefore, variations are present anytime. This simply interprets differences of the individuals of the offspring of a species. In the production process of the reproductive cells, since various factors (disease, ageing, mood, temperature, smoothness of the biomagnetic field pathway or not) and various factors of the external magnetic state outside the living organism are different with the change of the time, the produced reproductive cells are also produced under effects of the internal and external factors of the living organism. In addition, in the course of the combination of the reproductive cells, the internal and external factors of the living organism are different. This is why the twins have fewer differences.

During the growth and development course of a living organism, the inheritance is relative, and variation is absolute. In the traditional genetics, variation does not have directivity and is general. By means of firstly massive reproduction, and then natural selection, the species are evolved. In practice, the reality is not as such.

Especially under circumstances where the control of the external environment is more and more fine, expression of the gene is subjected to less and less impact caused by the external environment (when the air supply is sufficient, the temperature and humidity stability is controllable, the illumination strength is controllable, and the supply of organic and inorganic fertilizers is sufficient, the plants in the growth may not be very thin and short dye due to the rigid environment, but the normal expression of the gene of the plants). Stability of the environment ensures stability of the inheritance and better expression of the gene in one aspect, and provides a more beneficial development trend for the variation in another aspect. Stable expression of the environment ensures that the animals and plants all grow, develop, and reproduce in a stable environment, thereby achieving better growth, development and reproduction.

During the reproduction process of the living organisms, reproduction periods of many living organisms may be in the season having stable environment condition and mild climate. This provides a more stable external magnetic environment. The reproduction process covers three periods, preparation period, combination period, and growth period. In the preparation period, the parents may select the environment. Generally, the parents will select an environment suitable for the species, including suitable temperature, humidity, illumination, nutrition substance origin such as abundant food and water, fewer dangers, and relatively stable earth magnetic field. In such environments, the biomagnetic field pathway in the internal magnetic state of the organisms of the parents, under self-regulation and control of the internal magnetic state and the external magnetic state in the spirit level of the organisms, is gradually repaired and smoothened, to finally reach orderliness and smoothness of the biomagnetic field pathway in the internal magnetic state, such that the external energy, substance and information taken in smoothly reaches the cells, tissues and organs of the organisms. In this way, an internal magnetic state of the organism having a relatively stable structure, a smooth and orderly pathway, and a accurately and effectively controllable spirit level is formed (with respect to the spirit level, the impacts to the spirit level such as the mood and emotion of such high level animals as human beings should be noted). When the organisms of the parents stay in this state and environment, the reproductive cells produced thereby are subjected to smaller and smaller impacts from the external environment. Production of these reproductive cells is also subjected to the entire internal magnetic state of the organisms. Therefore, stable, orderly and smooth internal magnetic state, and environment-suitable and stable external magnetic state creates a suitable external magnetic state environment for the production of the reproductive cells. Finally, the produced reproductive cells have stable structure, orderly and smooth magnetic pathway, and have the characteristics of accurate and effective control in the spirit level. To be specific, the biomagnetic field energy in the internal magnetic state is enhanced. This provides a basis for combination of sperm and ovum. In the process of the combination, the reproductive cells having stronger biomagnetic field energy in the internal magnetic state generated in the previous period (the preparation period) are produced. In addition, to bring out a more powerful living organism upon the combination of sperm and ovum, an environment which is suitable for birth of a new living organism through transient is desired. When chromosomes of the parents are associated, crossed and exchanged, the mutation probability is high. Therefore, it is very important to provide a biomagnetic field in the external magnetic state and the entire biomagnetic field of the organism that are more stable and smoother. In this case, this provides a sufficient condition for the transient process because these determine the occurrence of the transient, for example, gender of the offspring, and differences between the offspring. In the growth period, growth and development of a new life is subjected to no obvious impact of the external magnetic state, and thus factors for causing the transient process are relatively reduced. Study on the essence of the heredity and variation of the organisms facilitates aristogenesis of the human beings and cultivation and reproduction of the animals and plants. (Note that human beings and animals all select a stable environment and a mild season during the reproduction period, which is favorable to better development during combination of the gene segments).

After the raised white mice are fed with a calculus-causing chemical agent, impacts caused by the raising with magnetized water and common water to formation of calculus are eliminated. Through the observation, it is found that only 26.5% of the white mice fed with the magnetized water are subjected to calculus, and on the contrary, 83.3% of the white mice fed with the common water are subjected to calculus. This experiment clearly indicates that the magnetized water exerts better effects on suppressing the formation and growth of the calculus. Although many experiments and observations have shown that the magnetic water has better effects on the growth of the some crops and raising of the animals. With intake of the magnetized water, the magnetized water is transported to various parts of the organisms via absorption. The magnetized water herein achieves two functions. When the magnetized water is transported to the kidney via absorption, the water around the kidney is substituted by the magnetized water. The solubility of the magnetized water is greater than that of the common water. Therefore, a physical solution effect is achieved on the calculus of the kidney. In this way, the formed kidney calculus is dissolved and then eliminated. In addition, the magnetized water enters the circulation system of the organism, and can thus be transported to the adjacency of any cell of the organism. As such, the biomagnetic field energy in the external magnetic state of the organism cells may be improved and act on the biomagnetic field in the internal magnetic state of the organism cells. The entire interaction between the internal magnetic state and the external magnetic state of the organism facilitates promotion of orderliness and smoothness of the biomagnetic field pathway in the internal magnetic state of the organism. Accordingly, the transmission and exchange of the substance, energy and information is more orderly and smoother. Therefore, thanks to the effect of the magnetized water in the kidney, the biomagnetic field pathway in the internal magnetic state in the kidney are more orderly and smoother, such that the calculus is hard to form in the kidney and the formed calculus is discharged out of the body.

The hypermagnetic states acts on the internal magnetic state in the form of substance, energy and information running in the biomagnetic field pathway in the internal magnetic state. The major functions of the hypermagnetic state is: enable the hypermagnetic state to enter the biomagnetic field pathway in the internal magnetic state in the form of substance, energy and information, such that the blocked and isolated biomagnetic field pathway in the internal magnetic state restores the orderliness and smoothness, the damaged internal magnetic state structure is repaired, and the substance, energy and information taken in by the internal magnetic state is converted and used, so as to hazardous substances are quickly and effectively discharged (including excretion and transformation). Many medical studies have been made on this aspect currently, for example, pharmacotherapy, dietotherapy, psychotherapy, antilepsis, seeing witch doctors, praying Buddha for help. The dietotherapy can directly provide substance, energy and information desired by the organism, and can inject such drugs as antibiotics into the organism to suppress the growth and reproduction of the etiology inducing the diseases of the organism, such that the factors damaging the structure of the organism and blocking the biomagnetic field pathway of the organism are suppressed. Although the pharmacotherapy achieves a positive effect within a specific period of time, for example, killing and controlling a part of bacteria and viruses, during administration of the drug, some drug-resistant bacteria are generated, for example, superbugs, resistant Staphylococcus aureus (for example, drug resistance to the corresponding antibiotic is generated by the bacteria via adaptation and evolution, that is, the bacteria cause the biomagnetic field in the internal magnetic state thereof to interact with the magnetic field in the external magnetic state of the corresponding drug, such that the structure of the internal magnetic state of the bacteria changes, and the biomagnetic field pathway in the internal magnetic field changes accordingly. In this way, under control of the spirit level of the internal magnetic state of the bacteria, under circumstances where the antibiotics is present in the external magnetic state, the biomagnetic field pathway in the internal magnetic state operates orderly and smoothly. In addition, the magnetic field energy of the antibiotics may be absorbed. Particularly, through multiple effects of the magnetic field in the external magnetic state of the antibiotics, superbugs resistant to multiple antibiotics are generated. To be specific, upon interactions between multiple internal magnetic states and multiple external magnetic states, the internal magnetic state capable of absorbing the magnetic field energy in the external magnetic state of multiple antibiotics is generated. In another aspect, the bacteria stays in the organism, and the bacteria and the organism cells both pertain to cell structures. Although the bacteria is the prokaryote, and the organism cells are eukaryotes, they are similar in terms of structure. The internal magnetic state of the bacteria is subjected to the effects caused by the magnetic field in the external magnetic state of the organism cells around. The biomagnetic field in the internal magnetic is compatible with the external magnetic field of the bacteria, i.e., is subjected to the magnetization effect of the magnetic field in the external magnetic field for a long period of time. The magnetic state of the bacteria in the internal magnetic state tends to be the same as the biomagnetic field of the organism cells in the external magnetic state. Since the biomagnetic field of the bacteria tends to be the same as the biomagnetic field of the organism cells, the bacterial may not cause activation of the immune system of the organism, but results in an immune reaction for the bacteria. Therefore, due to these two effects, superbugs are formed. In one aspect, the superbugs are difficult to be suppressed and killed, and in another aspect, the superbugs are difficult to be identified by the immune system and killed by the immune cells of the organism. In addition, a portion of mutated new virus strains are propagated, for example, bird flu H5N1. This causes a great difficulty in controlling inflection and propagation of such diseases. In some nutritional diseases, pharmacotherapy and dietotherapy are both effective. However, after intake of the drugs stops, the symptom recurs, and the disease fails to be fundamentally cured. For example, since the synthesis or absorption mechanism of the organism is blocked, the biomagnetic field pathway of the synthesis or absorption mechanism needs to be cleared and smoothened, such that the substance, energy and information operates in the biomagnetic field pathway in the internal magnetic state orderly and smoothly under control of the spirit level. In this way, stability of the entire structure of the organism is ensured, and as such the nutritional diseases can be fundamentally solved.

With respect to compatibility of the magnetic field: 1. The internal magnetic states having the same structure are simply subjected to magnetic field compatibility. For example, the biomagnetic fields of the identical twins are very similar, and less reject reaction is present in the transplant of organs or tissues thereof. 2. The internal magnetic states having the same structure or similar structures are in the (bio)magnetic field in the external magnetic state in the same environment. After action for a period of time, these internal magnetic states having the same structure or similar structures exhibit the same or similar biomagnetic fields. For example, the biomagnetic fields of the identical twins are very similar, and less reject reaction is present in the transplant of organs or tissues thereof. Still for example, with respect to a couple living together for years, although they are different in structure, they live in the same external magnetic state in the same environment and have the same diets and life styles, in addition to their communications, finally, they are subjected to magnetic field compatibility. Some cases show that transplant of organs between couples is not necessarily subjected to any rejection. 3. One or (a plurality of) internal magnetic states are placed into a plurality of external magnetic states (in the case where the number of external magnetic states is greater than the number of internal magnetic states) which is absolutely different from the biomagnetic field in the internal magnetic state but has the same structure or similar structures. In this case, the biomagnetic field in the internal magnetic state tends to become the biomagnetic field in the external magnetic field. That is, the biomagnetic field in the internal magnetic state is magnetized such that the biomagnetic field in the internal magnetic state tends to be the same as the biomagnetic field in the external magnetic state, for example, the superbugs as described above. 4. The internal magnetic states having similar structures and having the similar biomagnetic fields are more simply subjected to magnetic field compatibility, for example, falling in love with each other at one sight, feeling between lovers and friends, just like the old saying "birds of a feather flock together".

The biomagnetic field pathway in the internal magnetic field: The orderliness and smoothness of the pathway varies with different life stages of the human bodies, animals or plants. Some pathways may be closed at a specific stage depending on the living demands. Most of the pathways are degraded with the degradation of the mechanism of the organism. Therefore, the transmission and exchange of the substance, energy and information is not very smooth, thereby causing blocking and isolation.

In the dormant state (stage) of the living organism, when the cells of the living organism fail to enter the inoculation state without suitable conditions, the biomagnetic field pathway of the cells need to be temporarily closed, to reduce the reaction to the effects caused by the magnetic field in the external magnetic state, and reduce the impacts and communications of the biomagnetic field pathway of the cells caused by the magnetic field in the external magnetic state. In this case, the substance, energy and information operating in the biomagnetic field pathway of the cells is in a sleeping state, and the control in the spirit level is also in a dormant state.

In the inoculation state (stage) of a living organism, the structure of the internal magnetic state and the biomagnetic field pathway in the internal magnetic state are established. In this stage, the environment of the biomagnetic field in the external magnetic where the internal magnetic state stays is relative stable. In the infant state (stage) of the living organism, the structure of the internal magnetic state and the biomagnetic field pathway in the internal magnetic state grow. In the teenager state (stage), the structure of the internal magnetic state and the biomagnetic field pathway in the internal magnetic state are improved, wherein the biomagnetic field pathway is very orderly and smooth, and the magnetic flux in the biomagnetic field is very great, that is, operation in a full load. In a juvenile state (stage), the structure of the internal magnetic state and the biomagnetic field pathway in the internal magnetic state upon improvement interact with the external magnetic field to form a relatively stable structure and pathway. In the middle age state (stage), the structure of the internal magnetic state and the biomagnetic field pathway in the internal magnetic state that are formed are maintained. In the aged state (stage), the structure of the internal magnetic state and the biomagnetic field pathway in the internal magnetic state that are stable are degrading.

The amount of the substance, energy and information transmitted in the biomagnetic field pathway in the internal magnetic state is referred to as magnetic flux in the biomagnetic field, called flux for short. The flux of the biomagnetic field pathway in the internal magnetic state that is in the most orderly and smoothest state is called a standard flux. The factors affecting the magnetic flux of the biomagnetic field includes: the external magnetic state, orderliness and smoothness of the biomagnetic field pathway, control in the spirit level, and internal magnetic state associated. The external magnetic state may affect orderliness and smoothness of the biomagnetic field pathway in the internal magnetic state via the (bio)magnetic field thereof, and the magnetic flux of the biomagnetic field increases in case of positive action, and decreases in case of negative action.

### Factors affecting the control in the spirit level

The sleep is the spontaneous and reversible tranquillization state that periodically appears in higher vertebrates, and is presented that the response of the organism to external stimuli is decreased and the consciousness is temporarily interrupted. About 1/3 of the time is spent in sleeping during people's life. When people are in the state of sleeping, the brain and body can get rest and recovery which is favorable to daily work and study.

Sleep is the interaction of the magnetic state of a living body bio-magnetic field and the external magnetic field between organisms down to a very low level, the equivalent of a temporary relatively closed state, is substantially closed state, within the magnetic field and the external magnetic state of the biological condition of biological door between the magnetic field in the opposite closed temporarily, the spiritual state of bio-magnetic field within the magnetic "connection", it is just a word here, a connection image representation, meaning that after the body goes to sleep in the magnetic state of biological relative to the external magnetic field at the state in a substantially closed state, and then the relationship between the formation of a state within the magnetic field and the body spiritual birth, bio-magnetic field within the magnetic state under the control of spiritual rest and recovery, the body influence within the state and outside the magnetic interaction of the magnetic state of sleep this process through the body to rest and recover, in order to reach an ordered state body biological magnetic field flow passage between the outer magnetic state interact with the process of struggle, it can accurately effectively a substance the body needs a quick and timely delivery to the premises, but also the waste throughout the body to produce timely and effective transport exclude transformed into the body. The organism performs a timely rest and recovery through the sleep, and meanwhile enables the irreversible influence caused by the biomagnetic filed of the internal magnetic state generated by the cumulative effect of the internal and external magnetic states to be reduced or to be disappeared. The sleep process also includes self-clear and repair of the biomagnetic pathway of the central nervous system in the spirit level. The external magnetic state during the sleep is various external environments (such as, earth magnetic field, universal magnetic field, ambient magnetism, light, temperature, oxygen content, surrounding emotion environment, and the like) that need to be borne in a healthy organism. In a non-healthy organism, besides of the external environment, the external magnetic state includes various factors (pathogenic bacteria, viruses, cancer, accumulated autologous excretion or decomposition, and the like) that cause the organism functionality to be changed and to be affected. The relationship between the internal magnetic state and the external magnetic state should be noted in the process of sleep, and the environment that the influence of the external magnetic state is weak is selected as much as possible, (such that the case where the strong external magnetic state biomagnetic field affects health of the organisms is prevented).

If the internal magnetic state having each biomagnetic field and composing of the living body is regarded as a small magnet each having its running rule; and the external magnetic state composed by various external environmental factors is regarded as different magnets acting around the body; these magnets stay in each direction of the body, all around the space at any point, and act on the body by randomly changing at different intensities; in this case, the small magnet is not only subjected to the magnet effect from different directions at different intensities, but also subjected to the action of the small magnet around the body, and the actions of a whole body, and the magnetism of each part. Therefore, under the action of these different magnetic forces, the normal running rules and features of the small magnet are affected (including the impact of the relative magnetization). For example, a compass is placed in Earth's magnetic field, and the compass points at the south under the influence of the earth magnetic field, but when a magnet with stronger magnetism (magnetic induction intensity) is placed, the direction of the compass will change; after the magnet is placed for a certain time, the magnetic induction intensity is much higher than the compass, and then the external magnet is removed, the direction of the compass will not point at the south again; how to store the original direction of the compass to point at the south that needs a re-magnetization of the compass. In the living body, the repair of the internal magnetic state (small magnet) affected by the external magnetic state during the process of sleep requires the control in the spirit level; if the body's self-repair can not restore the internal magnetic state affected by the external magnetic state, the assistance of the hypermagnetic state is needed.

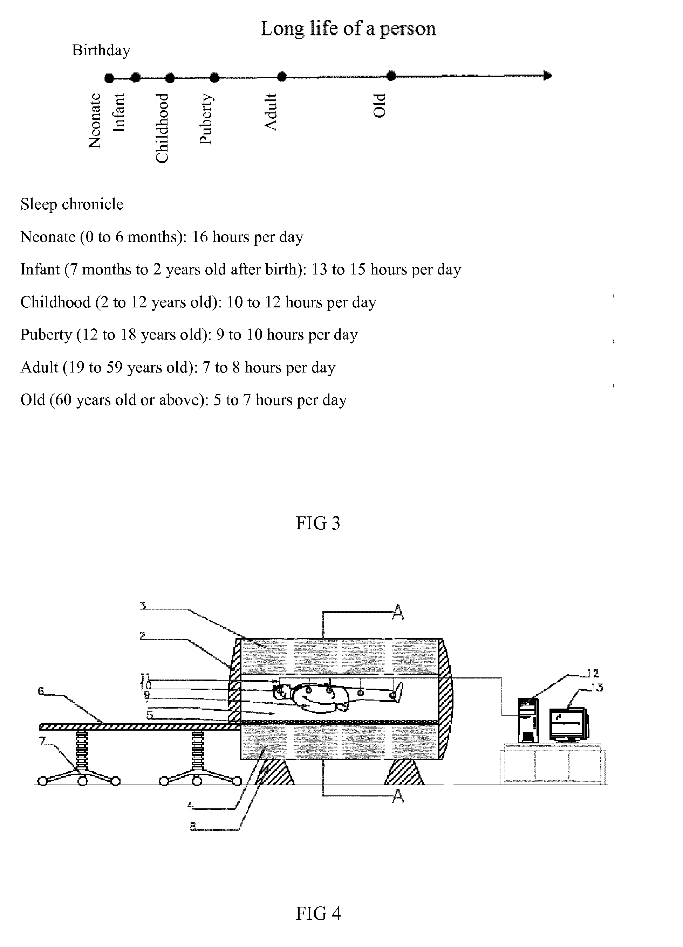

The quality of the sleep is also affected by the internal and external magnetic states; when the biomagnetic field pathway of the internal magnetic state is blocked (such as illness, pain, ageing, or the like), the quality of the sleep is affected; when the body is in a new biomagnetic field of the external magnetic state or a in a unstable biomagnetic field of the external magnetic state, the quality of the sleep is also affected. For example, the time of the sleep is greatly reduced at the age of 45; for a healthy young man, the time of the fast sleep is generally 100 minutes during eight hours of sleep every night; however, at the age of 45, the time of the fast sleep is reduced to only a quarter, or even 5 to 10 minutes. The sleep chronicle is illustrated in FIG. 3, it can be seen from FIG. 3 that the more the organism in the period of growth (from infancy to adulthood), the longer the average sleep time. During the period of the growth, the assimilation of the organism is greater than catabolism, the number of cells of the organism increases, and the weight and height also increase. The entire period of the growth is also the process of completing stable structure of the magnetic state of the organism, and orderly and smooth biomagnetic field pathway. During the process, the organism must stay in the environment with little influence by the external magnetic state; and the action and exchange with the external magnetic state also need to be reduced. In this way, a more stable internal magnetic state structure and more orderly and smooth biomagnetic field pathway can be built under the control of the spirit level of the organism; the "imprint" that the external magnetic state acts and remains can be reduced, and thus healthy and normal development of the organism can be ensured. However, during the adulthood, the assimilation of the organism is equal to the alienation, and the metabolism of the organism also achieves a relatively stable equilibrium. Therefore, the sleep time of the organism tends to stability (typically 8 hours), and the spirit level of the organism mainly maintains stable internal magnetic state structure of the organism and orderly and smooth biomagnetic field pathway at this time. At the old age, the assimilation of the organism is less than the alienation, and the metabolism of the organism also achieves a relatively declined process. With occurrence of the ageing, partial diseases come, which affects stable internal magnetic state structure of the organism and orderly and smooth biomagnetic field pathway. At this time, the control of the spirit level of the organism to the biomagnetic field of the internal magnetic field of the organism reduces which causes the sleep time of the organism tends to be reduced.

Sleepwalking (hallucinations): In the spirit level, the more energy is exceeded after recovery.

Experts believe that they are in a state of half sleeping. Dr. Triplitez has spent 10 years on the question, he says that the moving organs of the sleepwalkers are awake, but the sensory organs fall asleep, at least partially falling asleep; in other words, the sleepwalkers are capable of walking and work in the state of the sleep, but they do not know what they are doing.

Professor Chrispton from St George's, Hospital of London has recently proposed a view, and he believes that the sleepwalkers actually are awake, but their brains is in a "split state"; in this state, the intact function of the brain is blocked, but some thinking processes of the brain continue. When people are severely depressed in spirit, he will try to release the depressed emotion by using the divided state of the brain after falling asleep. It is regarded as a protective mechanism for the spirit of the depressed people.

The patients at "sleep disorder specialist outpatient" of the George Hospital get a series of routine personality examination. From the inspection results, it is found that there is no significant difference for many indicators between the sleepwalkers and the normal person in these examinations. However, in a special project inspection, certain checking indicators of the sleepwalkers is high, and these people show the personalities of loving fun too much, restless, and pushy. These personalities are evidently presented in the person suffering from personality schizophrenia, the person who easily wakes up from a deep sleep, and the sleeper in a panic state. It indicates that there is a link between schizophrenia patients and the sleepwalking patients.

Sleepwalking occurs mainly because the body during sleep, the recovery on the internal magnetic outer magnetic state due to the normal state. Because of the role of various factors (the characteristics of the body's spirit structure, or because of the body growth and development process caused by the effect of external magnetic state of spiritual structural change, or because of certain chemical substances such as alcohol cause temporary changes in the structure of the spiritual body), a portion of energy in the spirit level can not be repaired or released (information) via sleep. This portion of extra information has restored in structure the operation of the biomagnetic field pathway in the spirit level, such that the living organism is subjected to sleepwalking or subjected to awakening from a deep sleep (for example, schizophrenia).

This body's self-repair by biomagnetic sleep (basic off) function, through the efforts of human long-term exploration and practice, has been used in traditional health and other therapies, such as: qigong, yoga, hypnosis, through the autonomic nervous system. Under the regulation, targeted part of the body is repaired by itself in the sleep state, such that more substance, energy and information is called under the command and control to reach parts of the organism where necessary. In this way, sufficient substance, energy and information desired for repair of the biomagnetic field in the internal magnetic state in this region is provided. In addition, the energy in the biomagnetic field of the surrounding cells, tissues and organs in the region is increased. Under the action of magnetic compatibility of the powerful biomagnetic field of the surrounding cells, tissues and organs, it is more simply that the biomagnetic field pathway is repaired or self-recovered.

Applications of the principles of magnetic recording (principles of the tape or hard disk): how to reproduce our daily lives, just like the case where there are equipped many cameras around us, and the pictures may be taken from the computer when necessary. For example, in American movies, the incidence taking place in the past is reproduced. Such scenes in the vision or imagination will come to reality under support of our theory, just like voice recoding via the tape. Each of our activities in the past may be "recorded" in the external magnetic state environment where we are. This external magnetic state environment includes the Earth's magnetic field, universe magnetic field, and also includes homes, offices, restaurants, and public places where we usually stay. In such places, "videos" of our activity may all be recorded. With respect to such "recording", for example, a permanent magnet is placed in a magnetic field for a specific period of time, and then the magnetic field is moved away; and in this case, the permanent magnet still has some remaining magnetic force. The cassette recorder employs such principles. At present, the research on the biology and the magnetism mainly focus on the effects caused by the Earth's magnetic field, magnetic field of the ambient environment, or human applied magnetic field to the life activities of the living organism. However, less research is conducted on the effects of the biomagnetic field of the living organism to various surrounding magnetic fields. We just start our invention from this aspect. The research subject is that the biomagnetic field of the living organism acts on the surrounding magnetic field, and causes the surrounding magnetic field to be subjected to corresponding changes. In the life activities of the living organism, during the interaction between the biomagnetic field in the external magnetic state and the internal magnetic state of the living organism, impacts are not only caused to the internal magnetic state but also caused to the external magnetic state. Such impacts are tiny and unperceivable, and no more studies are made on such impacts. Such impacts are characterized by tiny changes of the external magnetic state. The track of the existence of the internal magnetic field is stored via such tiny changes. Corresponding to changing and mobility of the life activities of the living organism, the external magnetic state is movable relative to the internal magnetic state. Therefore, through changes of such tiny impacts, life activities of the internal magnetic state of the living organism in the external magnetic state are recorded in the biomagnetic field in the external magnetic state, just like the case where the cassette recorder stores the sound. However, we have not developed a "projector" that is capable of projecting the information in the biomagnetic field in the external magnetic state. Many phenomena indicate that the living organism itself is a good "projector". For example, the ghost does not go away, the ghost is haunting (the human body is possessed by spirit). Such phenomena are in a critical region between the science and superstition. When the biomagnetic field pathway of the organism is severely blocked, the commanding, control, regulation and interaction of various parts of the organism in the spirit level are degraded, and the efficiency is lowered, such that the biomagnetic field in the internal magnetic state in the organism is simply subjected to the impacts of the biomagnetic field in the external magnetic state. In this case, the biomagnetic field in the external magnetic state stores information of life activities of other organisms into the biomagnetic field in the external magnetic state. Therefore, when the living organism whose the biomagnetic field pathway in the internal magnetic state is in such an environment, under effects of various other factors, the organism becomes a "projector", such that the phenomenon that the ghost is haunting (the human body is possessed by spirit). Other factors include impacts caused by the emotion of the organism to the spirit level, impacts on the orderliness and smoothness of the biomagnetic field in the internal magnetic state in the spirit level, and compatibility of the strength and type of the information stored in the external magnetic state with the biomagnetic field in the internal magnetic state of the organism. However, when the biomagnetic field pathway in the internal magnetic state of the organism is slightly blocked and the biomagnetic field pathway in the internal magnetic state in the spirit level is severely blocked, the phenomenon that the ghost does not go away will occur.

Dream and self control of the dream. Dream is a passive and random reaction in the process of self regulation in the spirit level when the biomagnetic field pathway in the internal magnetic state changes from blocking to orderliness and smoothness through self regulation and control of the organism. Such reaction is slightly subjected to the automatic nerves of the organism. In the sleep of the organism, the affected internal magnetic states of various parts of the organism gradually recover. The affected biomagnetic field also carries information. When affected the internal magnetic state gradually recover, the information will be released. Release of the information results in dreams. Such information release takes ling time sometimes or takes short time sometimes. Therefore, some dreams will be produced constantly, and some dream will be produced once or last for very short time. Release of the information is also subjected to the external magnetic field in the surrounding environment and the biomagnetic field in the internal magnetic state, for example other factors such as changing place, direction, vibration in the sleep for better sleep, and health degree of the organism (diseases, damages, inflammations and the like), i.e., orderliness and smoothness of the magnetic field pathway of the organism. (whether the part of the memory only contains the brain; whether other areas has the possibility of memory capacity; and a reminder about memory is transiently added).

Dynamic smoothness of the pathway is achieved by means of assistance of the hypermagnetic state (externally-applied magnetic field) to regulate the biomagnetic field pathway in the internal magnetic state (cells).

First, the smooth of the running exchanges of the energy, substance, and information that lie in both sides of cell membrane (biomembrane) must be kept. The study shows that the applied magnetic field can affect the permeability of the ions (such as, Na +, K +, Cl, and the like) to the biomembrane, and then affect metabolism in the inner side of the loving bodies, biochemical processes and membrane potential, and the like. Many experimental observations show that the magnetic field exerts effects on various cells. The target spot through which an extremely-low frequency and low-intensity magnetic field acts on the cell is firstly the cell membrane. The experiments and studies show that the cell membrane plays a main role during the reaction of the living organisms to electromagnetic field. The initial site of the electromagnetic field is the cell membrane, and enables protein molecules on the membrane surface to produce electrophoretic effect. Through changing the charge distribution on the membrane surface, regulating ligand binding, activating signal transduction systems, and affecting the electrical properties of ion channels on the cell membrane, finally, cell life activities are changed. The nerve physiology laboratory of the Bordeaux University in France finds that the permeability to calcium ion by the cell membrane increases, and the concentration of the calcium ion increases at a 50-Hz 1-mT sinusoidal magnetic field. Ottaviani and Rosen studied the influence of 50 Hz power frequency electromagnetic fields and 125 mT static magnetic field on membrane ion transmembrane transport capacity; Jie-FeiShen and others observed changes of transient outward potassium current and delayed rectifier potassium current with the 125 mT static magnetic field (SMF) acting on the excited nerves of the root of the trigeminal nerve in rats, and found that the current of the irradiated group has a slight change over the comparison group, and 125 mT static magnetic field can affect the inactivation mechanical characteristics of the two types of the current by changing the inactivation rate, and the activation parameters did not change significantly. These findings suggest that membranes are subjected to deformation at the influence of a moderate intensity SMF, and the hypothesis that the physiological characteristics of the ion channels on membranes are also affected is valid. Adair believes that the magnetic field which the frequency is less than 200 Hz and the amplitude is approximately 50 µT can change the activities of ions through the cell membranes. Some people argue that straightforward mutual inductance between the ions and fields is very weak. However, KW Wang acquired a conclusion in 1994 that the influence of the static and low frequency magnetic fields on the gramicidin channel has been recorded by patch clamp experiments, and 0.3% change of membrane conductance containing a lot of channels can be detected. Through experiments, CLM Baure proves that a suitable combination of the 27 mt-37 mT static magnetic field and the alternating magnetic field with the frequency ranging from 7 Hz to 72 Hz and the amplitude ranging from 13 mT-114 mT can affect calcium ion channel protein on the cell membrane.

For example, the magnetic field will change an electrical activity of nerve cells of rabbits. When plasma cells of rabbits and mice are placed in the non-uniform magnetic field with the intensity 1.46 T, the gradient 0.5 T/cm for vitro culture, it is detected that the growth rates of the rabbits and mice get a significant increase. It is an issue that is worthy of attention and study as whether the different significant effects reflected by the two experiment results are associated with cell category or cultivation manner, or associated with the uniformity of the applied magnetic field. If the vitro cultured S-37 mouse tumor cells is placed in the 0.44-0.8T uniform magnetic field for 18 hours at 37 °C, it is bereaved that these tumor cells through magnetic treatment will get a degenerated denaturation; but if the S-37 mouse tumor cells is put in 0.1 0.2T uniform magnetic field at the same temperature for processing the same time, there is no any noticeable change. The experiment shows that the generation of this denaturation effect requires a magnetic field over a certain strength, i.e., with a specific threshold. It is also found from the experiment that the S-37 tumor cell respiration is greatly subjected to the magnetic field; when the magnetic field is increased from 0.8 mT to 0.73 T, the cellular respiration is changed from a significant excited state to a significant inhibition state. It clearly shows that the magnetic field has a significant influence for metabolic processes of some cells.

### CONTENT OF INVENTION

General description of the invention:
To solve the above technical problem, the present invention is intended to provide application of a drift and suspension device in human bodies, animals or plants.

The application of suspension in human bodies, animals or plants specifically comprises:
A: application of magnetic suspension in human bodies, animals or plants;
B: application of drift and suspension in plant cultivation;

The A: application of magnetic suspension in human bodies, animals or plants comprises the following steps:
1) subjecting the human bodies, animals or plants to a magnetic suspension treatment;
2) measuring dynamic information of the human bodies, animals or plants in a magnetic suspension state; and
3) providing nutrients and/or drugs desired by the human bodies, animals or plants according to a measurement result obtained in step 2).

The desired nutrients and/or drugs in step 3) may be provided when the human bodies, animals or plants in a suspension warehouse, wherein the nutrients and/or drugs are preferably provided via spraying, duct transportation, placing and the like manner; or are provided after the human bodies, animals or plants leave the suspension warehouse.

The B: application in plants comprises the following steps:
1) detecting a state of the plant;
2) defining a planting environment condition according to the state of the plant; and
3) transplanting the plant to an environment with the defined condition.

The present invention further provides equipment for the A: application of magnetic suspension in human bodies, animals or plants. The equipment comprises: a suspension device, a dynamic information processing device, and a nutrient providing device. To be specific, the suspension device subjects a magnetic suspension treatment to the human bodies, animals or plants; the dynamic information processing device detects dynamic information of the human bodies, animals or plants in a suspension state; and the nutrient providing device provides in real time nutrients and/or drugs desired by the human bodies, animals or plants.

The suspension device mainly comprises a suspension warehouse, wherein the suspension warehouse is an airtight box in which a suspension magnet is provided.

The dynamic information processing device comprises an information detecting system configured in the suspension warehouse and a control system configured outside the suspension warehouse, wherein the two systems are communicated with each other via an information transmission line. The information detecting system detects dynamic information of the human bodies, animals or plants in the suspension state, and transmits the detected data to the control system via the information transmission line. The control system analyzes and processes the data, and issues an instruction to the nutrient providing device.

The nutrient providing device provides nutrients and/or drugs desired by the human bodies, animals or plants according to the instruction issued by the control system of the dynamic information processing device.

The present invention further provides equipment for application of drift and suspension in plant cultivation. The equipment comprises: a suspended nutrition trough in a greenhouse, a plant state detecting device, and a plant fixing and moving device. The suspended nutrition trough is fixed in the greenhouse via a rope or a frame; the plant state detecting device detects a state of the plant, and moves the plant to a suitable nutrition trough or suspends the plant via the plant fixing and moving device.

The plant state detecting device detects in real time demands of the plant for moisture, mineral elements, illumination, and oxygen.

The plant fixing and moving device comprises a plant fixing frame or string bag serially coupled to the rope suspending over the nutrition trough.

The present invention further provides a method for treating seawater/sewage water by using the B: application of drift and suspension in plant cultivation.

### Further description of the invention:

The present invention provides application of a drift and suspension device, comprising:
A: application in human bodies or animals, providing a "hypomagnetic state" environment for the human bodies or animals;
B: application in plants, providing a "hypomagnetic state" environment for the plants and converting a passive and relatively stationary growth of the plants into an initiative growth and development pattern; and
C: application in irrigation and/or purification of seawater or sewage water.

The A: application in human bodies or animals comprises the following steps:
1) preparing for a magnetic suspension treatment of the human bodies or animals;
2) formulating an operational procedure of the corresponding magnetic suspension treatment according to vital signs of the human bodies or animals;
3) adjusting the drift and suspension device according to the operational procedure of the magnetic suspension treatment formulated in step 2) to achieve a "hypomagnetic state" environment most suitable for the human bodies or animals;
4) placing the human bodies or animals into the drift and suspension device to conduct the magnetic suspension treatment; and
5) monitoring various life and physiological data related to the human bodies or animals in a magnetic suspension device by using a dynamic information monitoring device, with the data transferred to a data processing center through a dynamic information transmission line, and further establishing the operational procedure of the magnetic suspension treatment upon analysis and research of the data to provide a "hypomagnetic state" environment more suitable for the human bodies and animals by a reality and operation system;

The B: application in plants comprises the following steps:
1) selecting plants required;
2) formulating an operational procedure most suitable to growth and development of the plants and other lower living organisms according to different demands of the plants for substance, energy and information (for example, nutrients, sunshine, dioxide, water, oxygen, humidity, magnetic field strength or the like) in different periods and at different growth and development stages;
3) placing the plants into the device and running in accordance with the procedure formulated so that the plants initiatively absorbs and acquires desired substance, energy and information (for example, for example, nutrients, sunshine, dioxide, water, oxygen, humidity, magnetic field strength or the like) at the most suitable time and growth and development stage, unlike the traditional planting industry in which passive photosynthesis, metabolism, normal growth and development are unavailable unless plants are exposed to sunlight, proper temperature, and rainwater or artificial irrigation; and
4) when the plants and other lower living organisms grow and develop, and are subjected to magnetic suspension treatment, dynamically detecting the plants and other lower living organism, with monitored information transmitted to a computer control center for analysis and research to formulate a "hypomagnetic state" environment more suitable for growth and development of the plants and other lower living organisms, thus controlling the whole device and actively acquiring the desired various substance, energy and information by means of demand information indicated by the plants and other lower living organisms;
wherein the plants initiatively absorb and acquire the desired substance, energy and information at a most suitable time and growth and development stage; since plants and other lower living organisms have different demands for substance, energy and information at different growth and development stages, dynamic information data acquired during a running process of the plants in the device by means of the innovative device of the present invention based on base data related to existing scientific achievements in plants, the base data being substance, energy and information needed for growth and development of the plants, is average data (for example, categories, growth cycle, most suitable temperature, desired water quantity, illumination strength, nutrient demand and the like) substance, energy and information demanded at a certain growth and development period, and the dynamic information data is instant data on the plants and other lower living organisms at a certain moment, both data is converted by the innovative device of the present invention into instructions of substance, energy and information desired by the plants and other lower living organisms at different growth and development stages, and becomes data information readable by human beings and computers, for example, data on substance, energy and information desired by plants at a certain growth and development stage is: temperature of a°C, humidity of b%, illumination of c lux, moisture of d grams, nitrogen phosphorus and potassium or the like of e grams, an organic fertilizer of f grams, light reaction of photosynthesis of g hours, dark reactions of photosynthesis of h hours, magnetic field intensity of i Tesla, CO2 concentration of j%, oxygen component k and the like; such data is substance, energy and information which the plants desire to absorb and acquire at this stage; and such data is sent to a control system of the device in real time for analysis and research, on the basis of which the control system formulates an operation scheme for the plants in the device, generates instructions for commanding various control systems, functional chambers and functional zones of the device, and provides substance and energy desired by a certain plant at this stage; and then the plant is placed, by means of a fixing system of the plant, into various functional chambers and functional zones in real time according to the operation scheme for absorbing and acquiring the desired energy and substance information, thereby forming a set of central systems of the whole device on the basis of demand instructions spontaneously sent by plants and other lower living organisms, control systems of respective parts, with dynamic information analysis and monitoring systems of respective parts of the device, lines between various systems and the computer control center for exchanging data, control and information, and the computer control center serving as accessory structures of the central system for commanding the whole device to serve for growth and development and reproduction of plants and other lower living organisms; in this way, an intelligent living body like higher animals is formed by combining plants and other lower living organisms with the device as a whole, which spontaneously acquires the substance, energy and information, spontaneously creates an ambient environment suitable for itself just like the human beings, and this combination converts the plants and other lower living organisms from relatively static growth, development and reproduction, and passive absorption and acquisition of substance, energy and information into dynamic and active living organisms.

In the application of the drift and suspension device in the plants, the plants achieve the whole process of growth and development and reproduction continuously in the device.

In the application of the drift and suspension device in the plants, the plants achieve a "pipelined" production continuously in the device.

In the C: application in irrigation and/or purification of seawater or sewage water, the drift and suspension device consists of a controlled chamber wall, a controlled chamber wall insulation layer, a controlled chamber foundation, controlled environment, a gangway between a chamber and another chamber, a gangway door, leaves and stems of a crop group highly resistant to seawater, stems and roots of a crop group highly resistant to seawater, leaves and stems of a crop group moderately resistant to seawater, stems and roots of a crop group moderately resistant to seawater, leaves and stems of a normal freshwater crop group, stems and roots of a normal freshwater crop group, a seawater tank, seawater, seawater processed by the crop group highly resistant to seawater, and seawater processed by the crop group moderately resistant to seawater; wherein the gangway between a chamber and another chamber is a gangway for crop to enter from one chamber to another chamber, after a certain stage of growth and development and reproduction or a certain stage of physiological process of a crop is completed in one chamber, the crop is transplanted into another chamber for a next stage of growth and development and reproduction or another stage of physiological process; the gangway door is arranged on the gangway between a chamber and another chamber, the gangway door is opened first when the crop enters from one chamber into another chamber and the gangway door is closed after the crop enters from one chamber into another chamber, the gangway door maintains relatively independent environment between one chamber and another chamber, which may contribute to completing growth and development of plants at different stages; in the leaves and stems and roots of the crop group highly resistant to seawater, leaves and stems and roots of the crop group moderately resistant to seawater, as well as leaves and stems and roots of the normal freshwater crop group, a crop group of each category is not a single crop but multiple plants within a certain range of tolerance concentrations, the seawater tank at each layer of the device is filled with seawater, the environment at each layer is adjusted by an environment control system into a state most suitable for the crop, then effective constituents of seawater used for irrigation of the crop are detected so as to determine the category of the crop according to detection results and get ready for entering of the crop group highly resistant to seawater which enters into the device through the gangway between a chamber and another chamber and the gangway door, the crop may either be in a normal physiological state (which is just transferred from an environment suitable for another crop) or in at an abnormal stressed physiological state (the crop has not intake desired energy and nutrient substances for a period of time, or has been treated in a relatively harsh environment) before the crop group highly resistant to seawater is placed into seawater, then stems and roots of the crop group highly resistant to seawater are placed in seawater on a first layer; after treatment of a certain time or growth of a certain physiological stage , the pristine seawater concentration or salt concentration is reduced after a portion of solutes or nutrient substances in seawater is absorbed and fixed by the crop group highly resistant to seawater, now the seawater with lower concentration is unsuitable for normal growth and development of the crop group highly resistant to seawater, afterwards, the crop group highly resistant to seawater is placed in seawater at a second layer to continue the process and then placed in seawater on a third layer after the above process; seawater on the first layer is converted from the pristine seawater into seawater with lower concentration after the crop group highly resistant to seawater is placed in seawater at the second layer; now the crop group moderately resistant to seawater enters into the device through the gangway between a chamber and another chamber and the gangway door; similarly, the crop may either be in a normal physiological state (which is just transferred from an environment suitable for another crop) or be in an abnormal stressed physiological state (the crop has not intake desired energy and nutrient substances for a period of time, or has been treated in a relatively harsh environment) before the crop group moderately resistant to seawater is placed into seawater with lower concentration, then roots of the crop group moderately resistant to seawater are placed in seawater with lower concentration on the first layer; after treatment of a certain time or growth of a certain physiological stage, the concentration of the seawater with lower concentration or salt concentration is further reduced after a portion of solutes or nutrient substances in the seawater with lower concentration is absorbed and fixed by the crop group moderately resistant to seawater; now the seawater with even lower concentration, similar to freshwater, is unsuitable for normal growth and development of the crop group moderately resistant to seawater; afterwards, the crop group moderately resistant to seawater is placed in the seawater with lower concentration on the second layer to continue the process and then placed in seawater on the third layer.

The magnetic suspension treatment comprises a hypomagnetic suspension treatment and a magnetic suspension treatment, differentiated according to magnetic field intensity provided by the drift and suspension device for human bodies or animals or the like; the human bodies or animals will be completely suspended by the device when the magnetic field provided by the drift and suspension device reaches a certain intensity Bf, this treatment as well as various restorations, physical therapies and other therapies or the like based on such a magnetic field intensity pertain to magnetic suspension treatment; when the magnetic field intensity provided by the device is smaller than Bf, various restorations, physical therapies and other therapies or the like based on such a magnetic field intensity pertain to hypomagnetic suspension treatment; either an integrated treatment or a partial treatment may be conducted on the human bodies or animals by a hypomagnetic suspension treatment or a magnetic suspension treatment.

According to the present invention, the drift and suspension device applied to the human bodies or animals comprises a suspension warehouse, a suspension warehouse wall, a suspension device shell, a suspension warehouse gate, an upper suspension magnet, a lower suspension magnet, a cushion pad, a preparation couch in for entry to warehouse, a device for supporting and fixing the preparation couch for entry to warehouse, a device for supporting and fixing the suspension warehouse, human for suspension regulation, a dynamic information monitoring device of various systems for human bodies, a dynamic information transmission line of various systems for human bodies, a data processing center, a reality and operation system, an upper suspension magnet energy supply, a lower suspension magnet energy supply, a system for monitoring and feedback information on suspension warehouse inside, an upper suspension magnet control system, a lower suspension magnet control system, an information transmission and feedback line, a human body energy regulation device and a human body nutrition regulation device; wherein
the cushion pad, the preparation couch for entry to warehouse and the device for supporting and fixing the preparation couch for entry to warehouse constitute a platform in preparation for a person subject to suspension regulation of the innovative device of the present invention; the cushion pad is placed on the preparation couch for entry to warehouse, after the person subject to suspension regulation gets ready, under the control of the reality and operation system, the person subject to suspension regulation is sent by the cushion pad to the suspension warehouse, the preparation couch for entry to warehouse is fixed by the device for supporting and fixing the preparation couch for entry to warehouse, one end of the preparation couch for entry to warehouse is connected to the suspension warehouse for bearing the weight of the person subject to suspension regulation and preparing for warehouse entry, the device for supporting and fixing the preparation couch for entry to warehouse is configured to fix and support the preparation couch for entry to warehouse, both the height and the length of the preparation couch for entry to warehouse are adjusted by the device for supporting and fixing the preparation couch for entry to warehouse as needed, and the bottom of the device for supporting and fixing the preparation couch for entry to warehouse is provided with wheels;
the suspension warehouse, the suspension warehouse wall, the suspension device shell, the suspension warehouse gate, the upper suspension magnet, the lower suspension magnet and the device for supporting and fixing the suspension warehouse constitute a suspension system in the innovative device of the present invention; the shape of the suspension warehouse is a circle, an oval, a rectangle or the like on the basis of a human body structure and equipment installed; the suspension warehouse is configured to suspend the person subject to suspension regulation, with some other equipment installed, for example, the dynamic information monitoring device of various systems for human bodies and some other monitoring devices; the suspension warehouse wall is made from diamagnetic materials or non-magnetic materials, such as plastics, rubber, copper or the like; the upper part and the lower part of the suspension warehouse are provided with the upper suspension magnet and the lower suspension magnet respectively, both the upper suspension magnet and the lower suspension magnet are mounted between the suspension warehouse wall and the suspension device shell and generate a magnetic field in the suspension warehouse as needed, with magnetic induction intensity of the magnetic field regulated under the control of the reality and operation system; a superconducting magnet or a superconducting coil is selected and used as the upper suspension magnet and the lower suspension magnet both of which are composed of an electromagnet or a permanent magnet; according to different demands of human bodies for assistant regulation and restoration and dredging, under the control of the reality and operation system, a weak magnetic field, a strong magnetic field, an ultrastrong magnetic field, a uniform magnetic field, a gradient magnetic field, an alternating magnetic field and a pulsed magnet field or the like are generated in the suspension warehouse; the suspension warehouse gate is arranged at a joint connecting the preparation couch for entry to warehouse and the suspension warehouse, and the suspension warehouse gate is automatically opened under the control of the reality and operation system before the person subject to suspension regulation enters into the suspension warehouse, and is automatically closed under the control of the reality and operation system after the person subject to suspension regulation enters into the suspension warehouse; a sealed end of the suspension warehouse is opposite to one end of the suspension warehouse gate, it is not allowed to open the sealed end unless otherwise specified, for example, in case of maintenance or malfunction of the suspension warehouse gate; or both ends of the suspension warehouse gate are respectively provided with a suspension warehouse gate; the suspension device shell is fixed to the device for supporting and fixing the suspension warehouse, and various components and devices in the suspension system are directly or indirectly fixed to the suspension device shell;
the dynamic information monitoring device of various systems for human bodies, the dynamic information transmission line of various systems for human bodies, the data processing center and the reality and operation system constitute an information monitoring and control operation system in the innovative device of the present invention; the dynamic information monitoring device of various systems for human bodies is mounted in the suspension warehouse, and is connected to the person subject to suspension regulation after the person subject to suspension regulation is sent to the suspension warehouse; data related to human body life activities and various environmental data are monitored, and these data are transferred to the data processing center through the dynamic information transmission line of various systems for human bodies for analysis and treatment to make a corresponding judgment, afterwards, the reality and operation system sends out instructions to adjust and control the magnetic induction intensity of the suspension warehouse and contents (oxygen, moisture, drug and the like hypomagnetic state substances achieving an assistance effect) of various substances in the environment and to communicate with the person subject to suspension regulation, thus finally unblocking internal magnetic state biomagnetic field pathway of an overall organism, strengthening the energy of the internal magnetic state of biomagnetic field pathway of the overall organism, and rejuvenating the vital activity of the organism;
under the control of the upper suspension magnet control system, the upper suspension magnet energy supply provides adjustable current for the upper suspension magnet and is connected to an outside power source; the upper suspension magnet control system is connected to the reality and operation system which issues a control instruction transmitted to the upper suspension magnet control system; under the control of the lower suspension magnet control system, the lower suspension magnet energy supply provides adjustable current for the lower suspension magnet and is connected to an outside power source; the lower suspension magnet control system is connected to the reality and operation system which issues a control instruction transmitted to the lower suspension magnet control system; both sides of the suspension warehouse are mounted with the upper suspension magnet energy supply, the lower suspension magnet energy supply, the system for monitoring and feedback information on suspension warehouse inside, the upper suspension magnet control system, the lower suspension magnet control system, the human body energy regulation device and the human body nutrition regulation device; the human energy regulation device is mounted on one side of the suspension warehouse and is controlled by the reality and operation system; the human body energy regulation device provides energy necessary for the person subject to suspension regulation in the suspension warehouse according to physiological needs of the person subject to suspension regulation for energy; first the human body energy regulation device detects the human body energy state, and then sends data to the data processing center to make a judgment through analysis and arrangement, and the reality and operation system issues an instruction to control the human body energy regulation device to provide a moderate amount of energy for human bodies, thus assisting organism in recuperation of internal magnetic state biomagnetic field; the human body nutrition regulation device is mounted on one side of the suspension warehouse and is controlled by the reality and operation system; supply of nutrient substances is based on physiological demands of the person subject to suspension regulation; first the human body nutrition regulation device detects the human body energy state, and then sends data to the data processing center to make a judgment through analysis and arrangement, and the reality and operation system issues an instruction to control the human body nutrition regulation device to provide an appropriate amount of nutrient substances for human bodies, thereby assisting organism in recuperation of internal magnetic state biomagnetic field; and
the drift and suspension device comprises a device of at least one set of suspension system; the position among all sets of suspension systems is an up-down structure, or front-back or left-right structures; the device exchanges human bodies or animals among various suspension systems for staged magnetic suspension treatment.

According to the present invention, the drift and suspension device applied to plants comprises: a controlled chamber wall insulation layer, a controlled chamber foundation, a controlled environment, a nutrition trough, a gangway between a chamber and another chamber, a gangway door, a nutrient solution, a turbid solution, roots of a crop, leaves and stems of the crop, a crop fixing disk (a main bracket and an auxiliary bracket are both fixedly mounted on the crop fixing disk, wherein the crop is fixed to the fixing system via the main and auxiliary brackets, and the crop is moved based on the program via a power system and a control system), a power system of the crop fixing disk, a control system of the crop fixing disk, a main bracket for fixing the crop (which is mainly for fixing the main stems and roots of the crop, and assisting the other fixing brackets, wherein the main bracket may extend and warp, and the main bracket may be shaped like a rod, a spiral or an irregular curve), an auxiliary bracket for fixing the crop (which is mainly for fixing the steps and fruits of the plants, wherein the auxiliary bracket may extend or warp, and may be shaped like a rod, a spiral or an irregular curve), a transverse bracket for fixing the crop (which may be shaped like a circular ring, a cruciform, a mesh structure or the like, wherein the bracket may extend or warp), a transverse bracket for fixing the roots of the crop (which may be shaped like a circular ring, a cruciform, a mesh structure or the like, wherein the bracket may extend or warp), a breeding matrix trough, breeding matrix, seeds, a permeable breeding tray, a nutrient solution feed pipeline, a nutrient solution recovery pipeline, a monitor probe for dynamic information of the nutrient solution, a transmission line for dynamic information of the nutrient solution, a temperature control system, a humidity control system, a CO2 concentration control system, an oxygen component control system, an illumination intensity uniformity control system, a microorganism control system, a magnetic field gradient control system, transmission control lines for various control systems, a temperature monitoring system, a humidity monitoring system, a CO2 concentration monitoring system, an oxygen component monitoring system, an illumination intensity uniformity monitoring system, a microorganism monitoring system, a magnetic field gradient monitoring system, an air quality monitoring system, transmission lines for various monitoring systems, a system for dynamically monitoring and analyzing items such as temperature, humidity, CO2, oxygen, illumination, air quality, microorganism and magnetic field gradient or the like, a central control system, a nutrient solution analysis and preparation system, a nutrient solution feed system, a system for dynamically monitoring and analyzing information on nutrient solution, a nutrient solution recovery system, a system for analyzing constituents of the nutrient solution recycled and extracting effective constituents, lines for exchanging data, control and information between various systems and a computer control center, and the computer control center;
the controlled chamber wall, the controlled chamber wall insulation layer and the controlled chamber foundation constitute a chamber which provides controlled environment for the innovative device of the present invention, in another word, a controlled environment chamber; the controlled chamber wall is a framework and a supporting part of the controlled environment chamber, serving as an installation foundation of thermal insulating material and other various equipment and facilities, and the controlled chamber wall is of a brick structure, a steel structure, or structures of other materials; it is determined the length, width and height of the controlled chamber wall and material selected on the basis of planting needs; the controlled chamber wall insulation layer is mounted on the controlled chamber wall by means of exterior wall installation, interior wall installation or inwall installation, guaranteeing stable temperature inside the controlled environment chamber; materials with the minimum thermal conductivity are selected for the controlled chamber wall insulation layer which is installed in the controlled chamber foundation or a terrace besides on the controlled chamber wall so as to reduce the exchange of inside and outside heats and maintain a stable interior temperature as much as possible; the controlled chamber foundation serves as the foundation of the whole device, which determines the service life of the whole device and resistance to damage by various natural forces or human factors, and the like.; the controlled chamber foundation shall not leak any harmful or polluting substance to the outside environment; the controlled environment is a controllable environment required for growth and development of plants; interior environment includes temperature, humidity, CO2, oxygen, illumination, air composition, microorganism, magnetic field gradient and the like, and these factors are subject to monitoring, analysis, regulation and control by means of corresponding devices; the controlled environment chamber of the device is a layout structure of at least one layer in vertical direction, more layers (for example, N layers, N ≥ 1) are laid out in the vertical direction according to different categories of crops and planting needs; wherein in each layer crops are of the same category or interplanted with various crops, in the same layer the same nutrient solution or different nutrient solutions are designed on the basis of different demands, and crops between one layer and another layer are the same crop or different crops;
the nutrition trough is filled with nutrient solution to provide nutrient substances desired for growth and development of plants, roots of a crop are immersed into the nutrient solution to absorb various nutrient substances and moisture desired by the crop if necessary; the shape of the nutrition trough is unlimited and is a circle, a square, a rectangle or an irregular shape on the basis of demands of the crop; according to the quantity and category of the crop in the nutrition trough, the nutrition trough is a nutrition trough for a single-plant crop, a nutrition trough for a trough single-plant crop, or a nutrition trough for multiple mixed crops; the size of the nutrition trough is determined on the basis of situation of the crop; the nutrition trough is made from plastic material, resin material, rubber material, metal material, synthetic material and the like not reacting chemically with the nutrient solution, thus avoiding the nutrient solution from being polluted, avoiding the nutrition trough from being damaged structurally, avoiding the growth and development of the crop from being affected and avoiding unsafe factors; the nutrition trough is provided with a dynamic information monitor probe for the nutrient solution so as to know in real time the concentration and major constituents of the nutrient solution in the nutrition trough and variation of constituents; data information acquired is sent by the information monitor probe to the system for dynamically monitoring and analyzing information on the nutrient solution instantaneously through the dynamic information transmission line for the nutrient solution; after immediately processing the data information received, the system for dynamically monitoring and analyzing information on nutrient solution develops a corresponding regulation and control scheme (for regulating the concentration and the like of the nutrient solution, and extracting the substances exhausted or exchanged from the roots of the plants), then sends the regulation and control scheme to the computer control center through lines for exchanging data, control and information between various systems and the computer control center; afterwards, the computer control center makes an overall analysis on the basis of the whole and individual situations in the device, and sends an instruction to the system for dynamically monitoring and analyzing information on the nutrient solution through lines for exchanging data, control and information between various systems and the computer control center; by means of the control instruction, the system for dynamically monitoring and analyzing information on the nutrient solution controls the nutrient solution feed pipeline mounted on the nutrition trough so as to maintain stable concentration and constituents of the nutrient solution, and transfers substances discharged or secreted by the crop to a recovery device through the nutrient solution recovery pipeline for extracting substances discharged or secreted by the crop; many constituents of the nutrient solution are soluble substances, but some substances (organic substances, which are mandatory substances in the soil) are insoluble, which may affect absorption and utilization of some substances of the crop, thus affecting health, yield and quality of the crop; this problem is solved by following solutions: (1) water for preparing the nutrient solution is processed to improve the solubility of the nutrient solution, thus improve the solubility, or temperature of the nutrient solution is properly increased without affecting growth and development of the crop; (2) some nutrient substances (effective substances in the soil affecting growth and development of the crop) in soil whose main functions fail to be determined at present are absorbed by the crop, water is mixed with corresponding soil to form suspension liquid or turbid solution, which facilitates absorption of certain special nutrient substances by the crop, thus maintaining special characteristics of the crop; in addition, after a magnetic field treatment of the water which constitute the nutrient solution, magnetic water formed increases the solubility of water, its main function is to serve as a hypomagnetic state for assisting an internal magnetic state of the crop and restoring and dredging the internal magnetic state of biomagnetic field pathway so as to maintain the internal magnetic state of biomagnetic field pathway of the crop unblocked;
wherein the crop fixing disk, the main bracket for fixing the crop, the auxiliary bracket for fixing the crop, the transverse bracket for fixing the crop and a transverse bracket for fixing roots of the crop constitute an innovative crop fixing system of the present invention; after being fixed by the fixing system, with the assistance of the fixing system and under the control of the computer control center, the crop spontaneously and selectively absorbs and uses various substances and energy on the basis of growth and development conditions of the crop and its demands for various substances and energy; the crop fixing disk serves as a fixing and supporting device of the whole fixing system, both the main bracket and the auxiliary bracket are fixed to the crop fixing disk, the crop is fixed to the fixing system by means of the main bracket and the auxiliary bracket, the crop moves in accordance with the procedure of the computer control center by means of the power system and the control system; the main bracket for fixing the crop is fixedly mounted on the crop fixing disk and is mainly used for fixing the main stem and main root of the crop and assisting other fixing brackets, the main bracket extends and warps, shaped like a rod, a spiral or an irregular curve and made from lighter materials such as plastic steel, plastics or other materials; the auxiliary bracket for fixing the crop is fixedly mounted on the main bracket for fixing the crop and is mainly used for fixing stems and fruits of the crop, the auxiliary bracket extends and warps, shaped like a rod, a spiral or an irregular curve; the transverse bracket for fixing the crop is fixedly mounted on the main bracket for fixing the crop and is mainly used for fixing stems and fruits of the crop, the transverse bracket extends and warps, shaped like a circular ring, a cruciform, a mesh structure or the like; brackets extend and warp so as to better fix the crop; the transverse bracket for fixing roots of the crop is fixedly mounted on the main bracket for fixing the crop and is mainly used for fixing roots of the crop and assisting roots of the crop to acquire moisture, nutrient substances and energy, shaped like a circular ring, a cruciform, a mesh structure or the like, the bracket extends and warps; after the crop is fixed to the fixing system, the crop moves under the action of the power system of the crop fixing disk, the power system of the crop fixing disk moves under the control of the control system of the crop fixing disk, the control system of the crop fixing disk receives an instruction from the computer control center, after the computer control center makes an overall coordination and analysis, an action instruction (physiological demands, life activity demands, and the like) required for the crop is transferred to the control system of the crop fixing disk; under the control of the control system of the crop fixing disk, the power system of the crop fixing disk completes voluntary movement of the crop and acquires energy and substances desired by the crop, thus meeting design demands of the innovative device of the present invention;
the gangway between a chamber and another chamber is a gangway for entering from one chamber to another chamber, after a certain stage of growth and development and reproduction or a certain stage of physiological process of the crop is completed in one chamber, the crop is transplanted into another chamber for a next stage of growth and development and reproduction or another stage of physiological process; the gangway door is arranged on the gangway between a chamber and another chamber, the gangway door is opened first when the crop enters from one chamber into another chamber and the gangway door is closed after the crop enters from one chamber into another chamber, the gangway door maintains a relatively independent environment between one chamber and another chamber, which contributes to completing growth and development of the crop at different stages;
the temperature control system, the humidity control system, the CO2 concentration control system, the oxygen component control system, the illumination intensity uniformity control system, the microorganism control system, the magnetic field gradient control system constitute a control segment of the innovative device of the present invention;
the monitor probe for dynamic information of the nutrient solution, the temperature monitoring system, the humidity monitoring system, the CO2 concentration monitoring system, the oxygen component monitoring system, the illumination intensity uniformity monitoring system, the microorganism monitoring system, the magnetic field gradient monitoring system and the air quality monitoring system constitute a monitoring segment of the innovative device of the present invention;
the system for dynamically monitoring and analyzing items such as temperature, humidity, CO2, oxygen, illumination, air quality, microorganism and magnetic field gradient or the like, the central control system, the nutrient solution analysis and preparation system, the nutrient solution feed system, the system for dynamically monitoring and analyzing information on the nutrient solution, the nutrient solution recovery system, the system for analyzing constituents of the nutrient solution recycled and extracting effective constituents and the computer control center constitute a central control system of the innovative device of the present invention;
the nutrient solution feed pipeline, the nutrient solution recovery pipeline, the dynamic information transmission line for the nutrient solution, transmission control lines for various control systems, transmission lines for various monitoring systems and lines for exchanging data, control and information between various systems and the computer control center constitute an accessory device of the innovative device of the present invention;
wherein in the temperature control system and the temperature monitoring system, temperature in the environment of the innovative device of the present invention, temperature in the nutrient solution and sensible temperature (including the sensitive temperatures of the roots and stems) of the crop are sent by the temperature monitoring system to the temperature control system in real time, after the temperature control system analyzes and sorts temperature information acquired, the temperature information is sent to the computer control center for an overall analysis; according to different demands of plants for temperature at different growth and development stages and different physiological metabolism time, various factors and data are synthesized so as to give an instruction to regulate and control temperature, and the instruction is sent to the temperature control system for regulating and controlling temperature, thus reaching a temperature range most suitable for growth and development and reproduction of the crop;
wherein in the humidity control system and the humidity monitoring system, humidity in the environment of the innovative device of the present invention is sent by the humidity monitoring system to the humidity control system in real time, after the humidity control system analyzes and sorts humidity information acquired, the humidity information is sent to the computer control center for an overall analysis; according to different demands of plants for humidity at different growth and development stages and different physiological metabolism time, various factors, such as humidity, wind velocity, growth and physiological conditions of plants and other data are synthesized so as to give an instruction to regulate and control humidity, and the instruction is sent to the humidity control system for regulating humidity, thus maintaining environment humidity within a range most suitable for plants;
in the CO2 concentration control system and the CO2 concentration monitoring system, the concentration of CO2 in the environment of the innovative device of the present invention is sent by the CO2 concentration monitoring system to the CO2 concentration control system in real time, after the CO2 concentration control system analyzes and sorts CO2 concentration information acquired, the CO2 concentration information is sent to the computer control center for an overall analysis; according to different demands of plants for CO2 concentration at different growth and development stages and different physiological metabolism time, data of growth and development conditions of the crop and data according to the demands for CO2 concentration are synthesized so as to give an instruction to regulate and control CO2 supply, and the instruction is sent to the CO2 concentration control system for controlling CO2 supply from the environment, thus maintaining CO2 concentration in the environment within a range most suitable for plants;
in the oxygen component control system and the oxygen component monitoring system, the concentration of oxygen in the environment of the innovative device of the present invention is sent by the oxygen component monitoring system to the oxygen component control system in real time, after the oxygen component control system analyzes and sorts oxygen concentration information acquired, the oxygen concentration information is sent to the computer control center for an overall analysis; according to different demands of plants for oxygen concentration at different growth and development stages and different physiological metabolism time, data of growth and development conditions of the crop and data according to the demands for oxygen are synthesized so as to give an instruction to regulate and control oxygen concentration, and the instruction is sent to the oxygen component control system for controlling oxygen concentration in the environment, thus maintaining oxygen concentration in the environment within a range most suitable for plants;
in the illumination intensity uniformity control system and the illumination intensity uniformity monitoring system, firstly, illumination intensity in the environment of the innovative device of the present invention is sent by the illumination intensity uniformity monitoring system to the illumination intensity uniformity control system in real time, after the illumination intensity uniformity control system analyzes and sorts illumination intensity information acquired, the illumination intensity information is sent to the computer control center for an overall analysis; according to different demands of plants for illumination intensity at different growth and development stages and different physiological metabolism time, data of photosynthesis of the crop and data according to the demands for illumination intensity are synthesized so as to give an instruction to regulate and control illumination intensity, and the instruction is sent to the illumination intensity uniformity control system for controlling illumination intensity in the environment, thus maintaining illumination intensity in the environment within a range most suitable for photosynthesis of plants;
wherein in the microorganism control system and the microorganism monitoring system, microorganisms and pests on the crop in the innovative device of the present invention are detected and damage of the crop resulted from these etiologies is evaluated by the innovative device of the present invention by means of the microorganism monitoring system, the information acquired is sent to the microorganism control system in real time; after the microorganism control system analyzes and sorts the information related to etiologies and to damage of the crop, the information is sent to the computer control center for an overall analysis, a scheme for prevention and treatment of diseases is formulated according to different growth and development stages and different physiological metabolism time of plant, then damage of the crop is targeted and repaired by means of the microorganism control system, thus maintaining healthy growth of the crop and guaranteeing high and stable yield of the crop;
wherein in the magnetic field gradient control system and the magnetic field gradient monitoring system, the internal magnetic state of biomagnetic field pathway of the crop in the innovative device of the present invention is detected whether to be orderly and unblocked by means of the magnetic field gradient monitoring system, also external magnetic state factors affecting the internal magnetic state are detected and analyzed, and then the information is sent to the magnetic field gradient control system in real time, after the magnetic field gradient control system analyzes and sorts the acquired information related to the internal magnetic state and the external magnetic state, the information is sent to the computer control center for an overall analysis, a corresponding restoration and recovery scheme is formulated according to different results of impact of the external magnetic state on the internal magnetic state biomagnetic field of plants, then the impact of the external magnetic state changed by means of the magnetic field gradient control system, and with the assistance of hypomagnetic state, restore and recover the impact on the internal magnetic state of biomagnetic field;

### Detailed description of the invention:

The device according to the present invention is based on an interrelation between magnetism and life, and is used for the treatment of such aspects as health and anti-ageing of the living bodies. Based on the interrelation between magnetism and life discussed in Magnetism and Life, the device according to the present invention employs the manner of the hypermagnetic state to assist, in an internal magnetic state, dredging and restoration of blocking and isolation of the internal magnetic state biomagnetic field pathway caused by the external magnetic state, so as to orderly dredge the internal magnetic state biomagnetic field pathway in living bodies. Under effective and accurate control of the psychological aspect of the living bodies, energy and substance information effectively and accurately transported, via the internal magnetic state biological pathway, to the portions of the living bodies where energy and substance are desired, and meanwhile various excrement discharged from the living bodies are effectively and accurately converted or exhausted via the internal magnetic state biomagnetic field pathway, so as to maintain normal operation of the magnetic state energy and substance information in the living bodies, keep the living bodies health, and prolong life time of the living bodies. The device according to Embodiment 1 assists the internal magnetic state of the living bodies in various aspects, comprising: overall assistance, breaking the blocked portion in the internal magnetic state magnetic field pathway in the living bodies to achieving overall dredging; overall and partial assistance, breaking the blocked portion in the magnetic field pathway via partial assistance, quickly eliminating the effect caused by partially blocking in the living bodies via overall assistance, and quickly and accurately transporting the hypermagnetic substances (drugs or the like) for repairing and dredging the blocked pathway to the positions where repair is desired, for partial repair and dredging.

By means of magnetic suspension, inseparably supplementary integrity is achieved between the internal magnetic state, the external magnetic state, and the hypermagnetic state of the organisms. To be specific, resistance and dissonance are eliminated to reach a balance and a highly harmony integral body, i.e., like the Chinese old philosophical saying "man is an integral part of nature".

### BRIEF DESCRIPTION OF THE DRAWINGS

FIG. 1 illustrates a comparison between a curve of weight increase of the mice raised in the magnetic field and a curve of weight increase of the mice not raised in the magnetic field;
FIG. 2 schematically illustrates three experiments wherein the maximum life time of the mice may be prolonged by 9% to 14% with rapamycin;
FIG. 3 illustrates a sleep chronicle of people;
FIG. 4 is a front schematic structural view illustrating structure of application equipment of magnetic suspension in human bodies according to the present invention;
FIG. 5 is an A-A schematic structural view illustrating structure of application equipment of magnetic suspension in human bodies according to the present invention;
FIG. 6 is a front schematic structural view illustrating upper-and-lower layered structure of application equipment of magnetic suspension in human bodies according to the present invention;
FIG. 7 is a front schematic structural view illustrating left-and-right structure of application equipment of magnetic suspension in human bodies according to the present invention;
FIG. 8 is a schematic structural view of application equipment of magnetic suspension in aquatic cultivation/plant cultivation according to the present invention;
FIG. 9 is a schematic structural view of application equipment of magnetic suspension in livestock and poultry cultivation according to the present invention;
FIG. 10 is a schematic structural view of a suspension warehouse of application equipment of magnetic suspension in livestock and poultry cultivation according to the present invention;
FIG. 11 is a schematic structural view of a plant fixing frame of application equipment of drift and suspension in plant cultivation according to the present invention;
FIG. 12 illustrates growth and development stages of wheat in the prior art;
FIG. 13 illustrates growth and development stages of wheat applied in the device according to Embodiment 8 of the present invention;
FIG. 14 is a schematic view of application equipment of suspension in purification of seawater according to Embodiment 9 of the present invention.

The drawings on pages 8 to 23 illustrate application equipment of drift and suspension in human bodies or animals; the drawings on pages 24 to 74 illustrate application equipment of drift and suspension in plant cultivation; the drawings on pages 75 to 98 illustrate application equipment of drift and suspension in purification of seawater; and the drawings on page 99 illustrate application of the device in wheat cultivation according to the present invention.

In the drawings, 1 denotes a suspension warehouse, 2 denotes a gate, 3 denotes an upper suspension magnet, 4 denotes a lower suspension magnet, 5 denotes suspension processing equipment, 10 denotes an information detecting system, 11 denotes an information transmission line, 12 denotes a data processing machine, 13 denotes display and operation equipment, 14 denotes an upper suspension magnet energy supply, 15 denotes a lower suspension magnet energy supply, 16 denotes a suspension information detecting system, 17 denotes a lower suspension magnet control system" 18 denotes a lower suspension magnet control system, 19 denotes an information feedback line, 20 denotes a nutrition trough, 21 denotes a nutrient solution, 22 denotes a thermal insulating layer, 23 denotes a warehouse base layer, 24 denotes an in-warehouse environment, 25 denotes warehouse walls, 26 denotes an environment control device, 28 denotes a human body energy regulation device, 29 denotes a human body nutrition regulation device, 30 denotes a suspension magnet, 31 denotes animals, 32 denotes grass, 33 denotes plant stems and leaves, 34 denotes plant roots, 35 denotes a gangway, 36 denotes a cultivation trough, 36 denotes a cultivation matrix, 38 denotes a plant fixing frame, 39 denotes seeds, 40 denotes a nutrient solution supply system, 41 denotes a seed breeding disk, 44 denotes a temperature control system, 45 denotes a humidity control system, 46 denotes a CO2 concentration control system, 47 denotes an oxygen component control system, 48 denotes an illumination uniformity control system, 49 denotes a microorganism control system, 50 denotes a magnetic field gradient control system, 52 denotes a temperature monitoring system, 53 denotes a humidity monitoring system, 54 denotes a CO2 concentration monitoring system, 55 denotes an oxygen component monitoring system, 56 denotes an illumination uniformity monitoring system, 57 denotes a microorganism monitoring system, 58 denotes a magnetic field gradient monitoring system, 59 denotes an air quality monitoring system, 70 denotes leaves and stems of a crop group highly resistant to seawater, 71 denotes stems and roots of a crop group highly resistant to seawater, 72 denotes stems and leaves of a crop group moderately resistant to seawater, 73 denotes stems and roots of a crop group moderately resistant to seawater, 74 denotes stems and leaves of a normal freshwater crop group, 75 denotes roots of a normal freshwater crop group, 76 denotes a water tank, 77 denotes seawater, 78 denotes seawater processed by the crop group highly resistant to seawater, and 79 denotes seawater processed by the crop group moderately resistant to seawater.

### DETAILED DESCRIPTION

The following embodiments are merely for illustrating the present invention, rather than limiting the scope of the present invention. Without departing from the spirit and essence of the present invention, any modification or replacement made to the method, steps or conditions of the present invention all fall within the scope of the present invention.

### Embodiment 1 Application equipment of magnetic suspension in human bodies according to the present invention

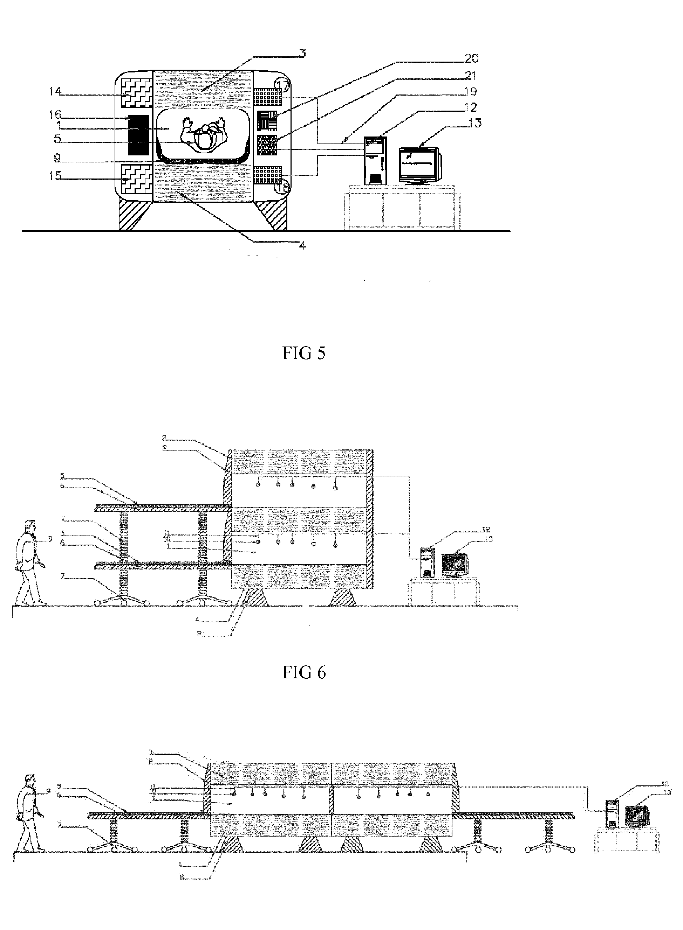

The application equipment of magnetic suspension in human bodies, as illustrated in FIG. 4 and FIG. 5, comprises: a suspension device, a dynamic information processing device, and a nutrient providing device. To be specific, the suspension device subjects a magnetic suspension treatment to the human bodies; the dynamic information processing device detects dynamic information of the human bodies in a suspension state; and the nutrient providing device provides in real time nutrients and/or drugs desired by the human bodies.

The suspension device comprises a preparation couch 6 and a suspension warehouse 1 which are coupled to each other. The preparation couch 6 and the suspension warehouse 1 are respectively provided with a preparation couch supporting device 7 and a suspension warehouse supporting device 8.

The preparation couch supporting device 7 may adjust the height and length of the preparation couch 6 according to actual needs, and the preparation couch supporting member is provided with wheels at the bottom thereof, which may be moved aside where unnecessary and moved to a destination where necessary. An upper surface of the preparation couch 6 is provided with a cushion pad 5. Under the control of display and operation equipment 13, the cushion pad 5 carries a person 9 subject to suspension regulation above a lower suspension magnet 4 in the suspension warehouse 1, thereby ensuring comfort of the person 9 subject to suspension regulation, such that the person 9 subject to suspension regulation keeps a calm and normal psychological state, achieving the effect of protecting the person.

The suspension warehouse 1 is an airtight box, and the suspension warehouse wall is made from diamagnetic materials or non-magnetic materials, such as plastics, rubber, copper or the like. One side coupling to the preparation couch 6 is provided with a gate 2, wherein the gate 2 may be opened and closed. The closed gate 2 may ensure stability of various environment factors in the suspension warehouse 1 during the entire operation of the device. The end opposite to the gate 2 is a sealed end of the suspension warehouse 1, which may be opened in a special situation, for example, for example, in case of maintenance or malfunction of the suspension warehouse gate 2.

The suspension warehouse 1 is internally provided with an upper suspension magnet 3 and a lower suspension magnet 4 which have the same magnetic field direction. Energy of the upper suspension magnet 3 and the lower suspension magnet 4 are respectively provided by an upper suspension magnet energy supply 14 and a lower suspension magnet energy supply 15, and the energy supply is respectively controlled by an upper suspension magnet control system 17 and a lower suspension magnet control system 18. The upper suspension magnet 3 and the lower suspension magnet 4 may generate desired magnetic fields in the suspension warehouse 1. The upper suspension magnet 3 and the lower suspension magnet 4 may be superconducting magnet (superconducting coils), or may be formed of an electric magnet and a permanent magnet. The upper suspension magnet and the lower suspension magnet may vary according to requirements of regulation and dredging of the human bodies.

The dynamic information processing device comprises an information detecting system 10 configured in the suspension warehouse 1 and a control system configured outside the suspension warehouse 1. The two systems are communicated with each other via an information transmission line 11. The information detecting system 10, the information transmission line 11, a data processing machine 12 and display and operation equipment 13 of the control system, and an information feedback line 19 constitute an information monitoring and control operation system in the device according to the present invention. The information detecting system 10 detects dynamic information of the person 9 subject to suspension regulation in the suspension state, and transmits the detected data to the control system via the information transmission line 11. The control system analyzes and processes the data, and issues an instruction to the nutrient providing device. The control system is provided with the data processing machine 12 and the display and operation equipment 13. The suspension warehouse 1 is further provided with a suspension information detecting system 16, configured to detect suspension information in the suspension warehouse 1 and transmit the detected data to the control system via the information transmission line 11. The control system transmits the feedback information to the upper suspension magnet control system 17 and the lower suspension magnet control system 18 via the information feedback line, regulates the magnetic fields generated by the upper suspension magnet 3 and the lower suspension magnet 4, thereby generating a weak magnetic field, a strong magnetic field, an ultrastrong magnetic field, a uniform magnetic field, a gradient magnetic field, an alternating magnetic field, and a pulsed magnet field.

The suspension warehouse 1 is further provided with a human body energy regulation device 28 and a human body nutrition regulation device 29.

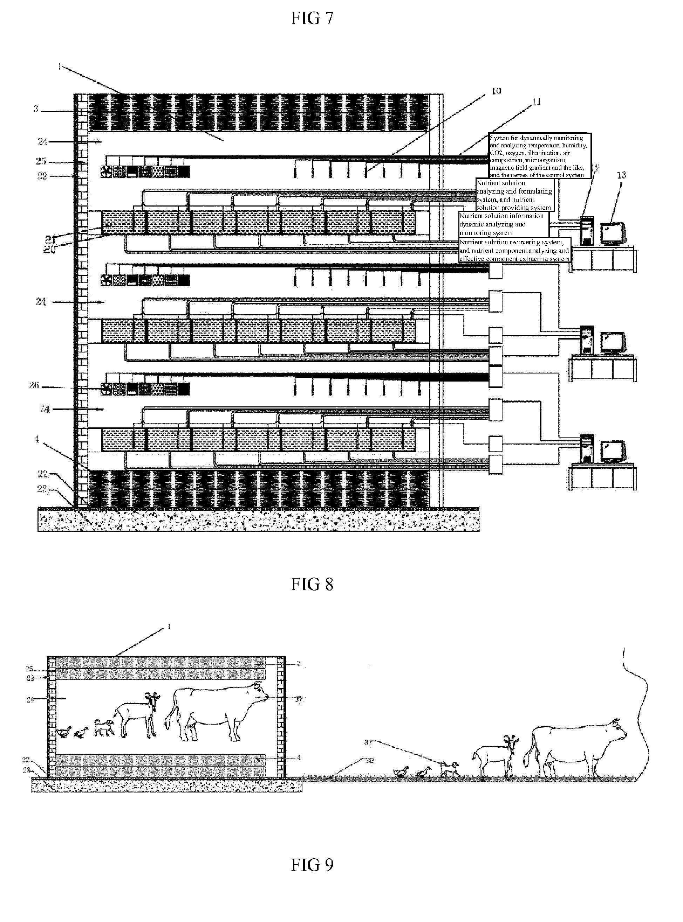

The drift and suspension device is a device comprising at least one suspension system. Suspension systems may be subject to an upper and lower structure in terms of position as illustrated in FIG. 6, or may be subject to a left and right structure in terms of position as illustrated in FIG. 7. The device may exchange the human bodies or animals in various suspension systems, and subject the human bodies or animals to staged magnetic suspension treatments.

### Embodiment 2 Method for using application equipment of magnetic suspension in human bodies according to the present invention

Application of magnetic suspension in human bodies according to the present invention is based on an interrelation between magnetism and life. The device according to Embodiment 1 of the present invention employs the manner of the hypermagnetic state to assist, in an internal magnetic state, dredging and restoration of blocking and isolation of the internal magnetic state biomagnetic field pathway caused by the external magnetic state, so as to orderly dredge the internal magnetic state biomagnetic field pathway in living bodies. Under effective and accurate control of the psychological aspect of the living bodies, energy and substance information effectively and accurately transported via the internal magnetic state biological pathway, to the portions of the living bodies where energy and substance are desired, and meanwhile various excrement discharged from the living bodies are effectively and accurately converted or exhausted via the internal magnetic state biomagnetic field pathway, so as to maintain normal operation of the magnetic state energy and substance information in the living bodies, keep the living bodies health, and prolong life time of the living bodies. The device according to Embodiment 1 assists the internal magnetic state of the living bodies in various aspects, comprising: overall assistance, breaking the blocked portion in the internal magnetic state magnetic field pathway in the living bodies to achieving overall dredging; overall and partial assistance, breaking the blocked portion in the magnetic field pathway via partial assistance, quickly eliminating the effect caused by partially blocking in the living bodies via overall assistance, and quickly and accurately transporting the hypermagnetic substances (drugs or the like) for repairing and dredging the blocked pathway to the positions where repair is desired, for partial repair and dredging. By means of magnetic suspension, inseparably supplementary integrity is achieved between the internal magnetic state, the external magnetic state of the human bodies, and the hypermagnetic state. To be specific, resistance and dissonance are eliminated to reach a balance and a highly harmony integral body, i.e., like the Chinese old philosophical saying "man is an integral part of nature".

Preparations of the person 9 subject to suspension regulation comprise examination, analysis and judgment of the physical, pathological and biomagnetic field pathway running conditions, so as to determine the positions and causes of the blocking of the internal magnetic state biomagnetic field pathway in the organisms, and then determine whether to firstly perform partial assistant regulation, restoration and dredging, or firstly perform overall assistant regulation, restoration and dredging, or perform both simultaneously. If it is determined to firstly perform the partial assistant regulation, restoration and dredging, partial assistant regulation, restoration and dredging is performed firstly by using hypermagnetic drugs, to break the blocked internal magnetic state biomagnetic field pathway in the organisms, and then the organisms are placed into the device according to the present invention for overall regulation and restoration. If it is determined to firstly perform the overall assistant regulation, restoration and dredging, the living bodies may be placed into the device according to the present invention for overall assistant regulation, restoration and dredging. After the internal magnetic state biomagnetic field pathway in living bodies is dredged, the biomagnetic field flux in the internal magnetic state biomagnetic field pathway increases, which quickens the repairing of the overall internal magnetic state biomagnetic field pathway, thus finally unblocking the internal magnetic state biomagnetic field pathway of an overall organism, strengthening the energy of the internal magnetic state of biomagnetic field pathway of the overall organism, and rejuvenating the vital activity of the organism. If it is determined to simultaneously perform the overall and partial assistant regulation, restoration and dredging, through overall assistance, the hypermagnetic substances (drugs or the like) for repairing and dredging the blocked magnetic field pathway are quickly and accurately transported to the positions where repair is desired, for partial repair and dredging. Through overall assistance, drugs (hypermagnetic substances, such as drugs or the like) prepared according to the formulation of the present invention for repairing the blocked magnetic field pathway are quickly and accurately transported to the positions where repair is desired, for partial repair and dredging, thus finally unblocking the internal magnetic state biomagnetic field pathway of an overall organism, strengthening the energy of the internal magnetic state of biomagnetic field pathway of the overall organism, and rejuvenating the vital activity of the organism.

After the person 9 subject to suspension regulation is transported to the suspension warehouse 1, the information detecting system 10 is connected to the person 9 subject to suspension regulation, monitors desired human body life activity data and various environment data, and transmits the monitored data to the data processing machine 12 of the control system via the information transmission line 11. After such data is analyzed and processed, a corresponding judgment is made, and then an instruction is issued by the display and operation equipment 13 to adjust the magnetic inductance strength of the suspension warehouse 1 and the contents of various substances (such hypermagnetic substances as oxygen, moisture, and drugs which exerts an assistance effect) in the environment, and communicate with the person 9 subject to suspension regulation. The information fed back by the information feedback line 19 is transmitted to the upper suspension magnet control system 17, the lower suspension magnet control system 18, and the information monitored by the human body energy regulation device 28 and the human body nutrition regulation device 29 is transmitted to the data processing machine 12 of the control system. In the mean time, a control instruction issued by the display and operation equipment 13 is transmitted to the suspension magnet control system 17, the human body energy regulation device 28, and the human body nutrition regulation device 90 for control, thus finally unblocking internal magnetic state biomagnetic field pathway of an overall organism, strengthening the energy of the internal magnetic state of biomagnetic field pathway of the overall organism, and rejuvenating the vital activity of the organism. In this way, diseases of the living bodies are eliminated, ageing may be relieved, health of the living bodies is improved, and life time of the living bodies is greatly prolonged.

### Embodiment 3 Application equipment of magnetic suspension in aquatic cultivation/plant cultivation according to the present invention

The application equipment of magnetic suspension in aquatic cultivation/plant cultivation according to the present invention is as illustrated in FIG. 8. The equipment according to this embodiment may be used for aquatic cultivation or plant cultivation, and comprises a suspension device, a dynamic information processing device, and a nutrient providing device. To be specific, the suspension device subjects aquatic animals for cultivation to a magnetic suspension treatment; the dynamic information processing device detects dynamic information of the aquatic animals for cultivation in a suspension state; and the nutrient providing device provides in real time nutrients and/or drugs desired by the aquatic animals/plants for cultivation.

The suspension device mainly comprises a suspension warehouse 1 and a dynamic information processing device.

The suspension warehouse 1 is an airtight box, wherein the bottom of the warehouse is a warehouse base layer 23, and the four walls of the warehouse are warehouse walls 25, and an upper suspension magnet 3 and a lower suspension magnet 4 which have the same magnetic field direction are respectively provided at the upper portion and the lower portion inside the warehouse 1. The upper surface of the warehouse base layer 23 and the inner surface of a side warehouse wall are both provided with a thermal insulating layer 22. An in-warehouse environment 24 inside the suspension warehouse 1 is partitioned into several layers by a plurality of nutrition troughs 20. The nutrition trough is provided with a nutrient solution 21, and aquatic animals or plants are cultivated in the nutrition trough 20.

The dynamic information processing device comprises an information detecting system 10 configured in the suspension warehouse 1 and a control system configured outside the suspension warehouse 1, wherein the two systems are communicated with each other via an information transmission line 11. The information detecting system detects dynamic information of aquatic animals in the suspension state, and transmits the detected data to the control system via the information transmission line. The control system analyzes and processes the data, and issues an instruction to an environment control device 26. The control system is provided with the data processing machine 12 and the display and operation equipment 13.

The warehouse walls 25, the thermal insulating layer 22, and the warehouse base layer 23 constitute an environment-controllable suspension warehouse 1. The warehouse walls 25 are the frames and supporting portions of the suspension warehouse 1, and serve as an installation base of the thermal insulating materials and other various facilities, which may employ a brick structure, a steel structure, or a structure made of other materials. According to the needs of the aquatic cultivation or plant cultivation, the length, width and height, and materials of the warehouse walls 25 are determined. The thermal insulating layer 22 is arranged on the warehouse walls 25 and the warehouse base layer 23, and may be arranged on the outer wall, on the inner wall, or inbetween the wall. The thermal insulating layer 22 ensures temperature stability in the suspension warehouse 1. The thermal insulating layer 22 is preferably made from a material having a smaller thermal conduction coefficient, so as to as much as possible reduce exchange of the temperature inside the warehouse with the ambient temperature, and maintain stability of the temperature inside the warehouse. The warehouse base layer 23 is a base of the entire device, which determines the service life of the entire device, and is subjected to various natural forces (earthquake, tsunami, hurricane, geological disaster, or the like) and human damages from the external environment. The warehouse base layer 23 shall not leak any harmful or polluting substance. The in-warehouse environment 24 is a controllable environment needed for aquatic cultivation or plant cultivation, and comprises temperature, humidity, CO2, oxygen, illumination, air component, microorganism, magnetic field gradient, and the like. In addition, such factors may all be monitored, analyzed, and adjusted via the information detecting system 10.

The upper suspension magnet 3 and the lower suspension magnet 4, under control and detection of the control system (the data processing machine 12 and the display and operation equipment 13), generates desired various types of magnetic field strength in the suspension warehouse 1 under a predetermined program according to the instruction issued by the data processing machine 12. The upper suspension magnet 3 and the lower suspension magnet 4 may be a permanent magnet, an electric magnet, or a superconducting magnet according to actual needs. Under normal circumstances, according to the predetermined program, the upper suspension magnet 3 and the lower suspension magnet 4 are enabled every day based on the program to regulate the aquatic animals or plants cultivated in the device. When the aquatic animals or the cultivated plants are in an abnormal state (for example, diseases, injuries or damages, blocking of the internal magnetic state, or the like), according to actual conditions of the organisms, regulation may be made by means of hypermagnetic state assistance and the device according to the present invention, such that the internal magnetic state biomagnetic field pathway of the aquatic animals and plants remains orderly and smooth, and thereby healthy, safe, nutritious and quality produces are produced. Aquatic animals or plants are raised/cultivated in the nutrition trough 20, and the nutrient solution 21 in the nutrition trough 20 provides all nutritious substances desired for growth and development for the aquatic animals and plants. The nutrient solution 21 is placed in the nutrition trough 20, and the nutrition troughs 20 are placed in the suspension warehouse 1 layer by layer.

The information detecting system 10 comprises: a temperature monitoring system, a humidity monitoring system, a CO2 concentration monitoring system, an oxygen component monitoring system, an illumination uniformity monitoring system, a microorganism monitoring system, a magnetic field gradient monitoring system, and an air quality monitoring system. The environment control device 26 comprises: a temperature control system, a humidity control system, a CO2 concentration control system, an oxygen component control system, an illumination uniformity control system, a microorganism control system, and a magnetic field gradient control system. The information detecting system 10 for detecting temperature, humidity, CO2, oxygen, illumination, air quality, microorganism, magnetic field gradient or the like information, and the environment control device 26 are respectively communicated with the control system (the data processing machine 12 and the display and operation equipment 13) via the information transmission line 11. The information detecting system 10 detects the temperature, humidity, CO2, oxygen, illumination, air quality, microorganism, magnetic field gradient or the like information in the suspension warehouse, and transmits the detected data to the data processing machine 12 of the control system. The control system issues an instruction regarding the measures to be taken upon processing of the information to the environment control device 26, for control of the environment inside the suspension warehouse 1.

By means of the detection and control implemented by the above described devices, there is provided an environment suitable for growth and redevelopment regulation of aquatic animals/plants, that is, an external magnetic state exerting minimum impacts to the internal magnetic state of the aquatic animals/plants. In this case, according to the degree of the impacts onto the internal magnetic state of the aquatic animals/plants during the production, corresponding solutions are provided to regulate, adjust, dredge, and repair the internal magnetic state. In the device, according to the degree of the impacts onto the internal magnetic state, the internal magnetic state of the aquatic animals/plants is regulated, adjusted, dredged, and repaired by controlling the upper suspension magnet 3 and the lower suspension magnet 4 to generate corresponding magnetic fields and some hypermagnetic substances (drugs, partial regulations, or the like). Finally, healthy, safe, nutritious and quality produces are produced.

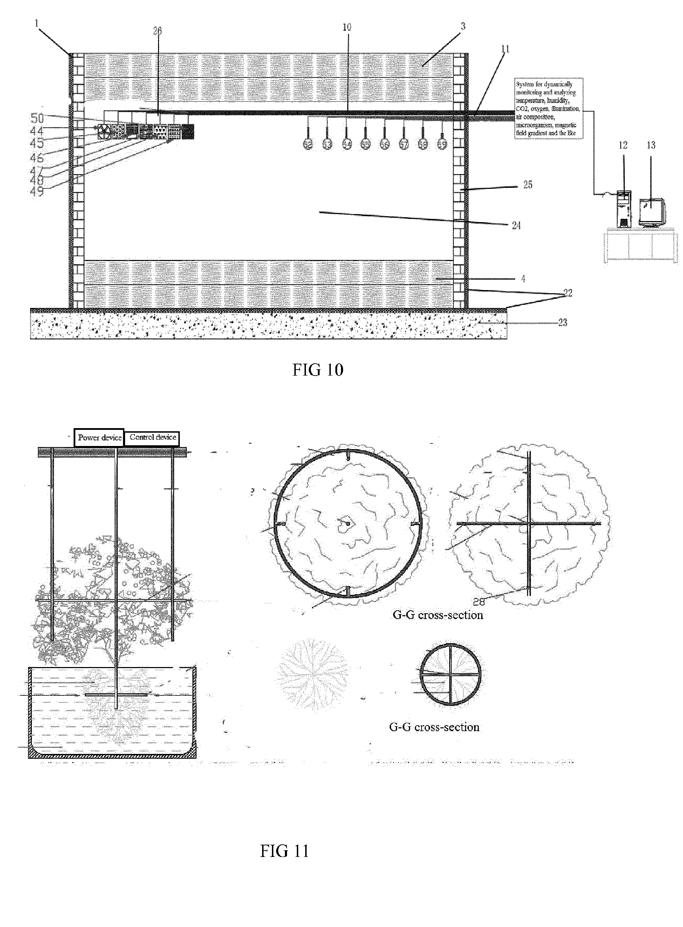

In addition, when the device according to this embodiment is used for cultivating plants, the fixed structure of the plants is as illustrated in FIG. 11, or is a device such as a string bag that is capable of fixing the plants. When the device according to this embodiment is used for cultivating plants, a conveyer device (which may be a rope and conveyer belt) is arranged above each layer of nutrition trough 20. After the plants are fixed by using the fixing structure, the fixing structure is mounted on the conveyer device, and the plants are transported via the conveyer device to a suitable environment.

### Embodiment 4 Application equipment of magnetic suspension in livestock and poultry cultivation according to the present invention

FIG. 9 (in which the dynamic information processing device is omitted), and FIG. 10 are schematic views of application equipment of magnetic suspension in livestock and poultry cultivation according to this embodiment. The equipment mainly comprises: a suspension warehouse 1, and a dynamic information processing device, and grass 38.

The suspension warehouse 1 is an airtight box, wherein the bottom of the warehouse is a warehouse base 23, and the four walls of the warehouse are warehouse walls 25, and an upper suspension magnet 3 and a lower suspension magnet 4 which have the same magnetic field direction are respectively provided at the upper portion and the lower portion inside the warehouse 1. The upper surface of the warehouse base layer 23 and the inner surface of a side warehouse wall are both provided with a thermal insulating layer 22, and the in-warehouse environment 24 in the suspension warehouse 1 is controllable. The warehouse walls 25 serve as an installation base for various equipment or facilities, and may employ a brick structure, a steel structure, or structures of other materials. The length, width and height of the warehouse walls 25, and the frame and supporting portion of the suspension warehouse 1 are determined according to the needs of livestock and poultry cultivation, which are made from a thermal insulating material or another selected material. The thermal insulating layer 22 is installed on the warehouse walls 25. To be specific, the thermal insulating layer 22 may be mounted by means of exterior wall installation, interior wall installation, or inwall installation, which ensures the stability of the temperature inside the environment-controllable warehouse. The thermal insulating layer 22 is preferably made from a material having a smaller thermal conduction coefficient. The thermal insulating layer 22 is not only installed on the warehouse walls 25, but also installed on the warehouse base layer 23 or on the terrace, so as to reduce temperature exchange between the interior and the exterior, and maintain stability of the temperature in the warehouse. The warehouse base layer 23 is a base of the entire device, which determines the service life of the entire device, and is subjected to various natural forces (earthquake, tsunami, hurricane, geological disaster, or the like) and human damages from the external environment. The warehouse base layer 23 shall not leak any harmful or polluting substance. The in-warehouse environment 24 is a controllable environment needed for livestock and poultry cultivation, and comprises temperature, humidity, CO2, oxygen, illumination, air component, microorganism, magnetic field gradient, and the like. In addition, such factors may all be monitored, analyzed, and adjusted via a corresponding device.

The upper suspension magnet 3 and the lower suspension magnet 4, under control and detection of the control system (the data processing machine 12 and the display and operation equipment 13), generates desired various types of magnetic field strength in the suspension warehouse 1 under a predetermined program according to the instruction issued by the data processing machine 12. The upper suspension magnet 3 and the lower suspension magnet 4 may be a permanent magnet, an electric magnet, or a superconducting magnet according to actual needs. Under normal circumstances, according to the predetermined program, the upper suspension magnet 3 and the lower suspension magnet 4 are enabled every day based on the program to regulate animals 31 raised in the device. When the animals 31 are in an abnormal state (for example, diseases, injuries or damages, unstable motion, blocking of the internal magnetic state, or the like), according to actual conditions of the organisms, regulation may be made by means of hypermagnetic state assistance and the device according to the present invention, such that the internal magnetic state biomagnetic field pathway of the animals remains orderly and smooth and thence the animals become healthy with injuries cured and mental disturbance and the like eliminated. As such healthy, safe, nutritious and quality produces are produced. The grass 32 provides energy sources or activity sites for various categories of livestock and poultry.

The information detecting system 10 comprises: a temperature monitoring system 52, a humidity monitoring system 53, a CO2 concentration monitoring system 54, an oxygen component monitoring system 55, an illumination uniformity monitoring system 56, a microorganism monitoring system 57, a magnetic field gradient monitoring system 58, and an air quality monitoring system 59. The environment control device 26 comprises: a temperature control system 44, a humidity control system 45, a CO2 concentration control system 46, an oxygen component control system 47, an illumination uniformity control system 48, a microorganism control system49, and a magnetic field gradient control system 50. The information detecting system 10 for detecting temperature, humidity, CO2, oxygen, illumination, air quality, microorganism, magnetic field gradient or the like information, and the environment control device 26 are respectively communicated with the control system (the data processing machine 12 and the display and operation equipment 13) via the information transmission line 11. The information detecting system 10 detects the temperature, humidity, CO2, oxygen, illumination, air quality, microorganism, magnetic field gradient or the like information in the suspension warehouse, and transmits the detected data to the data processing machine 12 of the control system. The control system issues an instruction regarding the measures to be taken upon processing of the information to the environment control device 26, for control of the environment inside the suspension warehouse 1.

By means of the detection and control implemented by the above described devices, there is provided an environment suitable for growth and redevelopment regulation of livestock and poultry, that is, an external magnetic state exerting minimum impacts to the internal magnetic state of the livestock and poultry. In this case, according to the degree of the impacts onto the internal magnetic state of the livestock and poultry during the production, corresponding solutions are provided to regulate, adjust, dredge, and repair the internal magnetic state. In the device, according to the degree of the impacts onto the internal magnetic state, the internal magnetic state of the livestock and poultry is regulated, adjusted, dredged, and repaired by controlling the upper suspension magnet 3 and the lower suspension magnet 4 to generate corresponding magnetic fields and some hypermagnetic substances (drugs, partial regulations or the like). Finally, healthy, safe, nutritious and quality produces are produced. The grass 32 provides energy sources or activity sites for various categories of livestock and poultry.

### Embodiment 5 Application of magnetic suspension in livestock and poultry cultivation according to the present invention

The following experiment was carried out by using the device according to Embodiment 4.

### 1 Animals for experiment

50 healthy adult male Wistar mice, having weight of 209±4.33, were raised in separate cages, and fed with normal particle-like feeds, and these mice drank as they please.

### 2 Experiment method

2.1 Grouping of the mice: After 1-week life in the laboratory, the 50 mice were randomly grouped into 5 groups, 10 mice in each group, that is, a normal control group, a model group 1, a model group 2, a magnetic field + magnetized water group, and a magnetic field + drug group.

### 2.2 Establishment of mice calcium oxalate calculus models

The mice in the normal control group were subjected to intragastric administration with 2 ml distilled water every morning.

The mice in model group 1 and model group 2 were subjected to intragastric administration with a calculus formation liquid formulated by 1% ethylene glycol (EG) and 2% ammonium chloride (AC) every morning, 2 ml for each mouse per day, to induce mice calcium oxalate calculus. The mice in model group 1 and model group 2 were subjected to intragastric administration with an equal-dose of physiological saline every afternoon.

The mice in the magnetized water group were subjected to intragastric administration with a calculus formation liquid formulated 1% ethylene glycol (EG) and 2% ammonium chloride (AC) every morning, 2 ml for each mouse per day, and were fed with the magnetized water all around the clock.

The mice in the magnetic field + drug group were subjected to intragastric administration with a calculus formation liquid formulated 1% ethylene glycol (EG) and 2% ammonium chloride (AC) every morning, 2 ml for each mouse per day, were each subjected to intragastric administration with 1.5 g secretly formulated drugs every morning, and were suspended in 16 T superconducting magnetic field for 3 minutes, 5 minutes and 8 minutes three times per day (morning, afternoon, and evening). The experiment lasted 30 days.

30 days later, the mice in model group 1 and model group 2 were mixed and raised, and then randomly grouped into two groups, 10 mice in each group, that is, a magnetized water treatment group, and a magnetic field + drug treatment group.

The magnetized water was supplied the mice in the magnetized water treatment group all around the clock for treatment.

The mice in the magnetic field + drug treatment group were each subjected to intragastric administration with 1.5 g secretly formulated drugs every morning, were suspended in 16 T superconducting magnetic field for 3 minutes, 5 minutes and 8 minutes three times per day (morning, afternoon, and evening) for treatment.

The experiment lasted 30 days.

### 3 Sample acquisition and detection

One day before the experiment ended, the mice were placed into a metabolism cage, and 24 hour urine samples were collected and measured. Upon completion of the experiment, the mice were weighed, blood was taken from the hearts of the mice, and the serums were separated. The urine biochemical and blood biochemical indicators were tested. The mice were dissected and the kidneys were taken out, and such indicators as oxalic acids and citric acids in the kidney tissues were tested.

### 4 Experiment results

Some of the experiments are as listed in Table 9 and Table 10.

**Table 9 Formation of renal calculus of various animals**

| Group | Quantity of animals for use in experiment (piece) | Quantity of animals having calculi (piece) | Percentage (%) |
|---|---|---|---|
| Blank control group | 10 | 0 | 0% |
| Model group 1 | 10 | 10 | 100% |
| Model group 2 | 10 | 10 | 100% |
| Magnetized water group | 10 | 6 | 60% |
| Magnetic field + drug group | 10 | 0 | 0% |

**Table 10 Treatment of renal calculus of various animals**

| Group | Quantity of animals for use in experiment (piece) | Quantity of animals with calculi cured (piece) | Percentage (%) |
|---|---|---|---|
| Magnetized water treatment group | 10 | 3 | 30% |
| Magnetic field + drug treatment group | 10 | 10 | 100% |

### Embodiment 6 Application of magnetic suspension in livestock and poultry cultivation according to the present invention

The following experiment was carried out by using the device according to Embodiment 4.

### 1. Materials and methods

### 1.1 Experiment materials

1.1.1 Animals and grouping of animals: 40 Kunming mice were randomly grouped into group A (10 mice), group B (10 mice), group C (10 mice), control group D (10 mice), and were raised.
1.1.2 Reagents: sterile water, physiological saline, trypan blue, 2% glacial acetic acid, formaldehyde, ethanol, dimethylbenzene, paraffin wax, and the like.
1.1.3 Instruments: a centrifugal machine, a microscope, a counting plate, an electronic weigher, a super-clean work bench, an incubator, a slicing machine, and the like.

### 1.2 Experiment methods

1.2.1 Model establishment: H22 tumor cells were taken and centrifuged at 3000 rpm, and then were washed for three times with sterile physiological saline and properly diluted. Afterwards, 40 microliter cell suspension was added with 10 microliter 0.4% trypan blue for dyeing, the cells were tested with the microscope and counted, and then three 106 cells/ml tumor cell suspensions were formulated. The mice in group A, group B, and group C were each subjected to tight axillary subcutaneous vaccination with 0.2 ml tumor cell suspension.

The mice in group A were each subjected to intragastric administration with 1.5 g secretly formulated drugs every morning, were suspended in 16 T superconducting magnetic field for 3 minutes, 5 minutes and 8 minutes three times per day (morning, afternoon, and evening).

The mice in group B, group C, and group D were normally raised and fed.

### 1.2.3 Treatment experiment establishment

The tumor of the mice in group B and group C obviously grew, and the mice in group B and group C were mixed and raised, and then were randomly grouped into two groups, 10 mice in each group, that is, group E: a control treatment group, and group F: magnetic field + drug treatment group.

### 1.3 Records

Upon the vaccination, the mice were observed every day to check whether they were subjected to any infections, and whether the tumor naturally disappears. The long diameter and short diameter of the tumor were measured with a vernier caliper every week, and an average diameter was calculated, r = (a + b) /2. The mice and the tumor thereof were weighed every day and then recorded, and based on such records, a curve was drawn.

### 2. Results

### 2.1 Tumor growth

(1) On the day of vaccination, vacuoles formed by cell suspension were present in the vaccination positions at the right rear leg subcutaneous parts of all the animals. Haft a day later, the liquid was absorbed by the tissue, and the surface of the skin recovers flatness. As seen from the growth of the tumor in the mice in group B and group C, the tumor saw no obvious growth in the first five days, but grew quickly and starts becoming larger starting from the sixth day.
(2) The average weight of the mice in group A decreased within almost one month, with a greatest decrease amplitude of 3 g, and then recovered there weight. Upon completion of the experiment, the weight of the mice recovered to their normal values.
(3) The average weight increase amplitude of the mice in group D within almost one month is 2 g, which is a moderate increase amplitude. The weight curve showed a sharp contrast against the curves of groups A and B.

### 2.2 Tumor treatment

(1) The tumor of the mice in group E grew quickly to a specific size, and then grew constantly and stably. Two weeks later, No. 3 and No. 5 mice were subjected to tumor spreading to the right front leg, and the mice substantially lost the action ability, and finally died in the third week. As seen from the curve of the tumor of the mice in group E, the growth rates of the tumor of the mice in the two groups were different, and were obviously categorized into three types. The first type of tumor grew slowly, and would not grow or grew very slowly after growing to a specific size. The second type of tumor firstly grew very quickly, and constantly and stably grew after growing to a specific size. In the third type of tumor, some grew sharply at a constant rate.
(2) The tumor of the mice in group F grew more slowly than the mice in group E. Two weeks later, the tumor did not grow any greater. Three weeks later, the tumor of No. 3, No., 5, No. 6, No. 8, No. 9, and No. 10 mice started decreasing, and the tumor of No. 1, No. 2, No. 4, and No. 7 mice did not grow any greater. Four weeks later, the tumor of No, 3, No. 5, No. 6, No. 8, No. 9, and No. 10 mice disappeared, and the tumor of No. 1, No. 2, No. 4, and No. 7 mice starts decreasing. Five weeks later, the tumor of all the mice disappeared.

### Embodiment 7 Application of suspension in plant cultivation according to the present invention

### 1. Materials and methods

### 1.1 Experiment materials

The experiment was carried out by using the equipment as illustrated in Embodiment 3 of the present invention. Oil cabbage categories: April oil cabbage. The fed fertilizer: organic fertilizer (N, P, K, totally 25.29%, organic matter content 27.59%, moisture 27.16%); carbamide (N ≥ 46%); diammonium phosphate (N ≥ 18%; P205 ≥ 46%); potassium sulfate (K2SO4 ≥ 50%). Nutrient solution: (a large number of elements: potassium nitrate 0.588 g, calcium nitrate 0.720 g, ammonium phosphate 0.152 g, magnesium sulfate 0.294 g, ferric chloride 0.142 g, totally 1.816 g; trace elements: Potassium iodide 0.00284 g, boric acid 0.00056 g, zinc sulfate 0.00056 g, manganese sulfate 0.0005 g, totally 0.00452 g.)

### 1.2 Experiment design

In the experiment, a conventional cultivation method was employed in the soil in the control group, and the cultivation technique according to the present invention was employed in the experiment group. Seeds were sown on March 5, 2012, and harvested on May 1, 2012. The direct sowing method was employed. When the oil cabbage grew to have three leaves and one bud, spacing between the plants was 10 cm x 15 cm. Fertilizer application to the control group: nitrogen fertilizer (40% root fertilizer and 60% additional fertilization), and diammonium phosphate (50 g/m2) + potassium sulfate (25 g/m2). During the harvesting, each strain of plant was measured in terms of strain height, strain weight, leaf quantity, and biomass.

### 1.3. Results

For the sake of security of the data involved in the patent technology, only a portion of the data is disclosed herein, to describe the characteristics of the drift and suspension cultivation device. Some of the results are as listed in Table 11.

**Table 11 Growth characteristics of the oil cabbage in the experiment of drift and suspension cultivation**

| Treatment | Plant height (cm) | Plant weight (g/plant) | Quantity of leaves leaves/plant | Biomass (g/10 plants) | Production increase (%) |
|---|---|---|---|---|---|
| Control group | 24.7 | 40.8 | 10.5 | 615.3 | -- |
| Experiment group | 52.2 | 84.9 | 12.3 | 1304.7 | 212.04% |

### Embodiment 8 Application of drift and suspension cultivation technique in wheat according to the present invention

The growth process of the wheat generally covers 12 stages: seed sowing, sprouting, tiller stage, overwintering, growth resuming, standing up, jointing, leaf flagging, heading, blooming, milky ripeness, wax ripeness, and ripe.

In the meantime, from sowing to harvesting, the wheat shall experience different fundamental changing stages. In different fundamental changing stages, unique organs of the wheat are formed. In each of the growth stages, the wheat needs not only overall external conditions, but also takes one of the conditions as a dominant factor. In addition to differences between the dominant factors in the growth stages, the growth stages are subjected to orderly limitation and irreversibility. Herein the detailed description is given with reference to two commonly concentrated growth stages at present, vernalization stage and illumination stage.
(I) The vernalization stage, which is also referred to as a temperature sensitive stage, is the first growth stage of the wheat. In the vernalization stage, comprehensive conditions such as illumination, temperature, water, air, and nutritious substances are desired, and the low temperature condition plays the most important role, achieving a dominance effect. The low temperature condition may be successfully satisfied upon sprouting of the seeds or in the young seeding stage. The low temperature degree and experienced time of different categories of plants in the vernalization stage are subject to a notable difference.
(III) Illumination stage, which is also referred to the light sensitive stage, is a stage of the wheat that follows the vernalization stage. In the illumination stage, the wheat needs not only overall environment conditions, but also takes duration of the illumination per day as a dominant factor. If the illumination duration is insufficient, heading of the wheat may fail. On the contrary, if the illumination is constantly supplied, the heading of the wheat is quickened. The sensitive degrees the illumination reaction are different depending on different specifies of wheat.

The growth period of the wheat is generally 240 days. By means of application of the device according to the present invention, the growth period may be at least shortened by 140 days, and one generation of the wheat may mature and may be harvested after 100 days or around. In this way, three generations of wheat may be harvested each year.

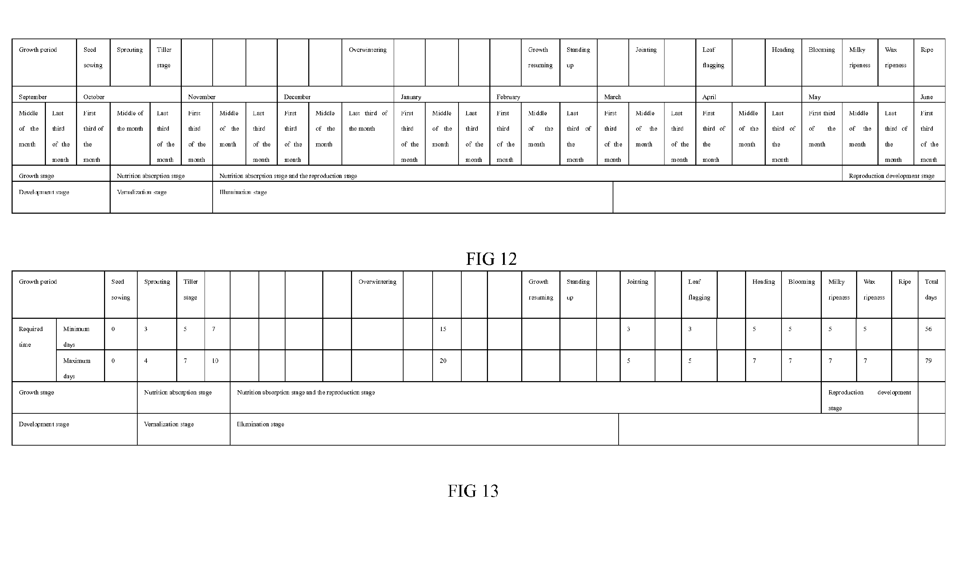

The current growth and development process of the wheat is as illustrated in FIG. 12. By means of application of the device according to the present invention, the growth and development of the wheat is illustrated in FIG. 13.

### Embodiment 9 Application equipment of suspension in seawater treatment according to the present invention

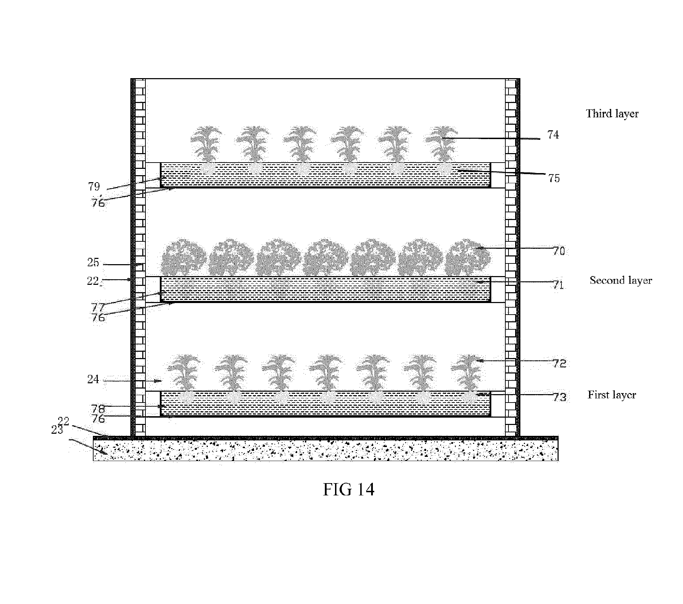

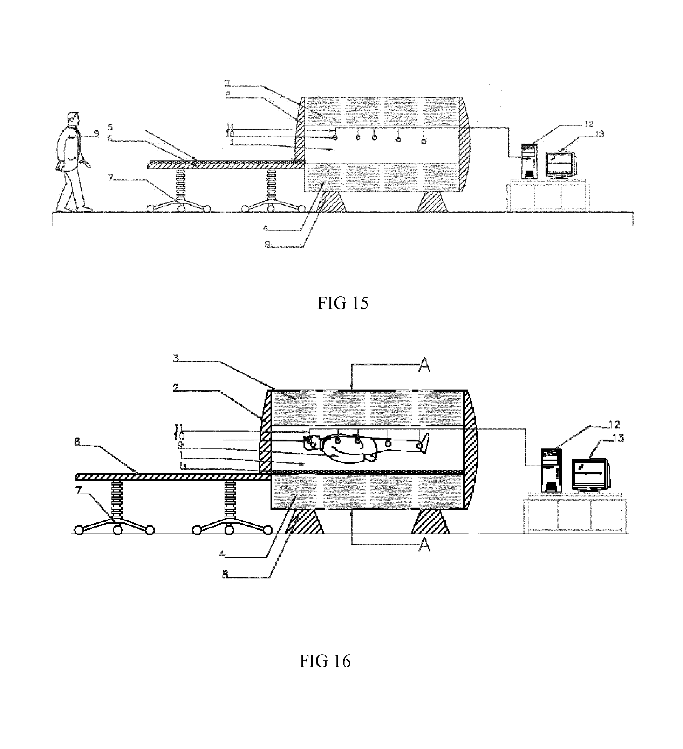

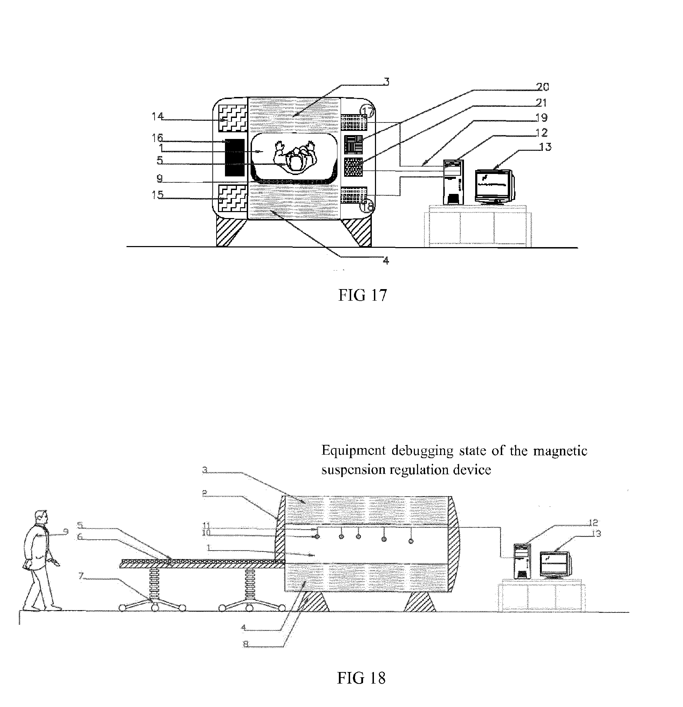

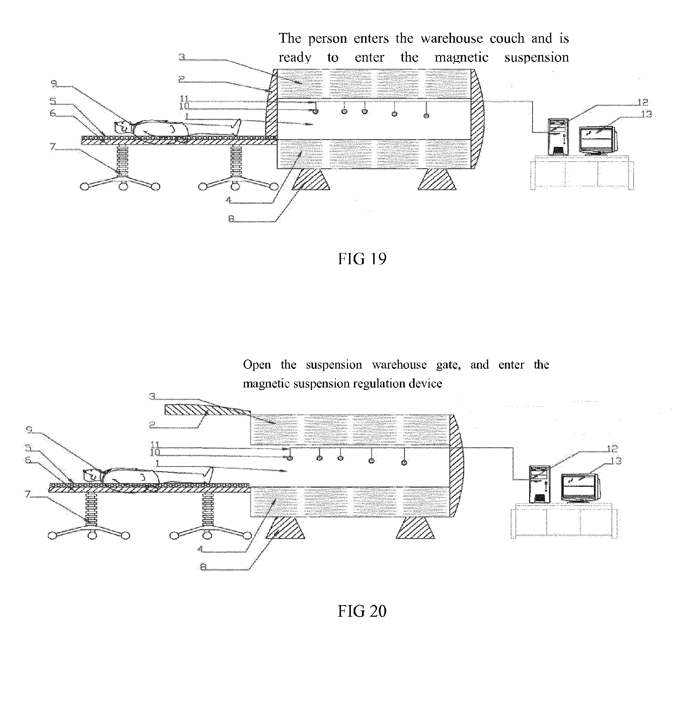

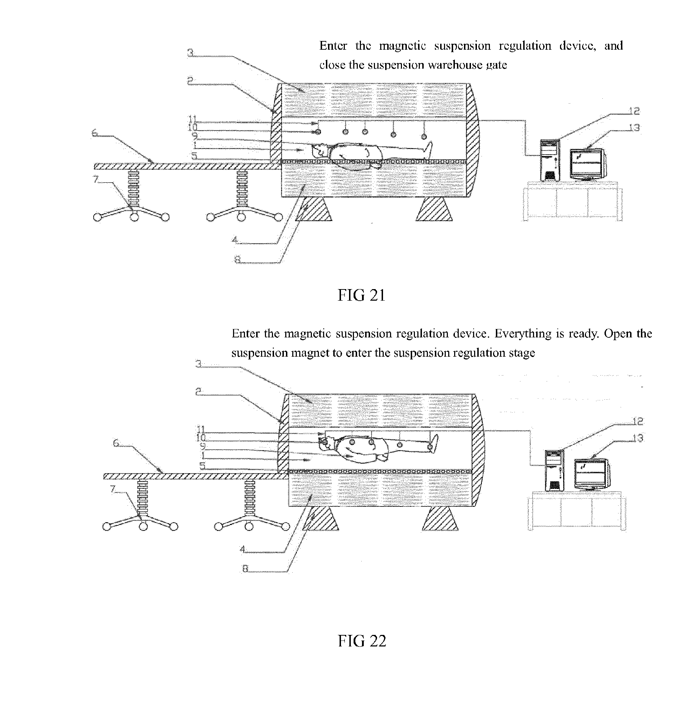

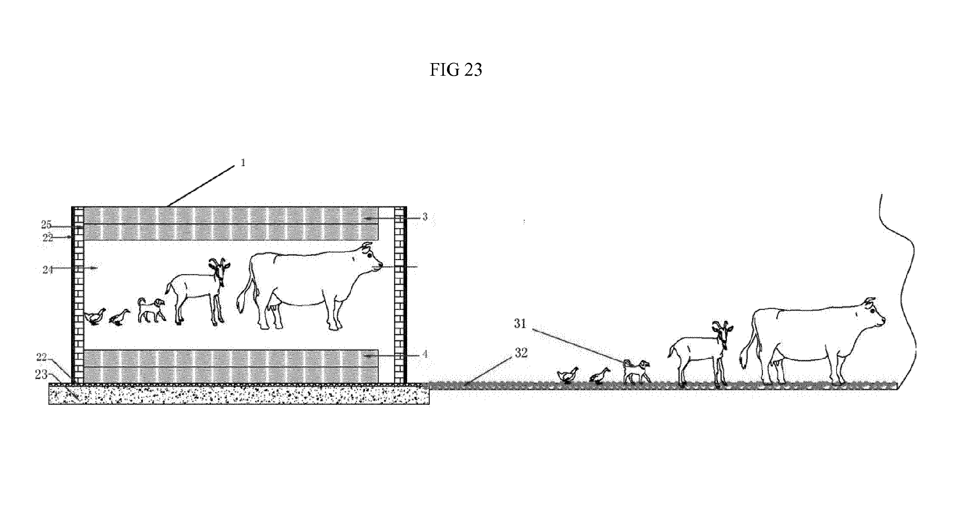

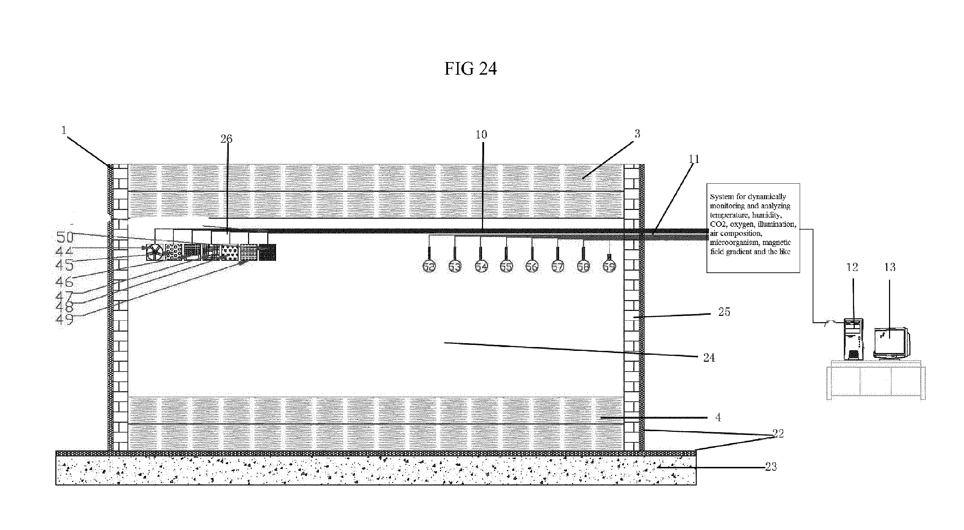

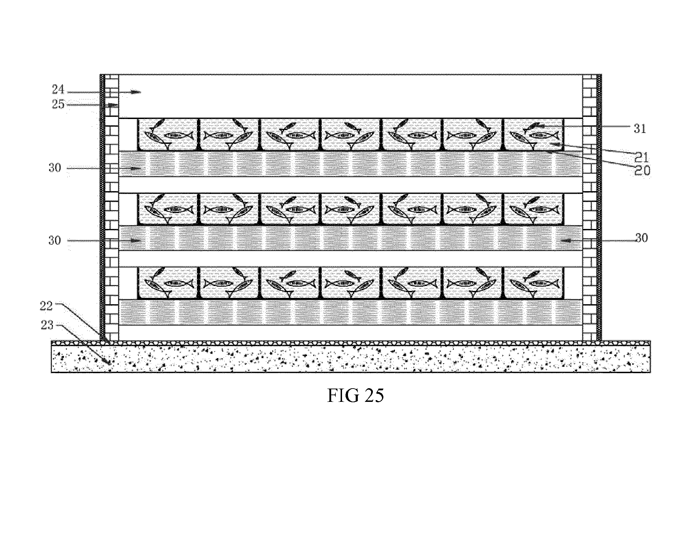

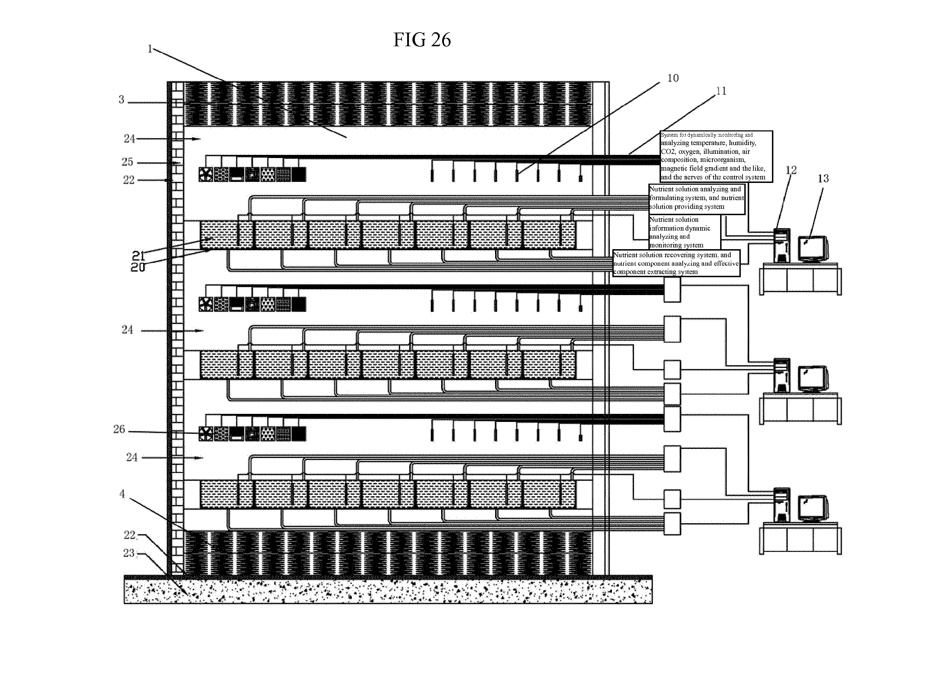

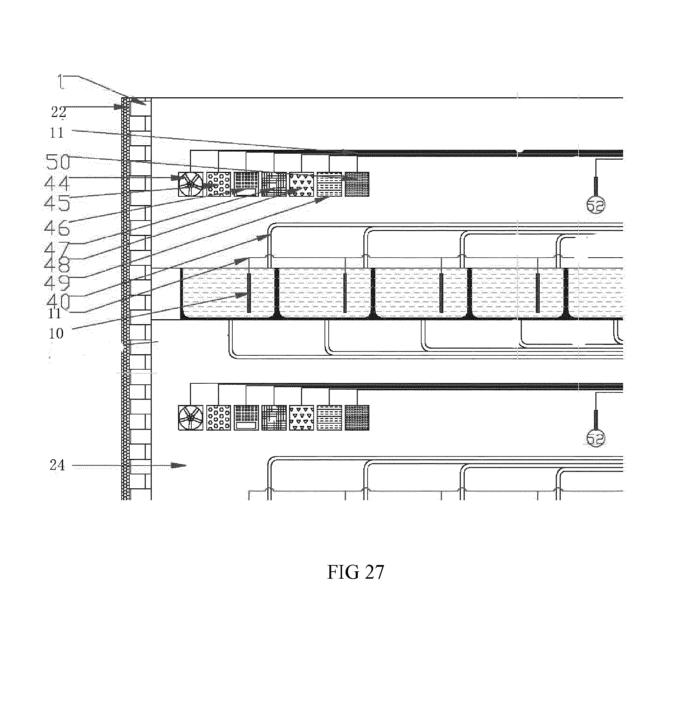

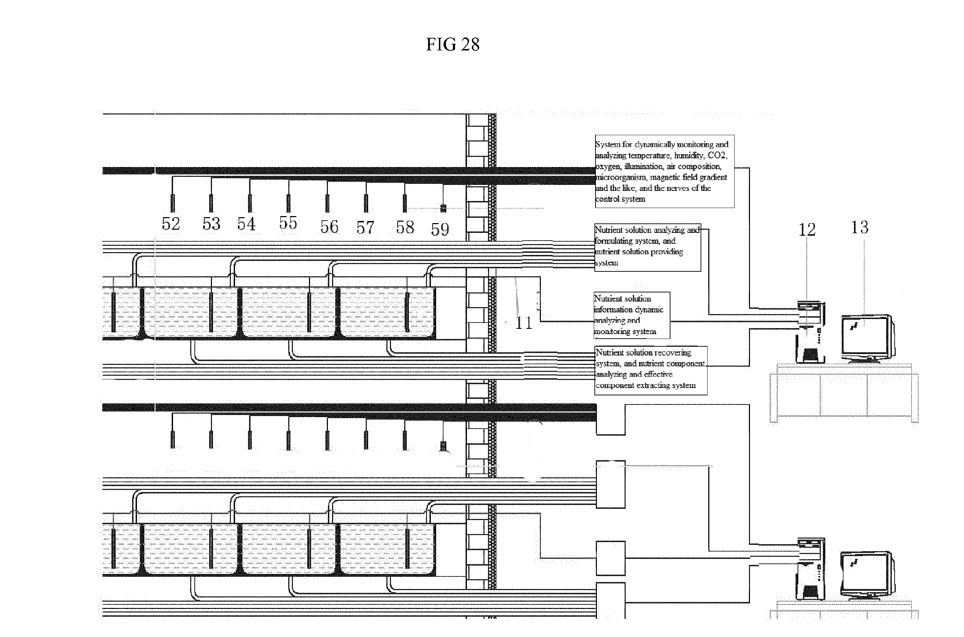

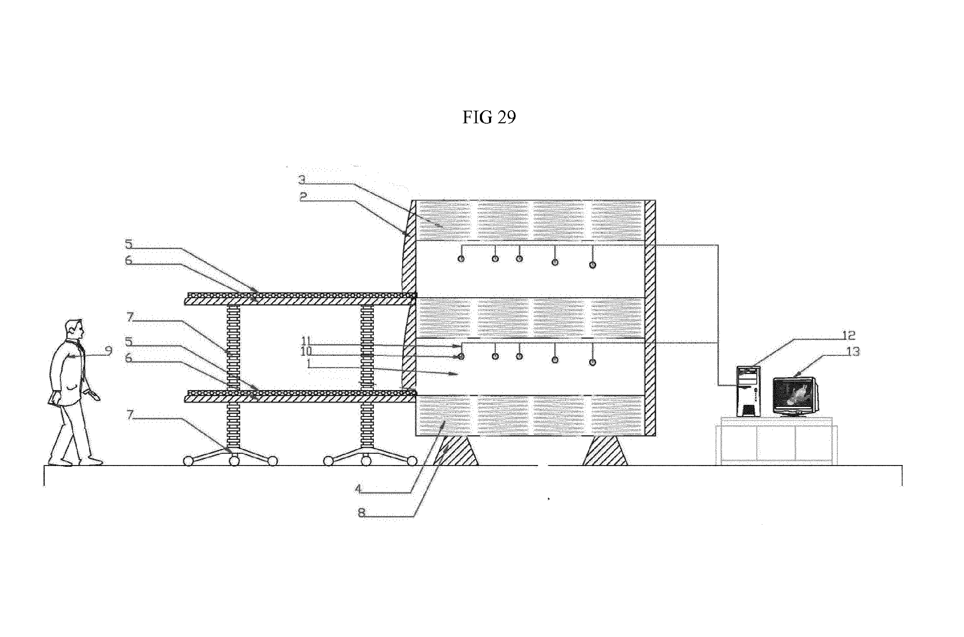

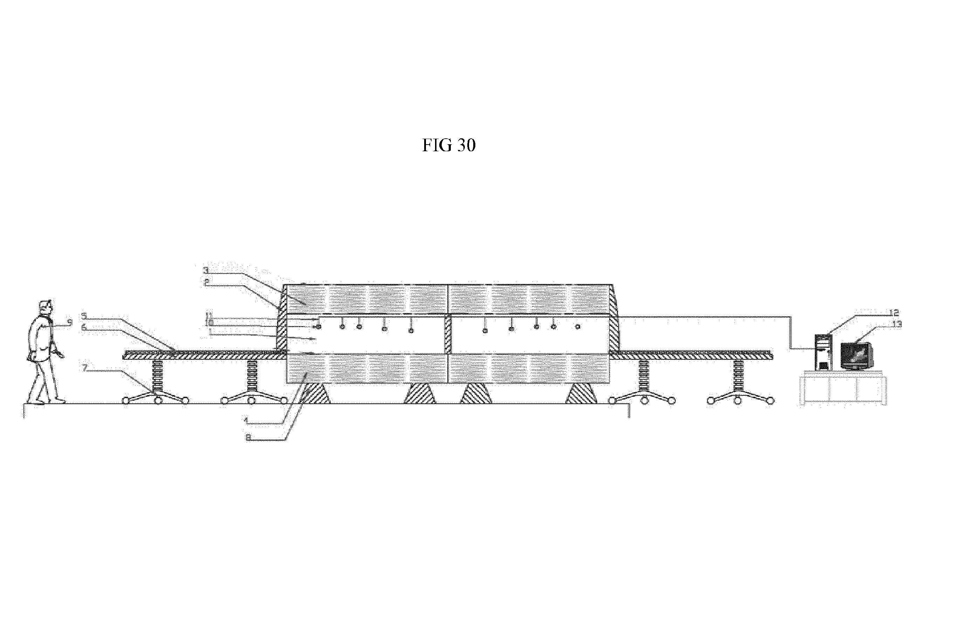

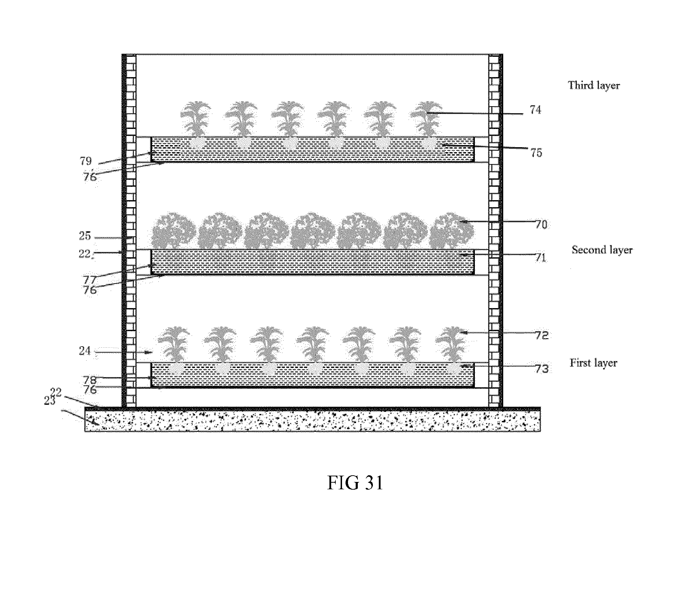

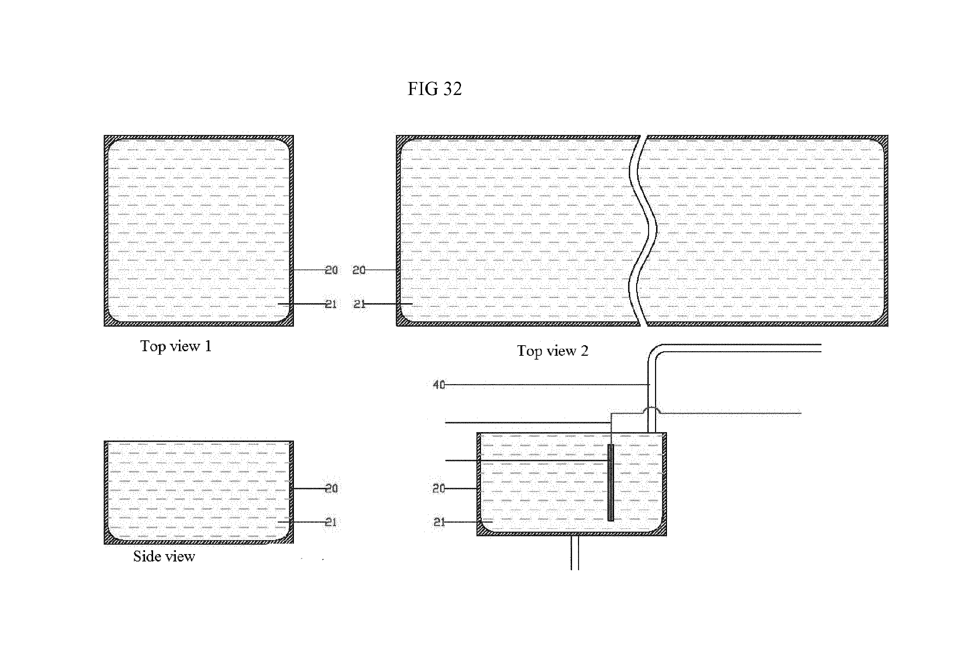

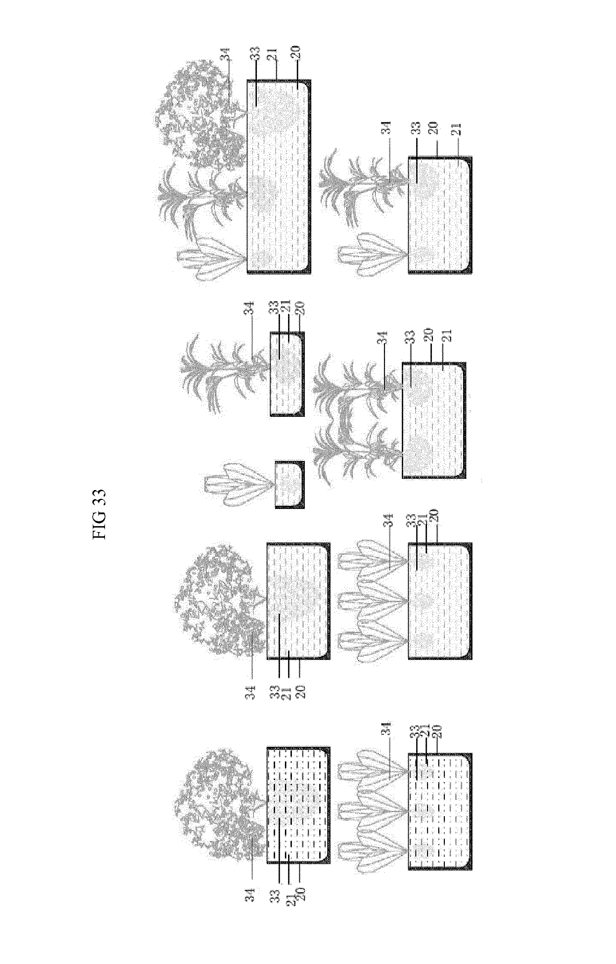

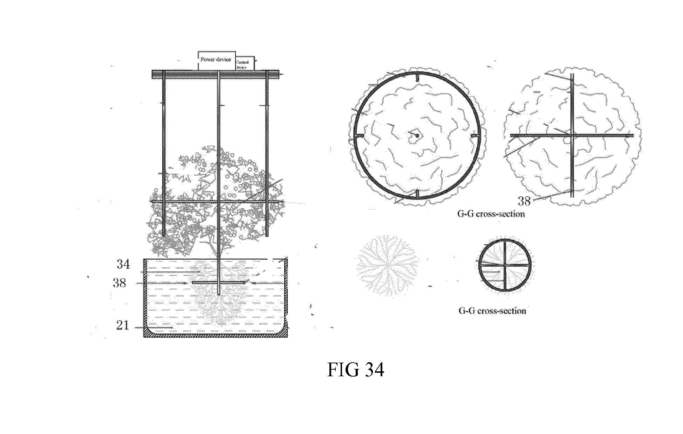

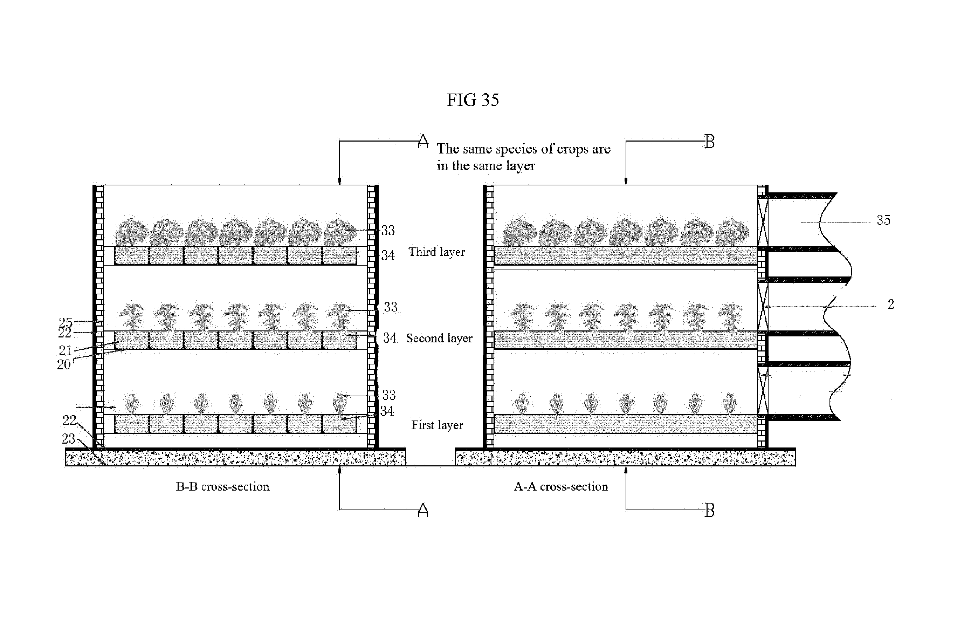

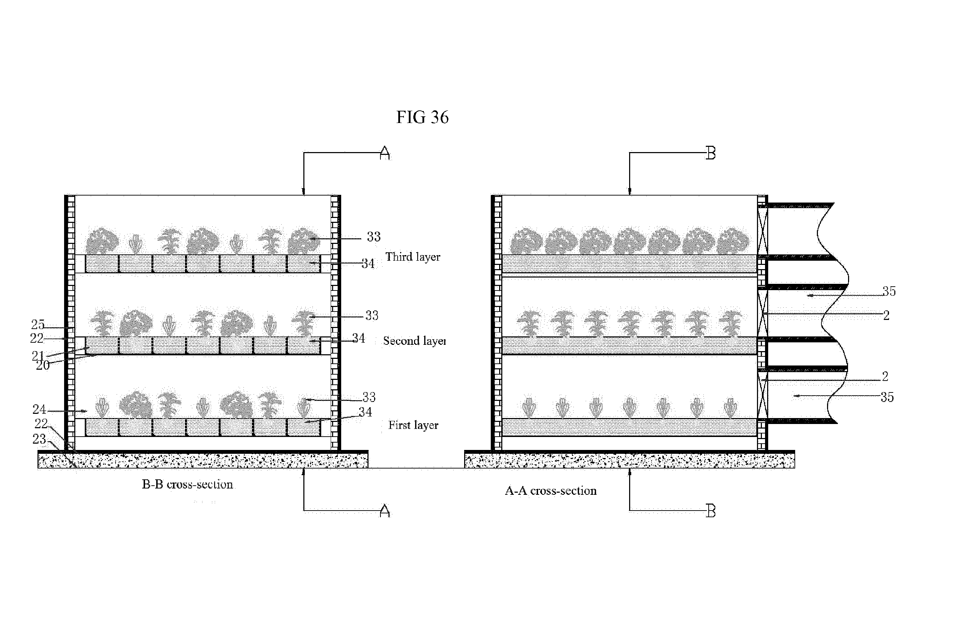

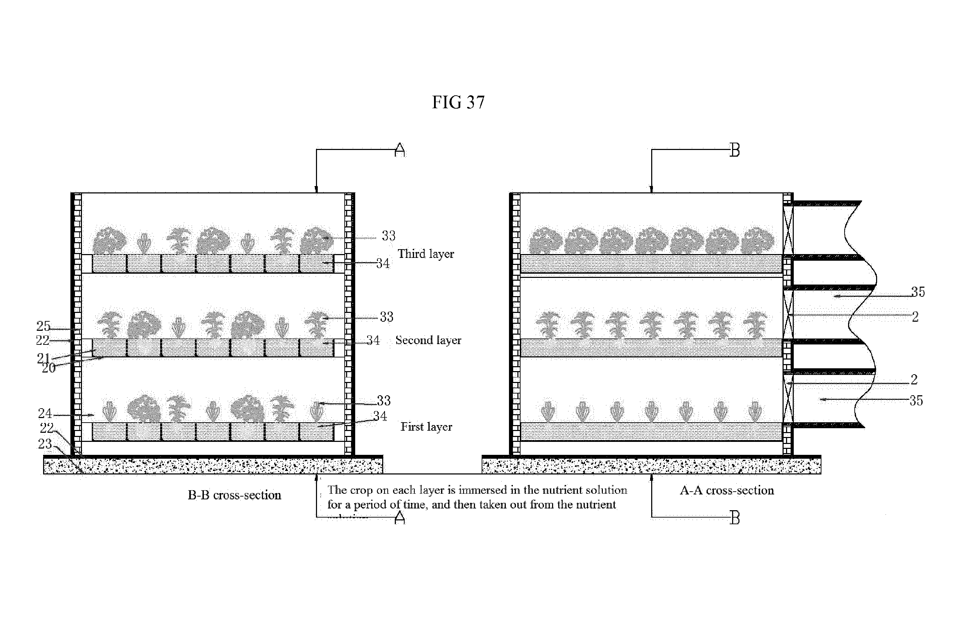

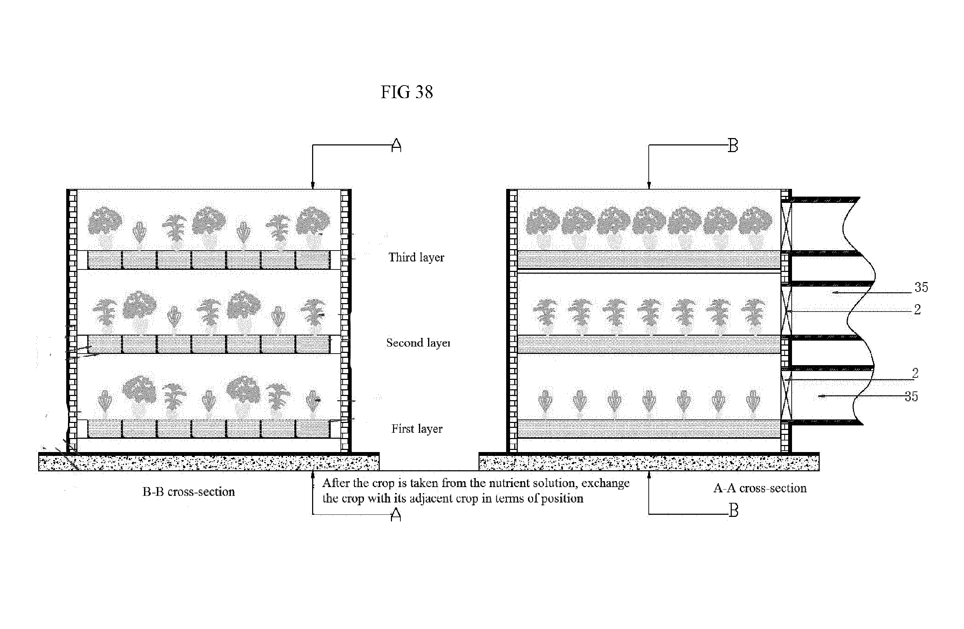

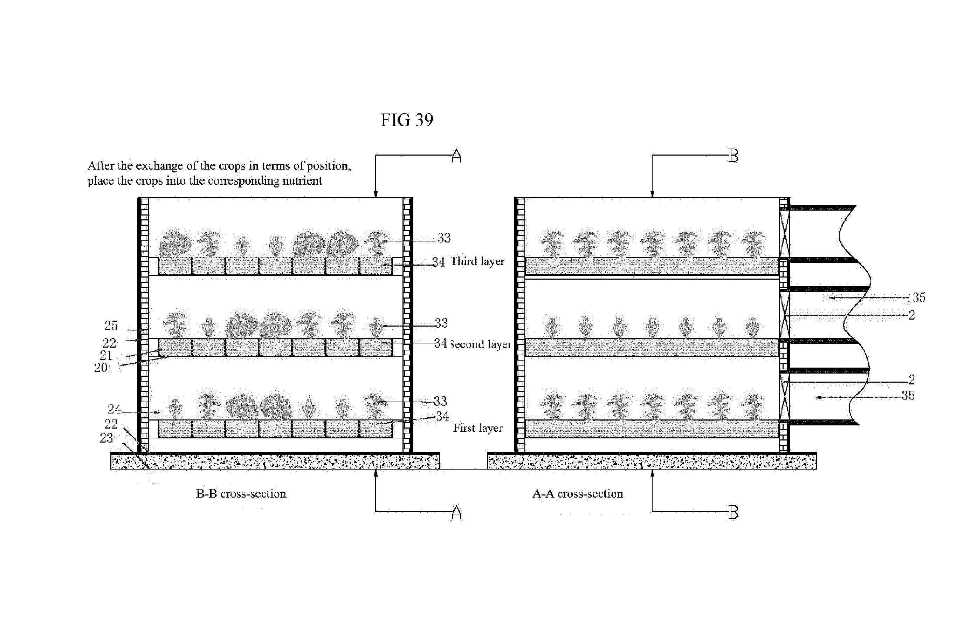

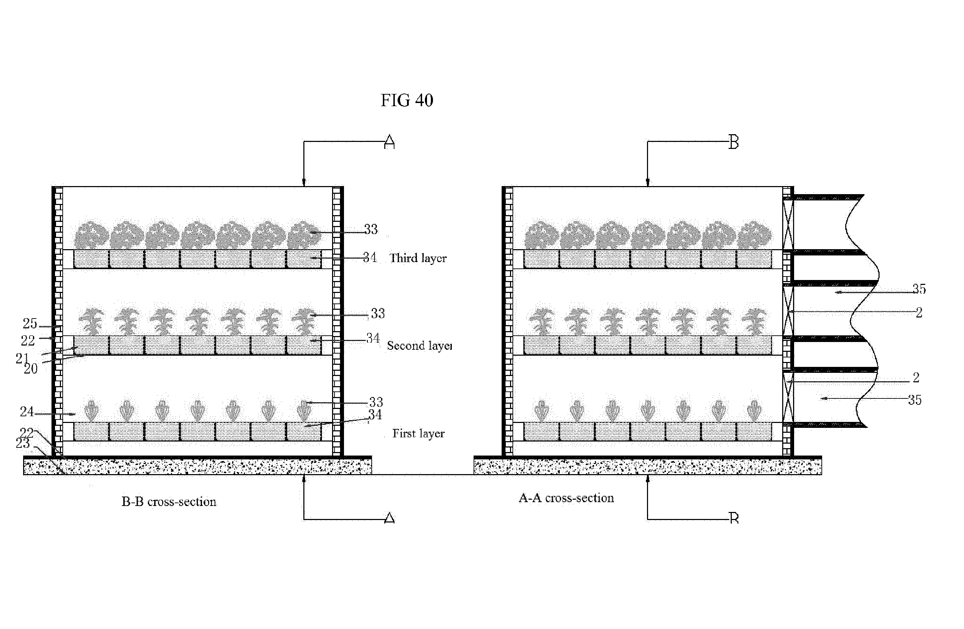

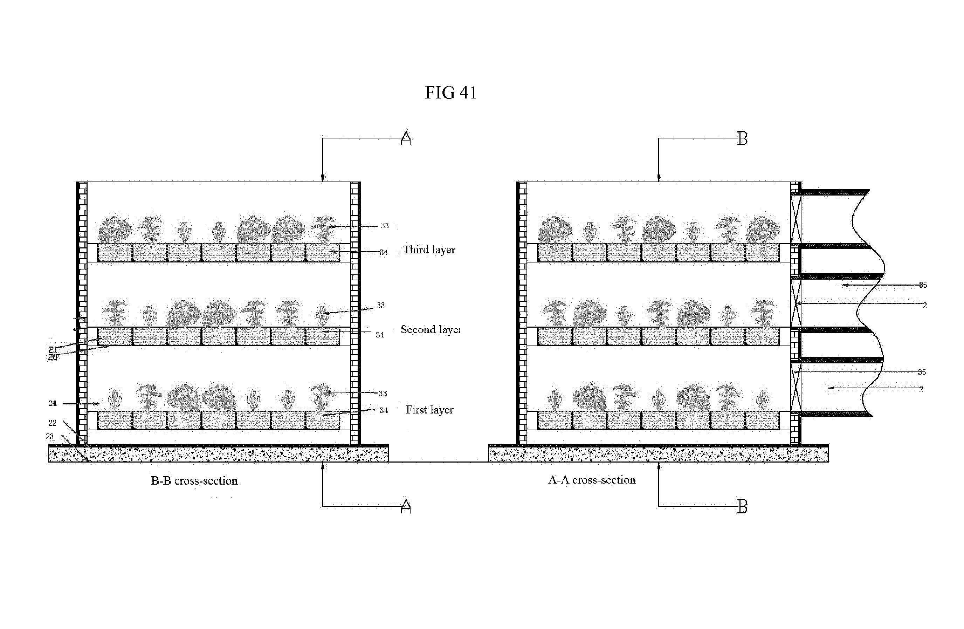

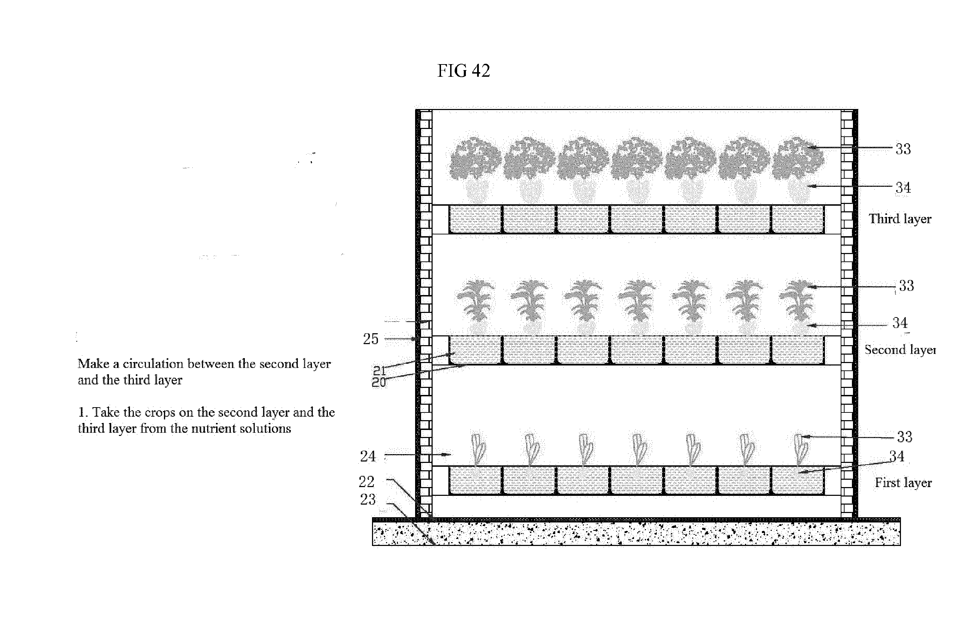

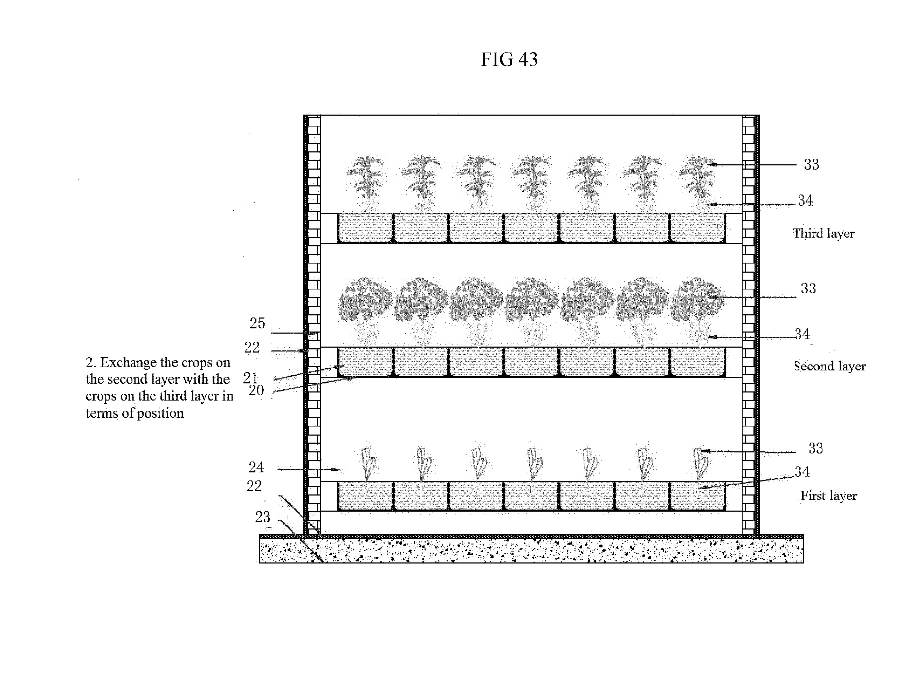

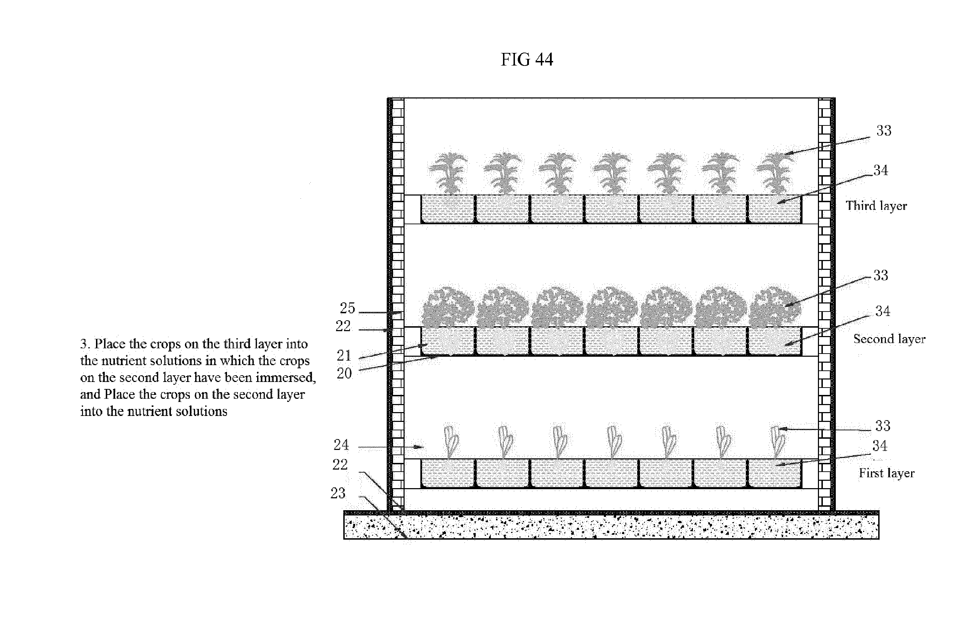

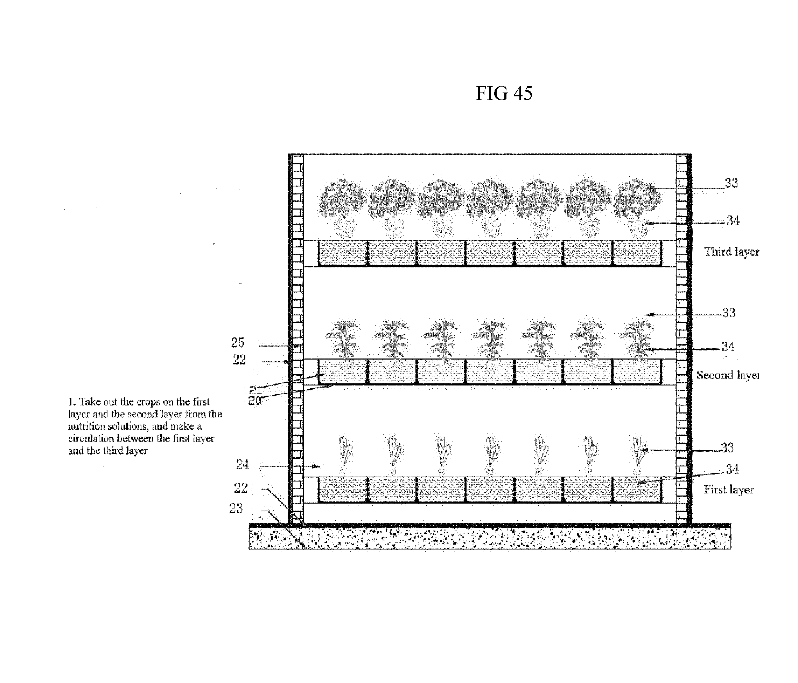

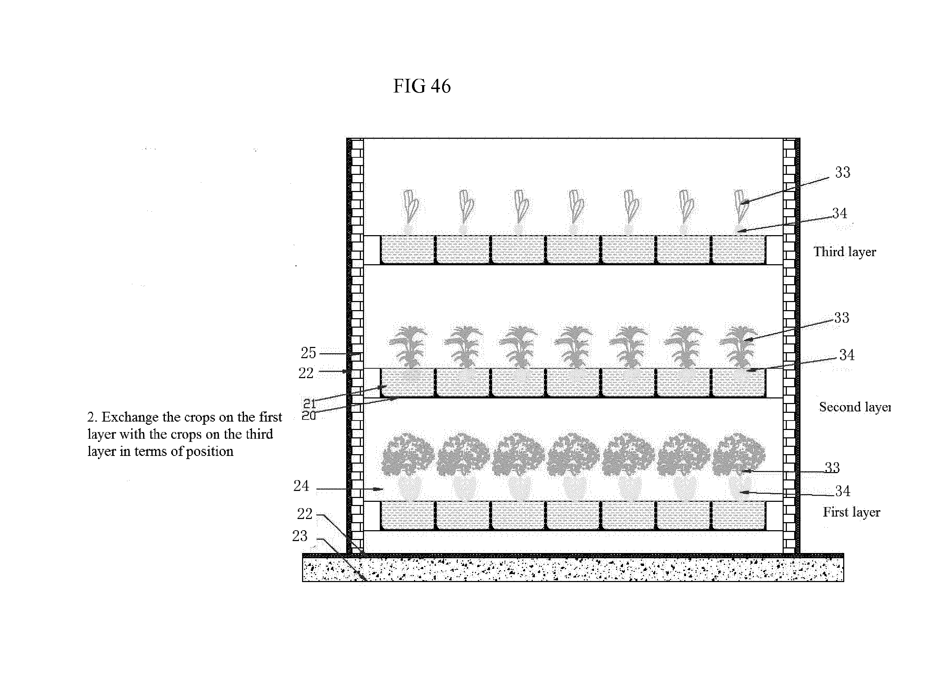

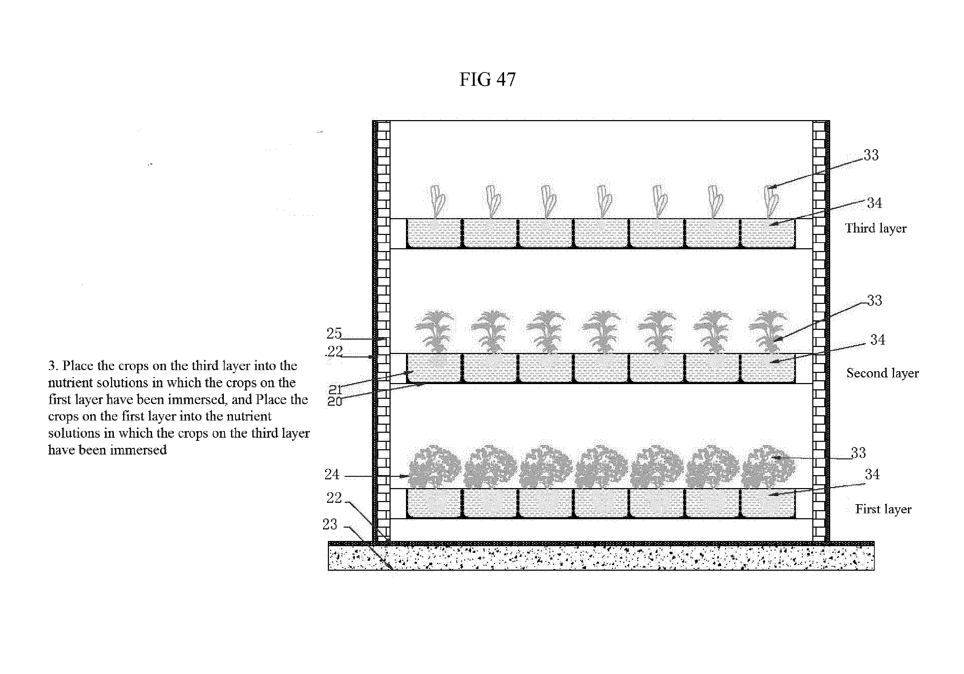

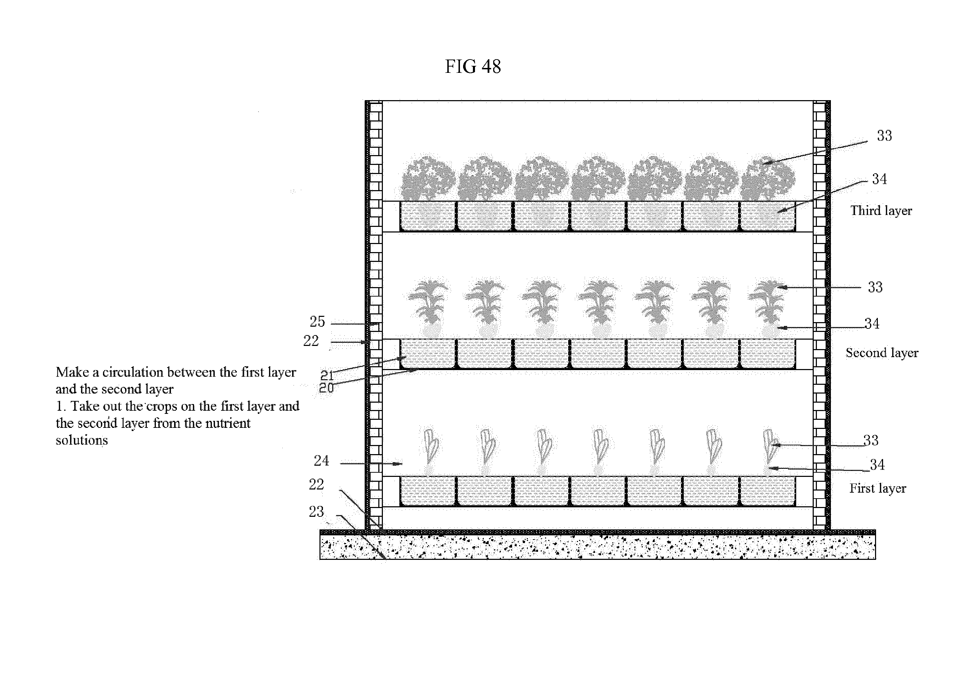

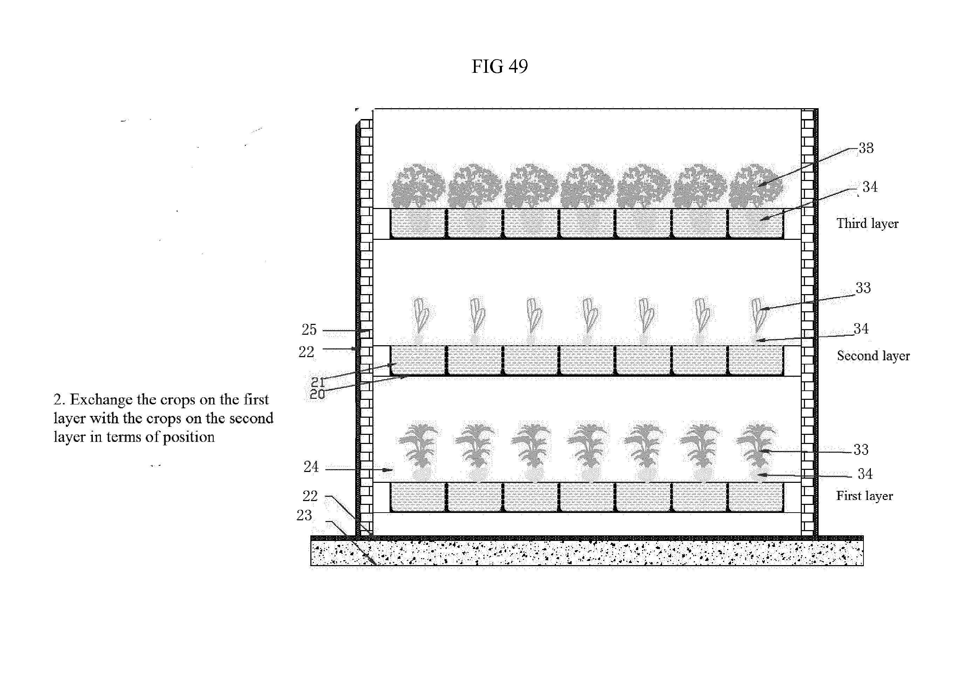

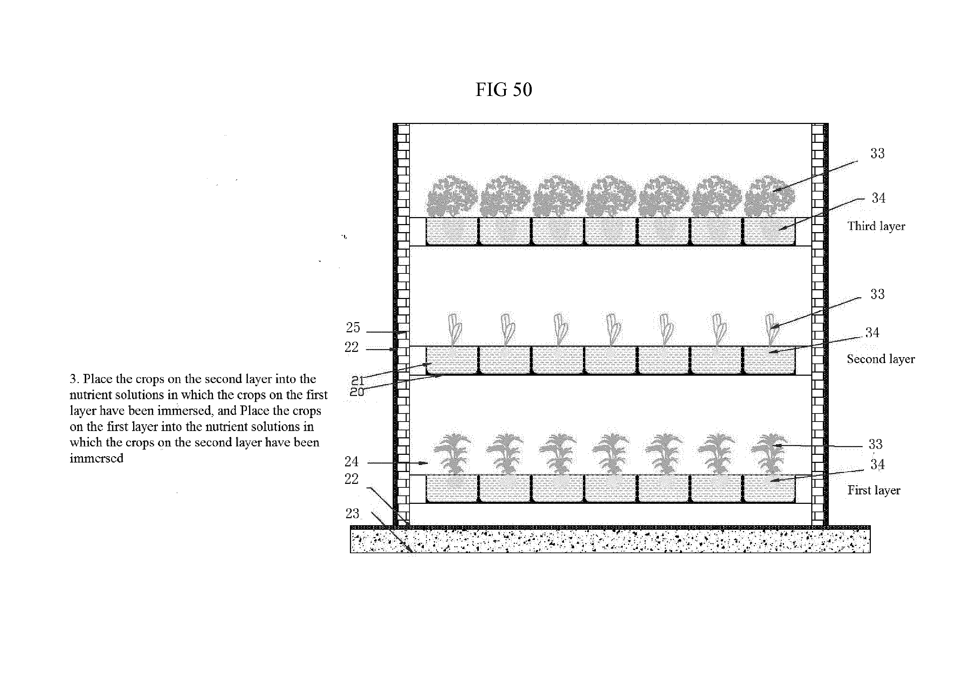

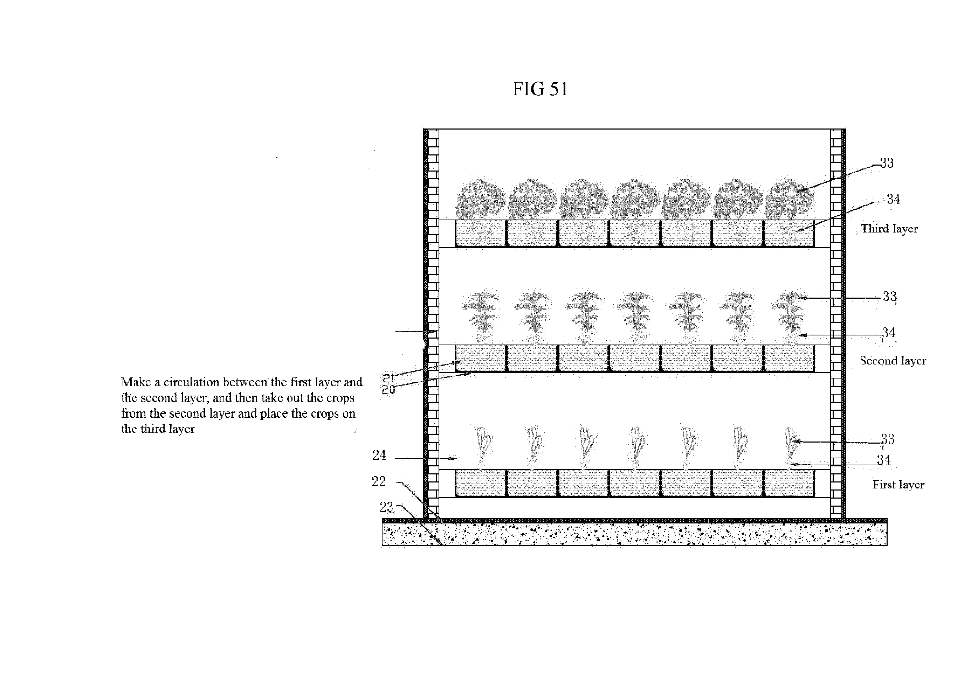

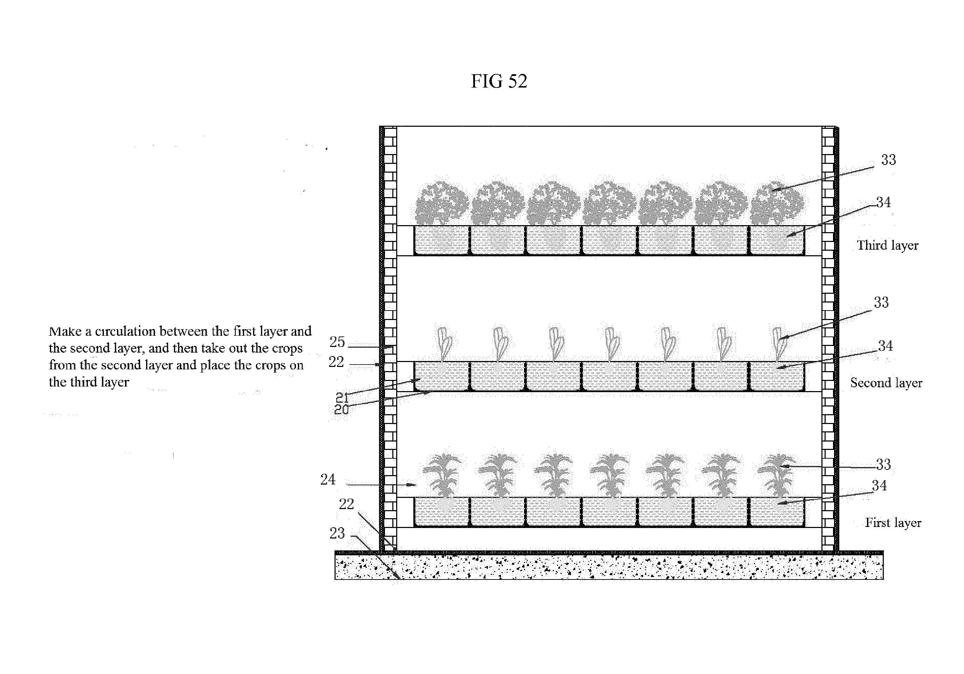

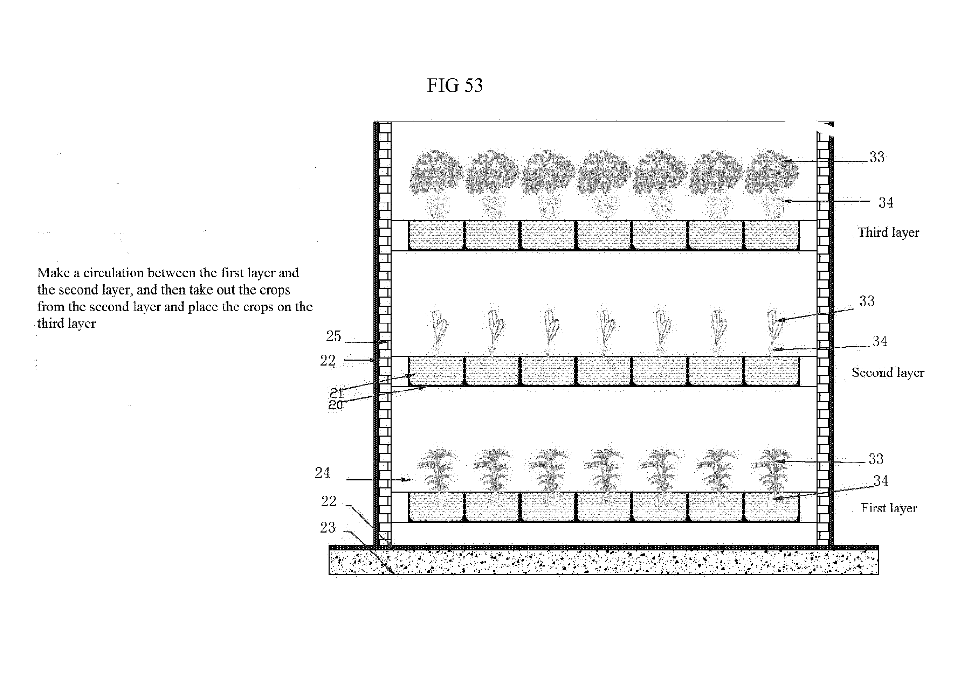

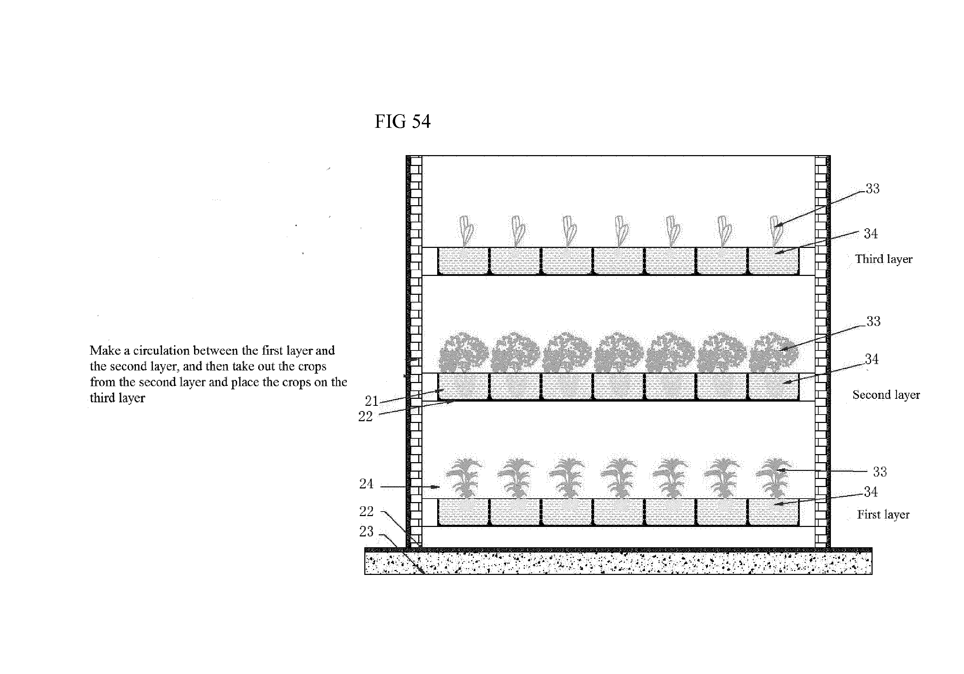

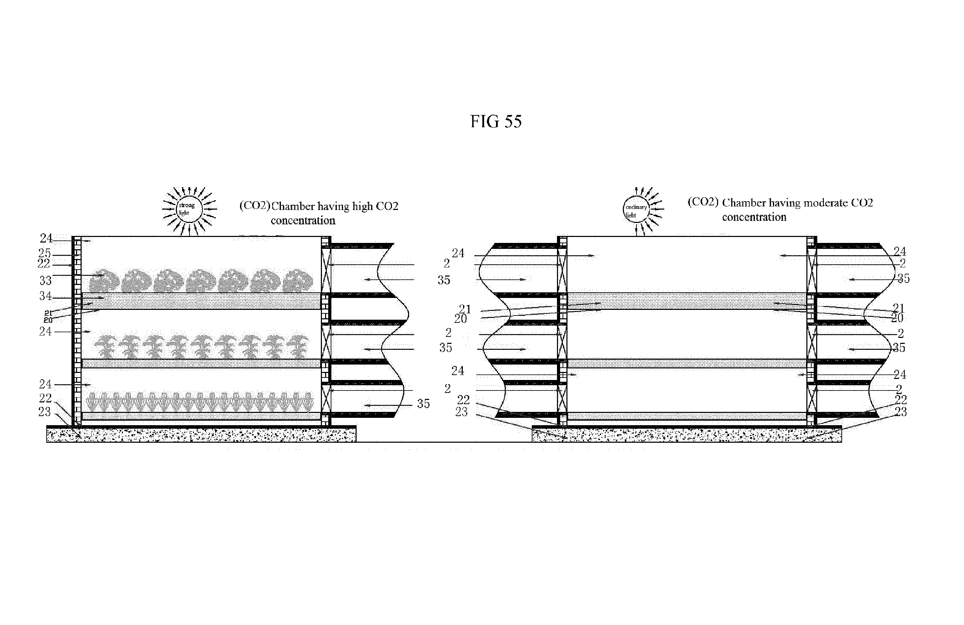

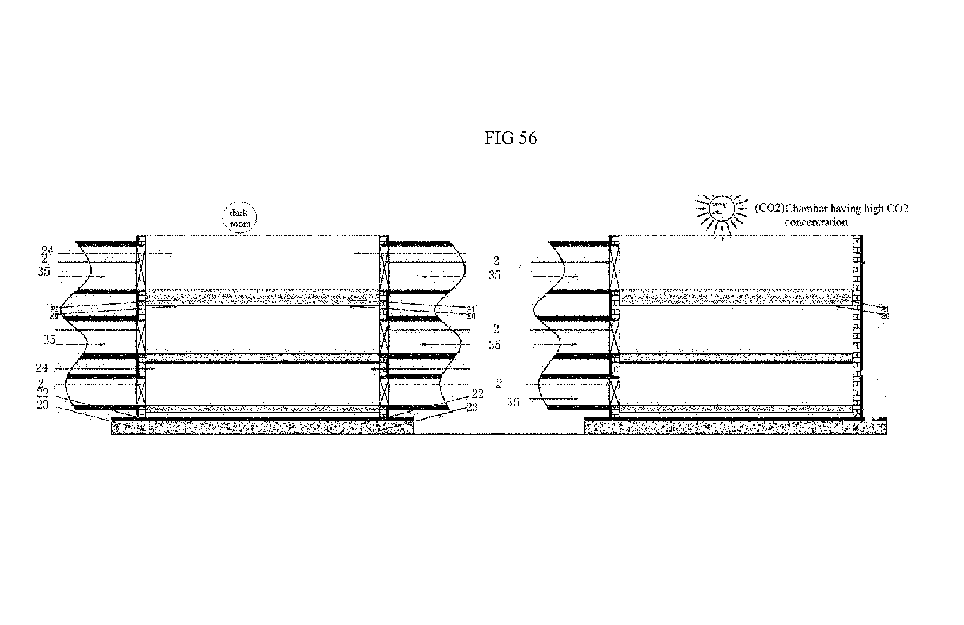

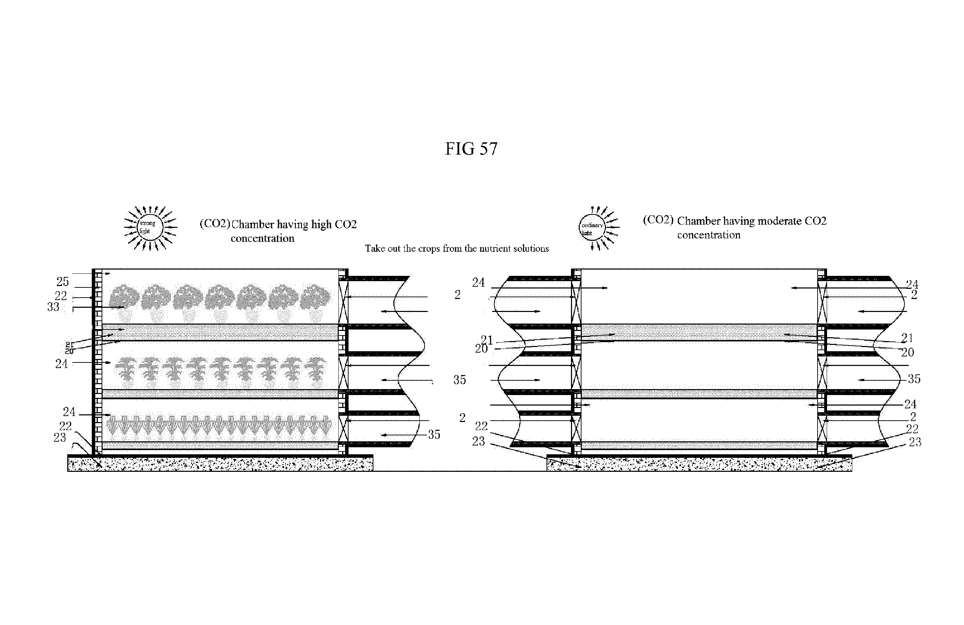

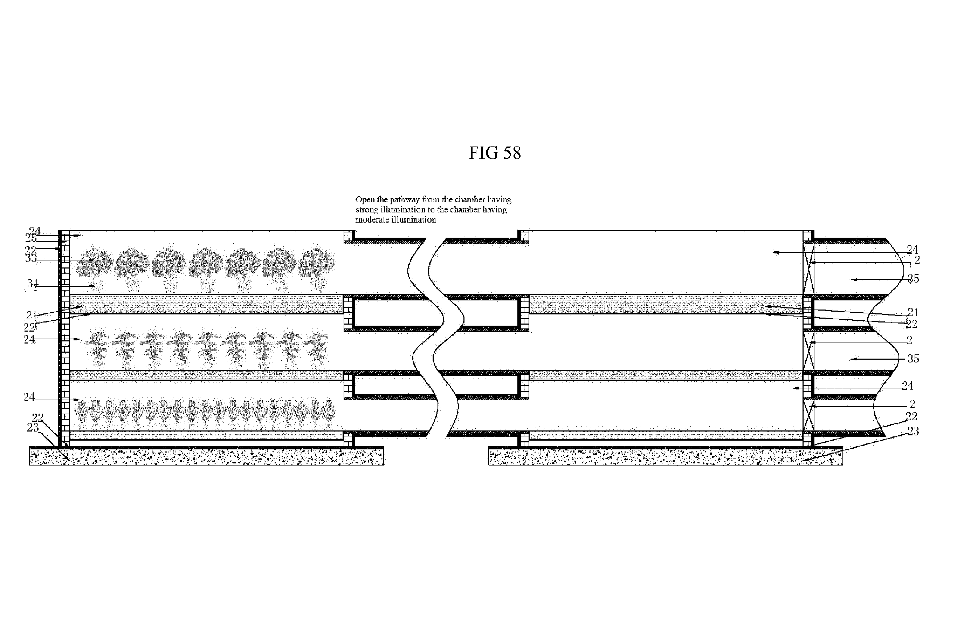

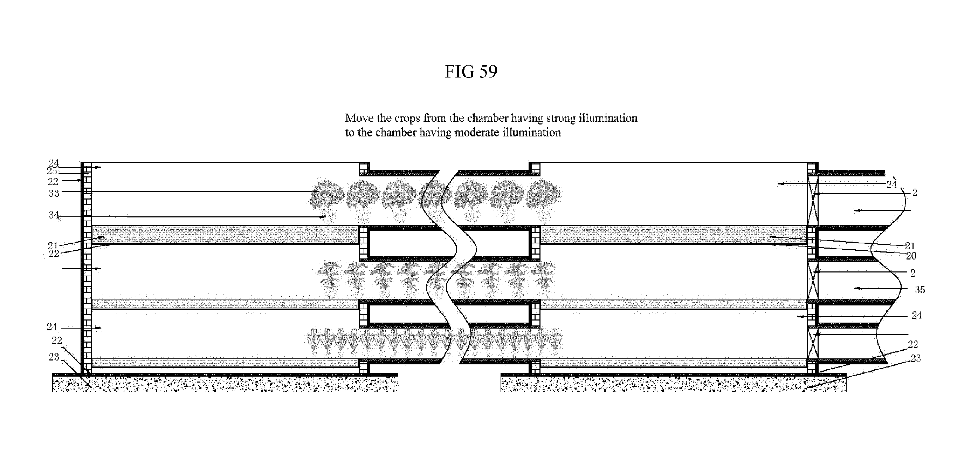

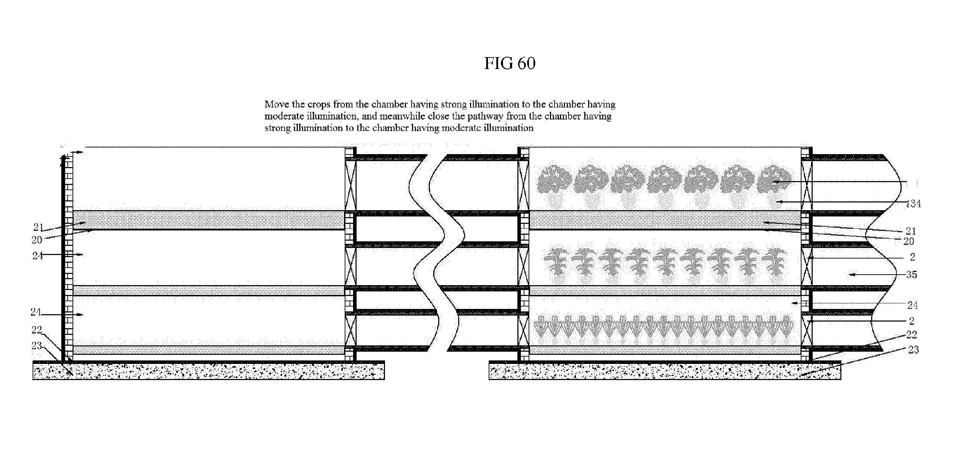

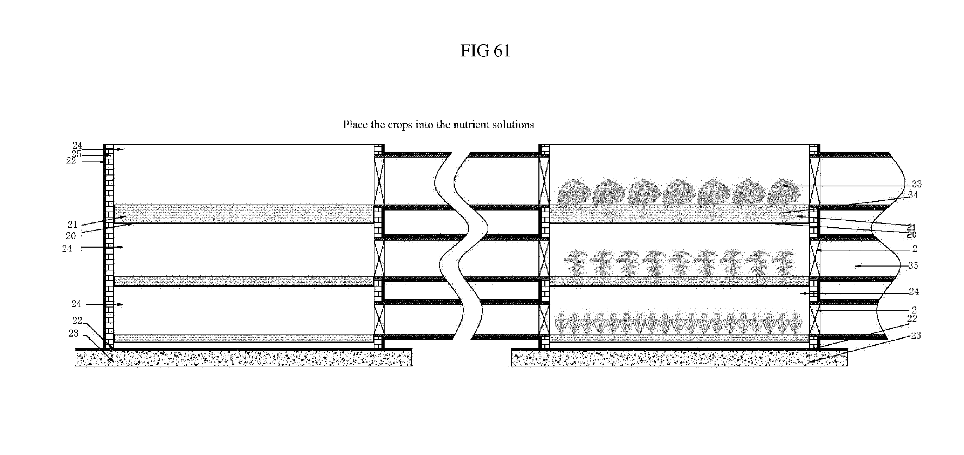

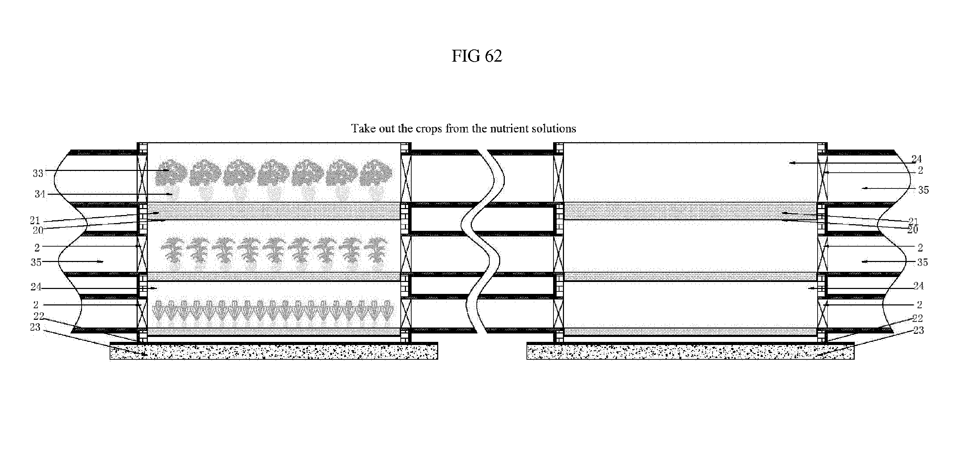

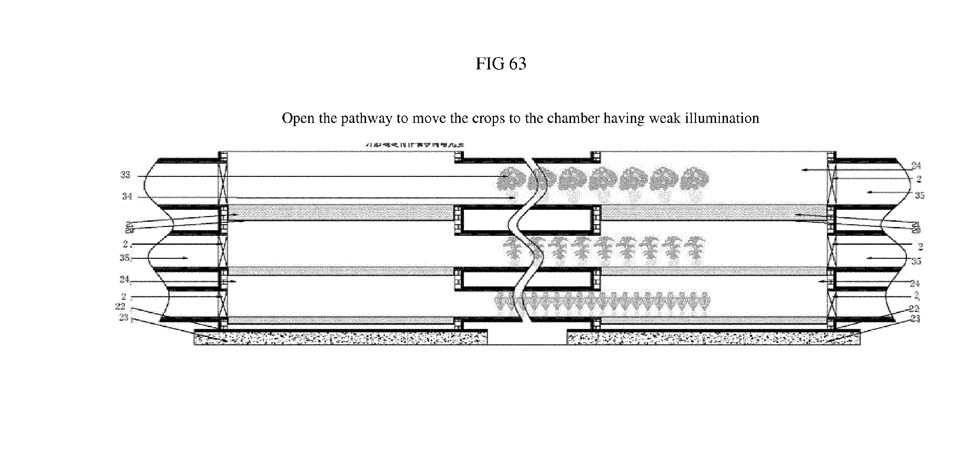

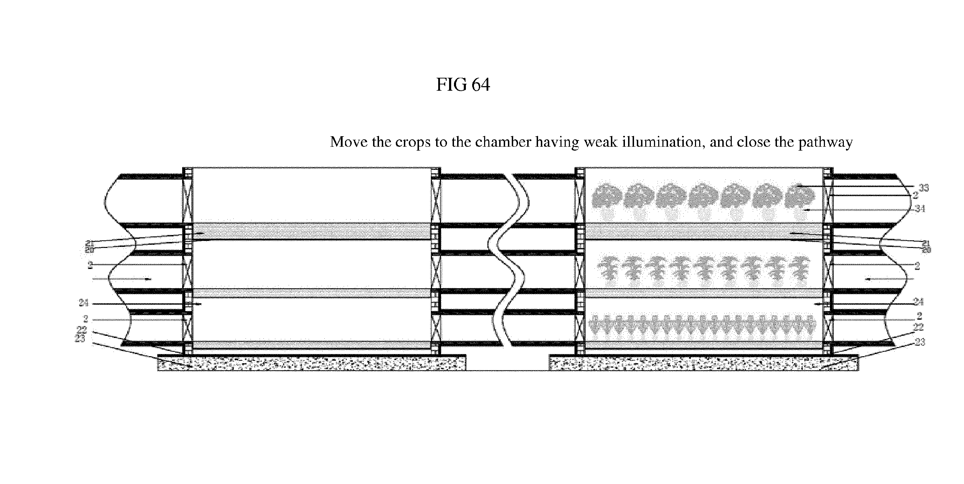

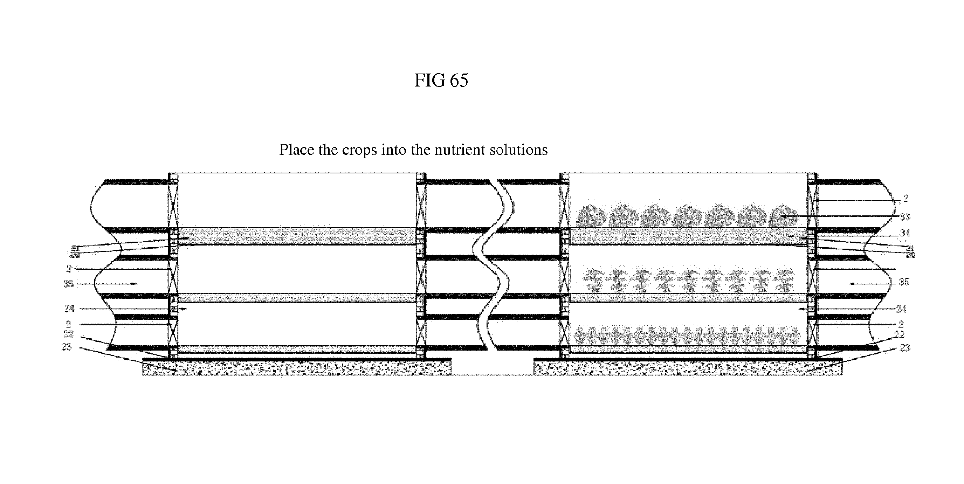

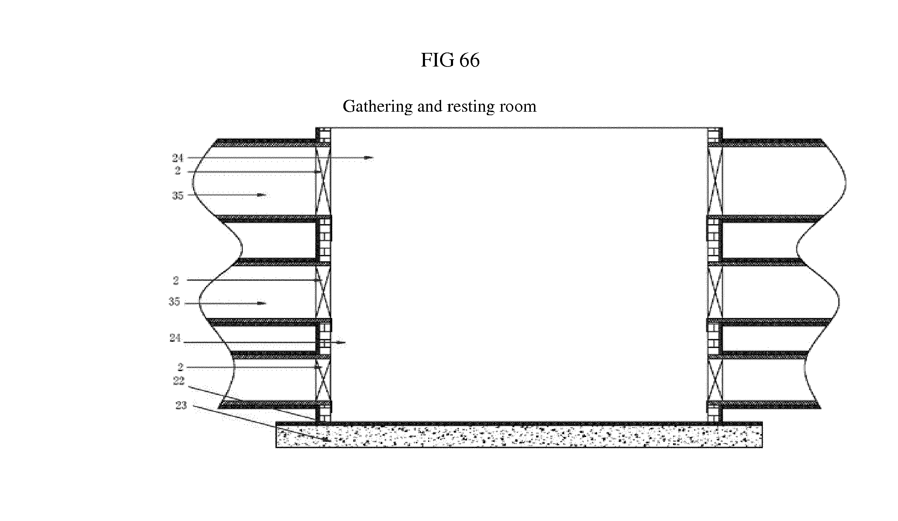

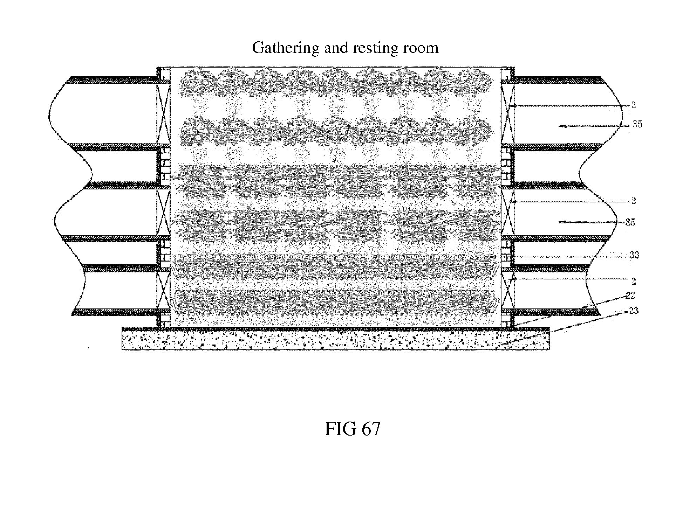

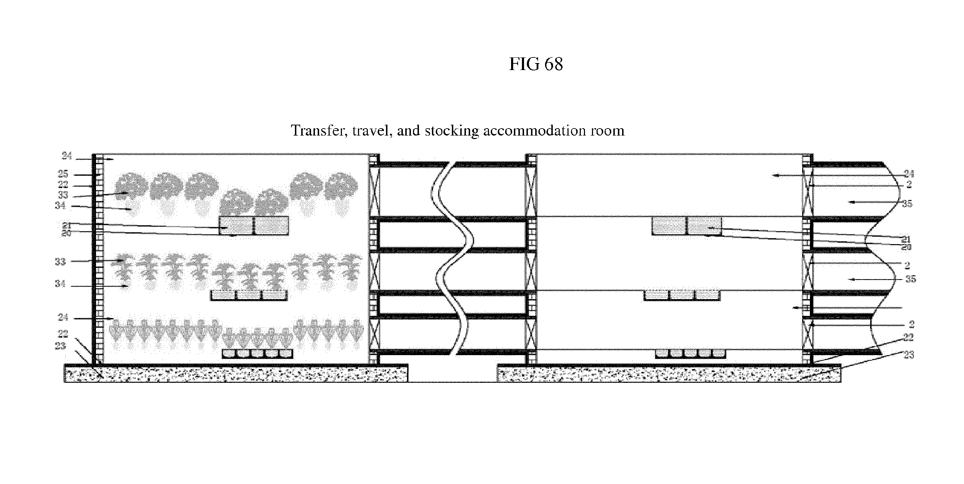

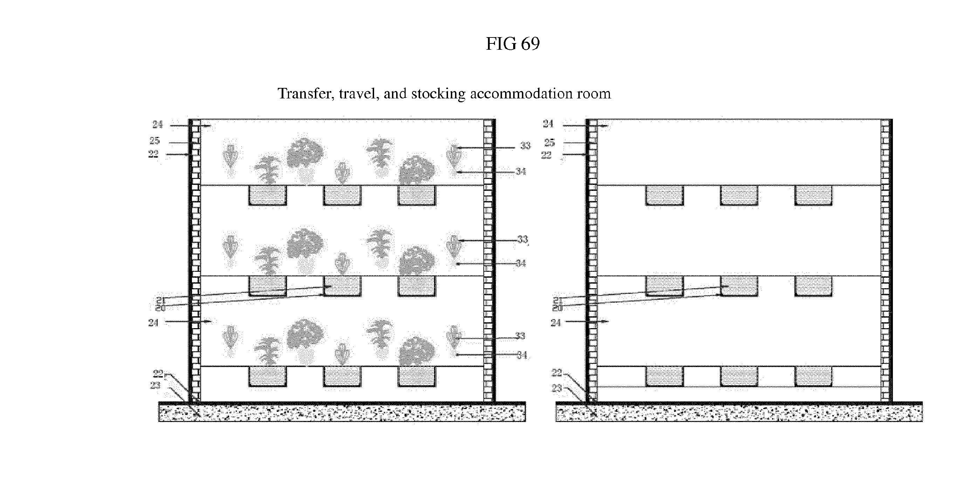

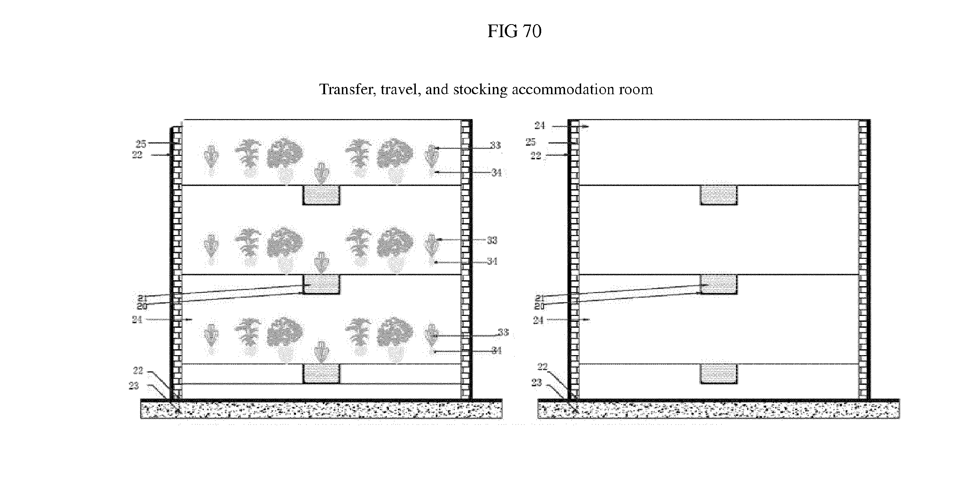

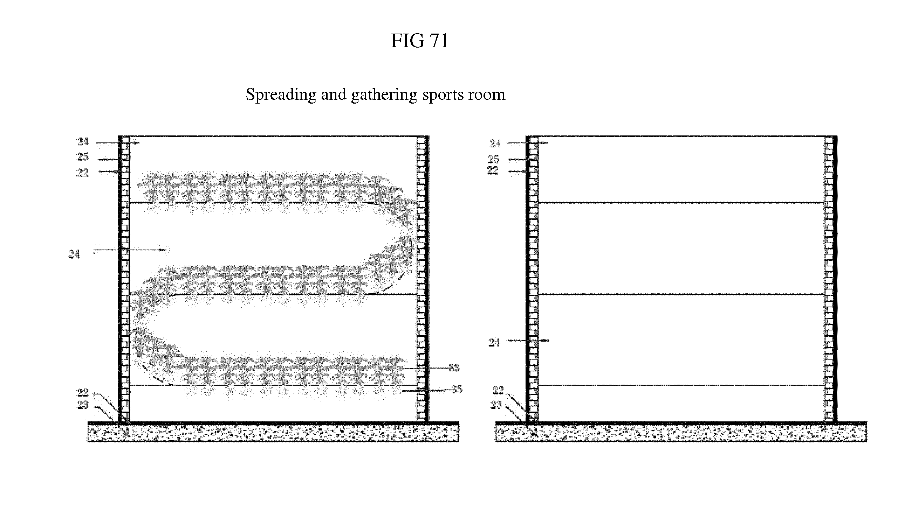

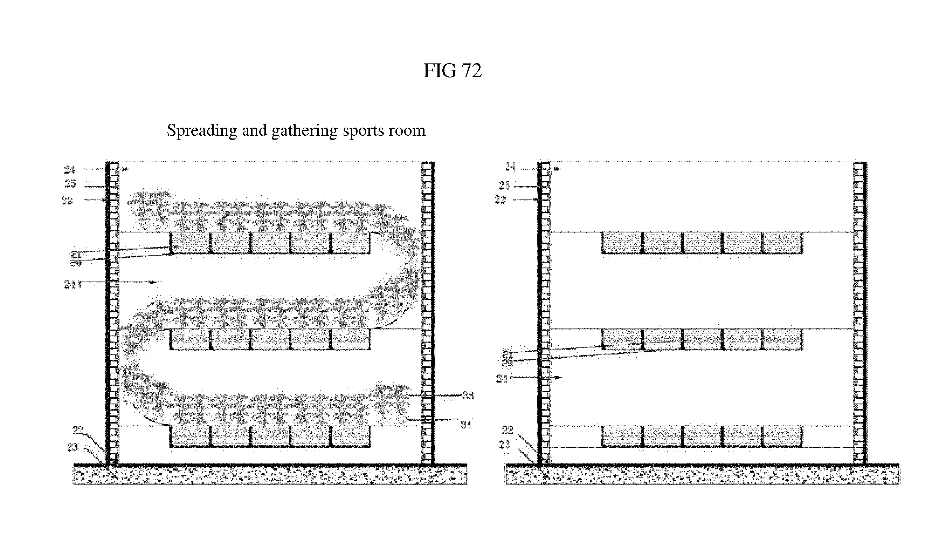

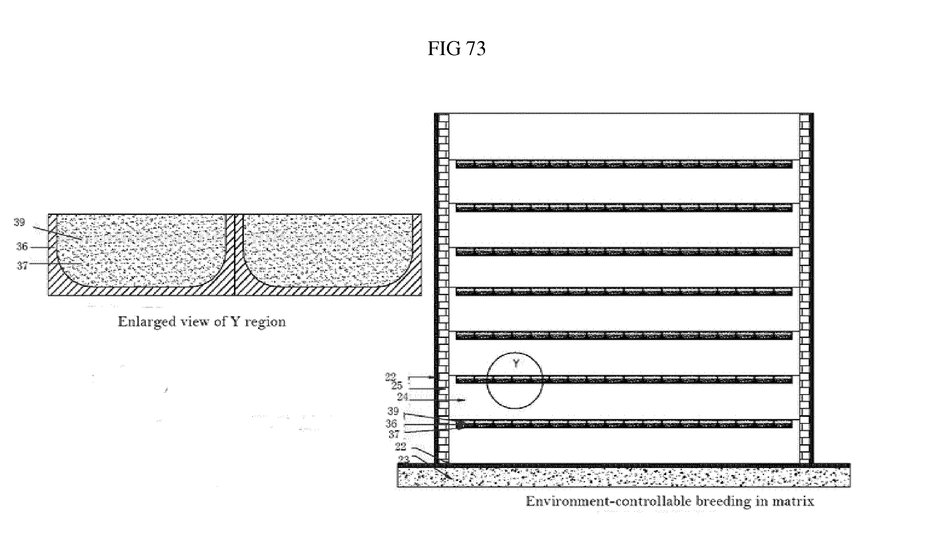

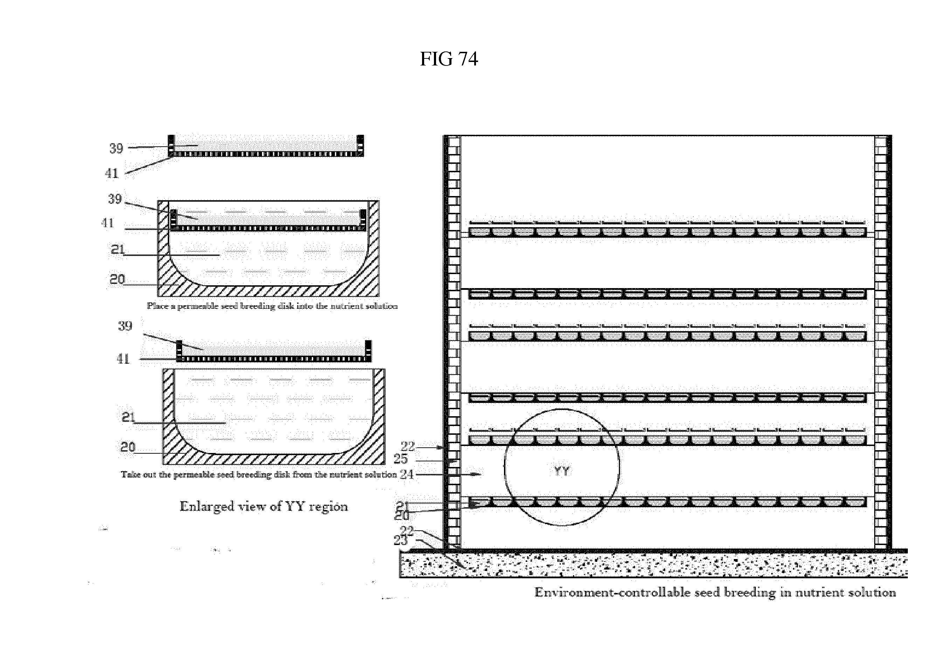

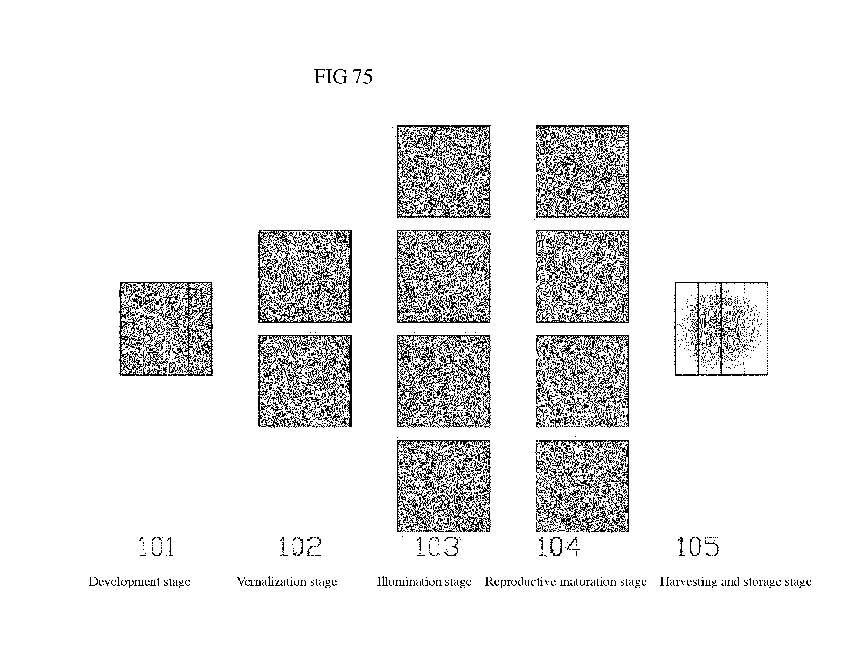

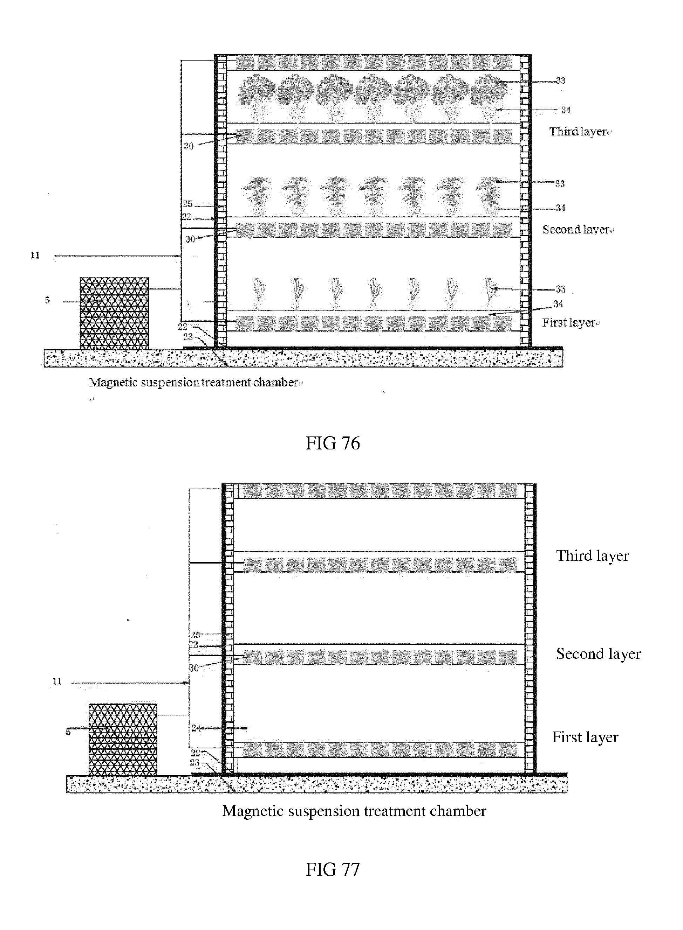

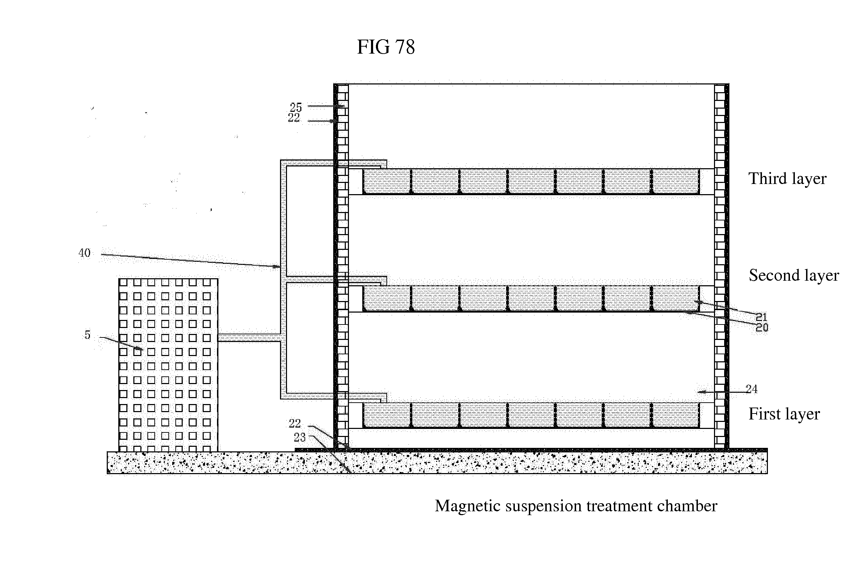

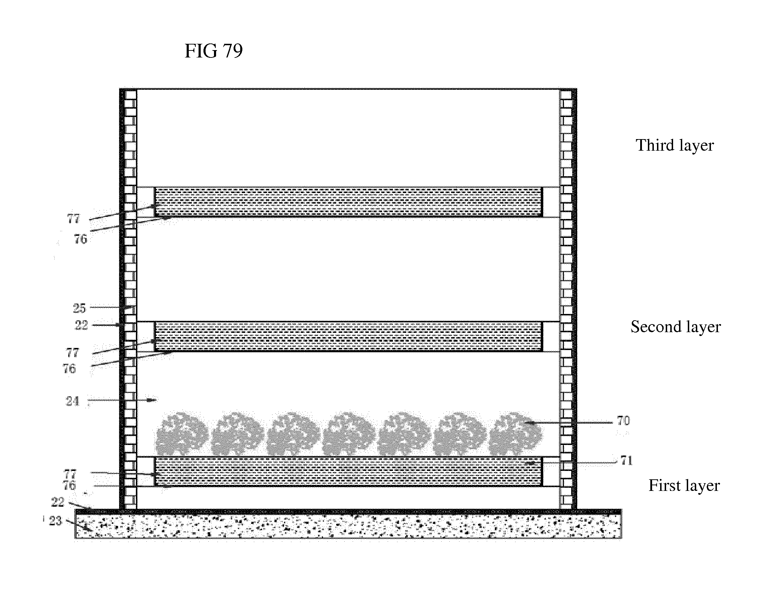

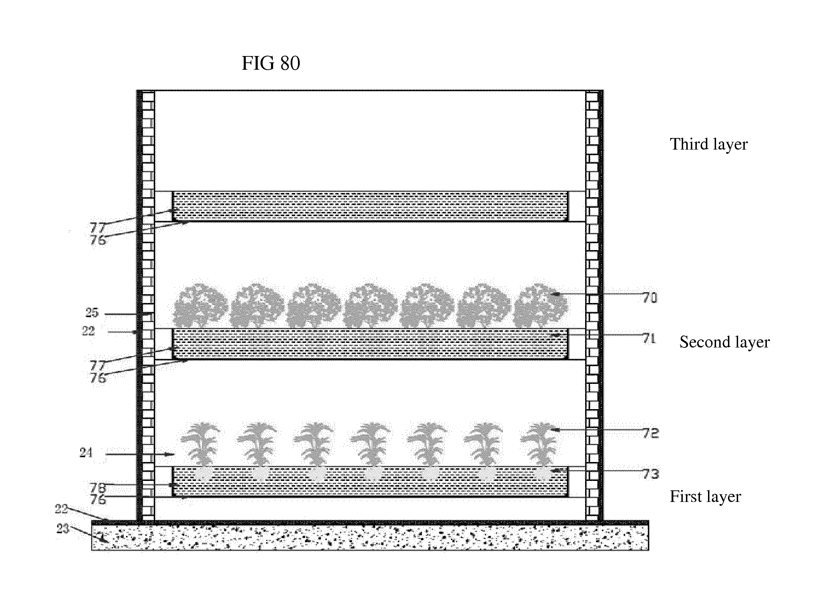

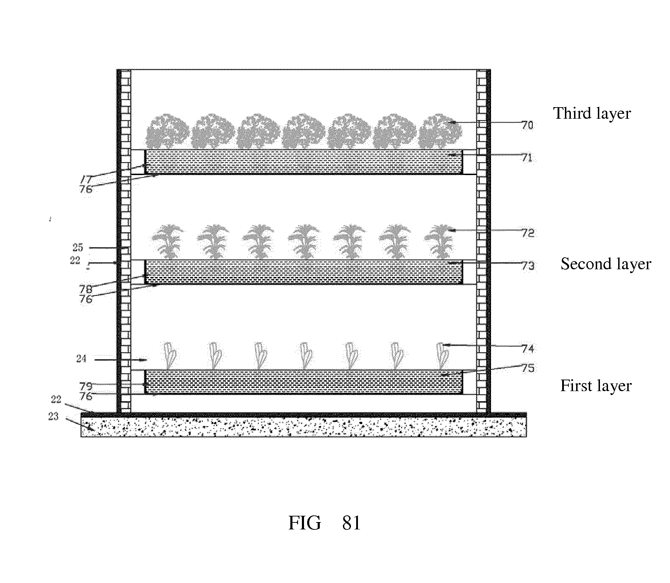

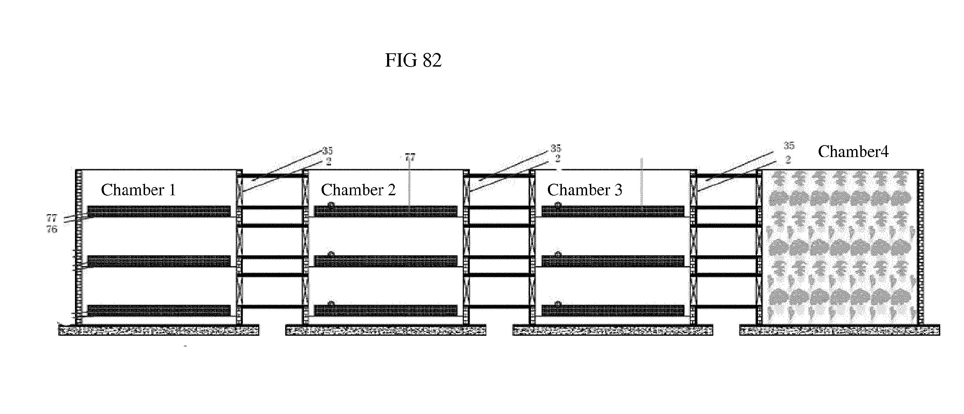

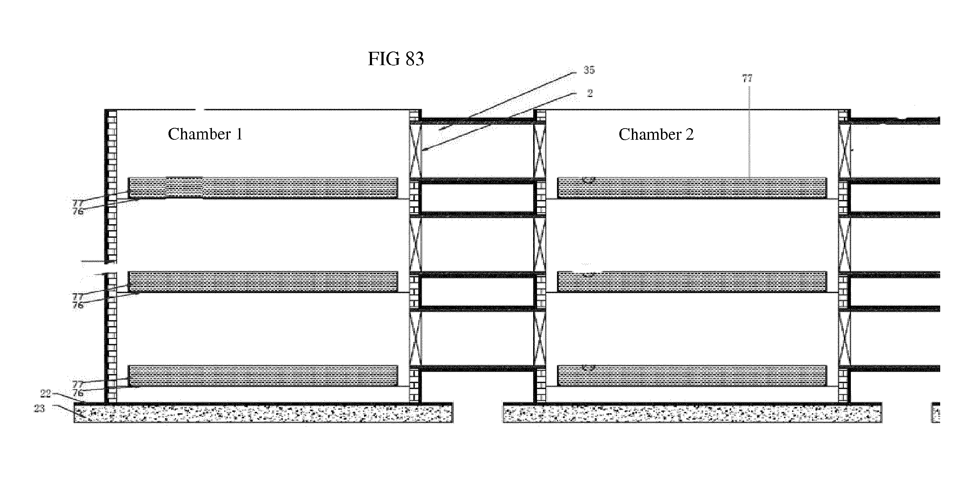

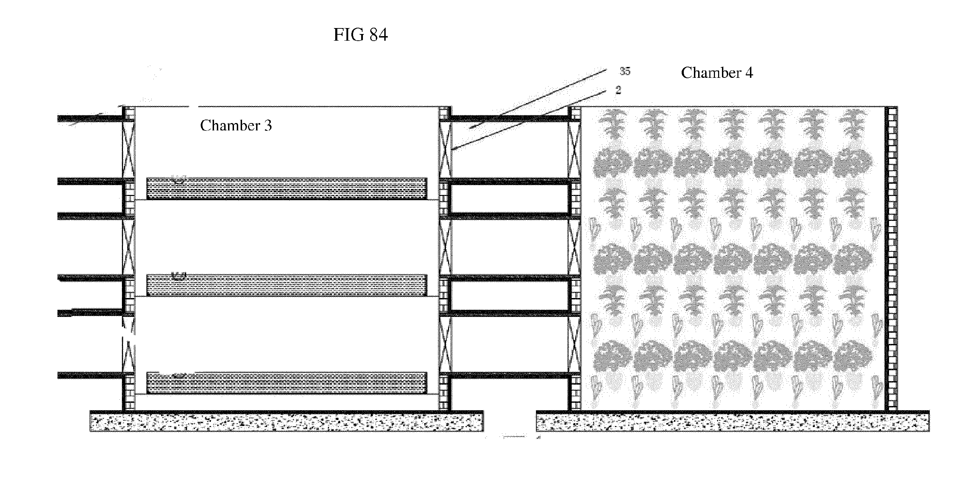

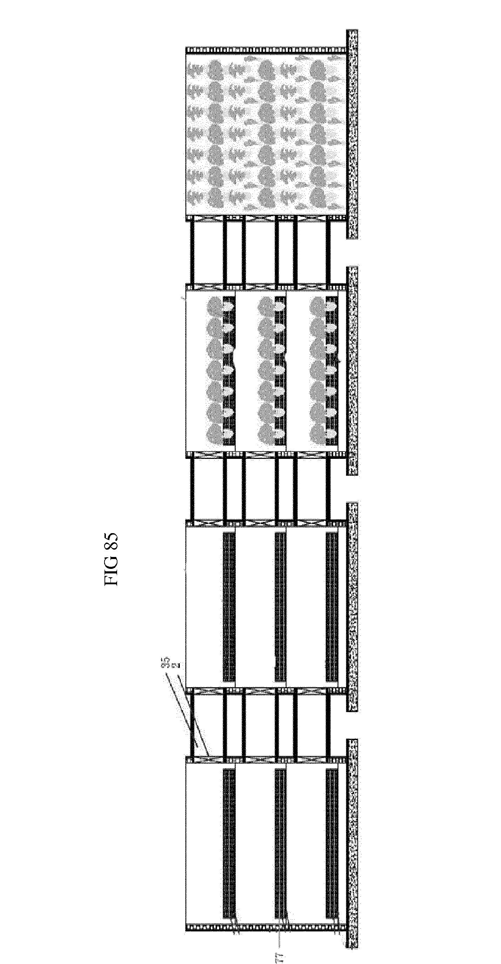

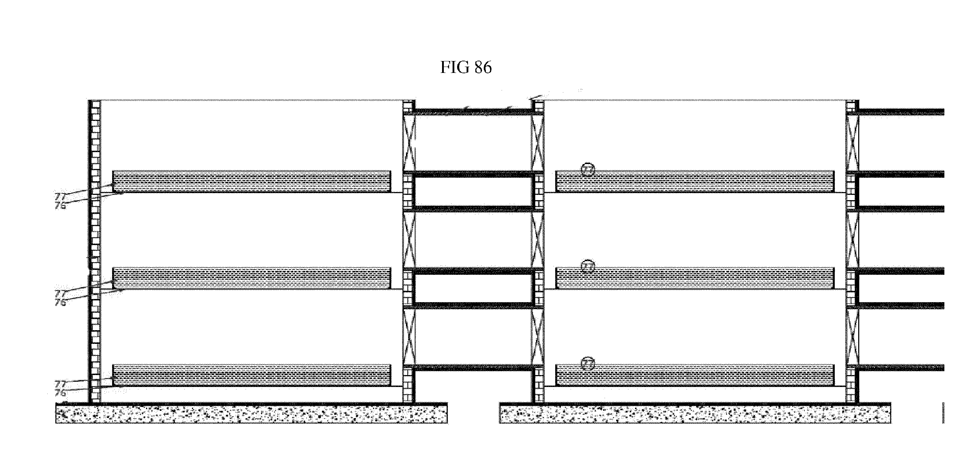

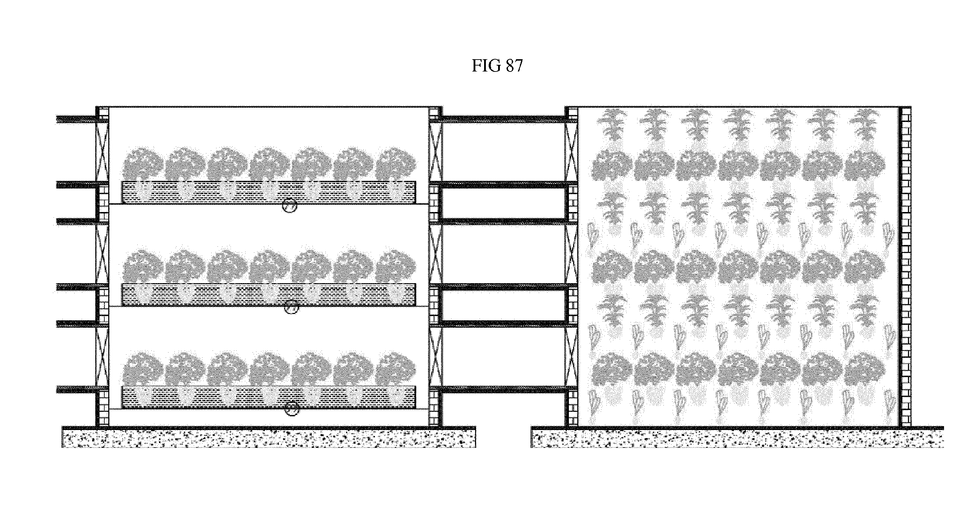

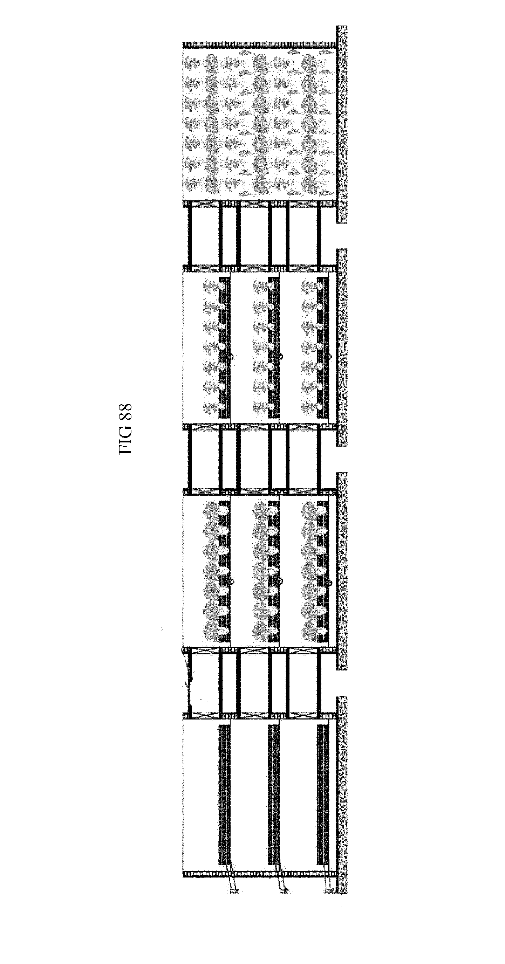

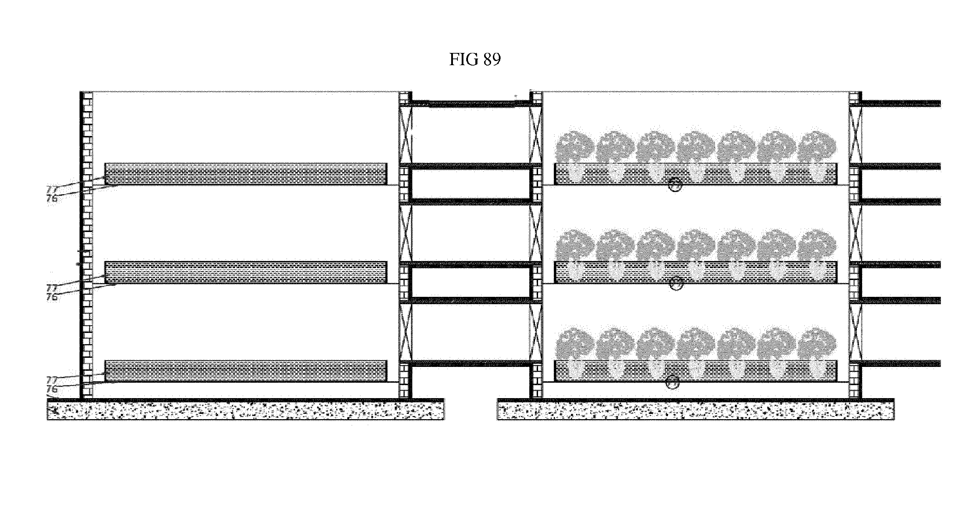

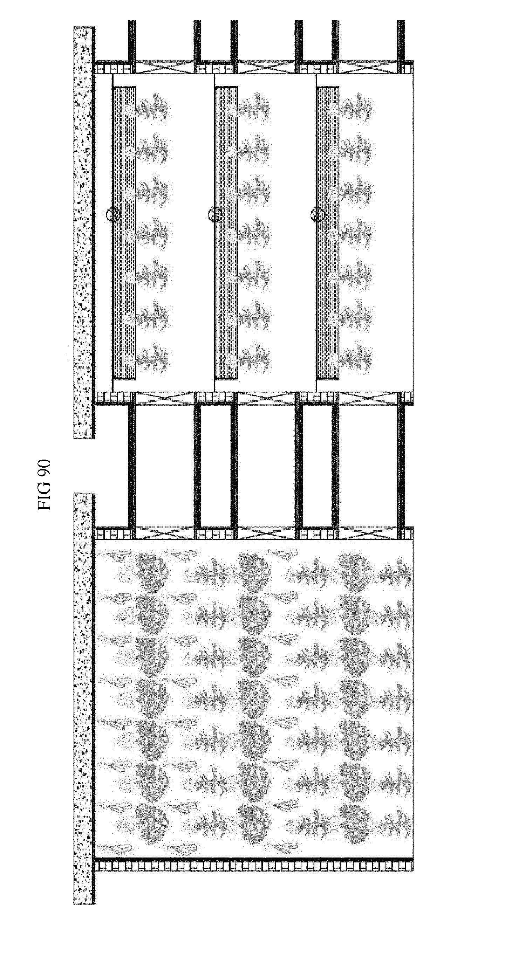

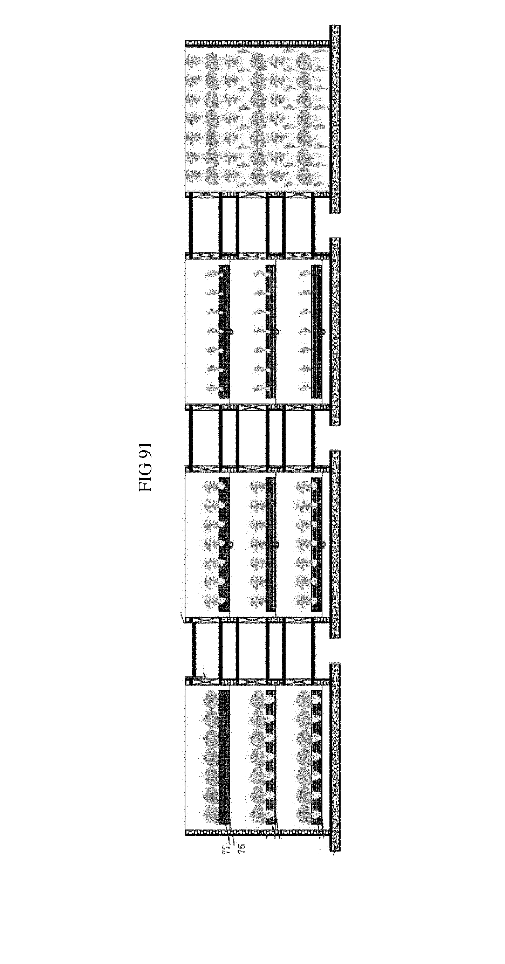

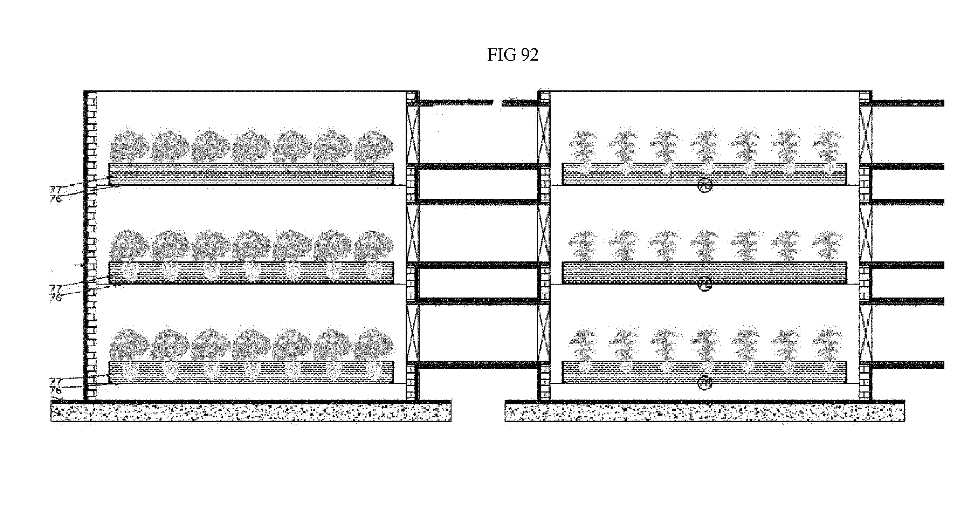

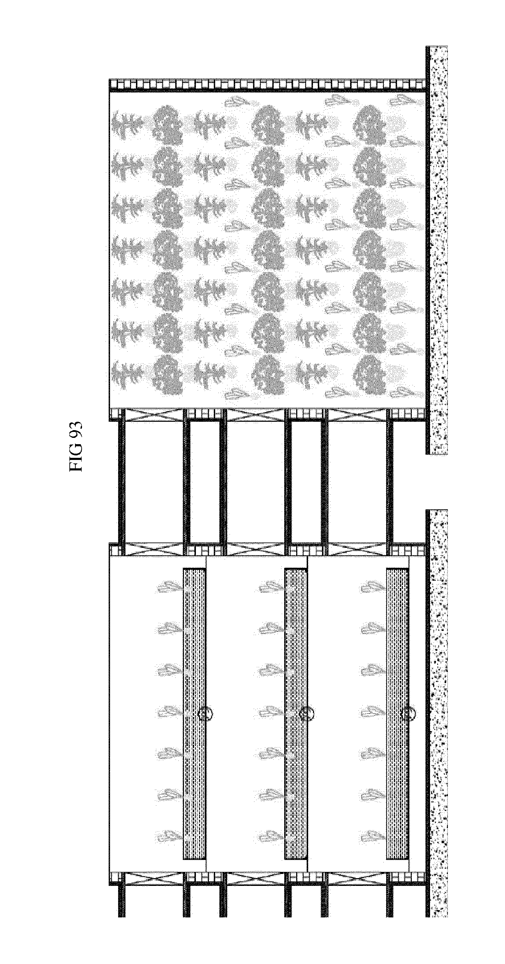

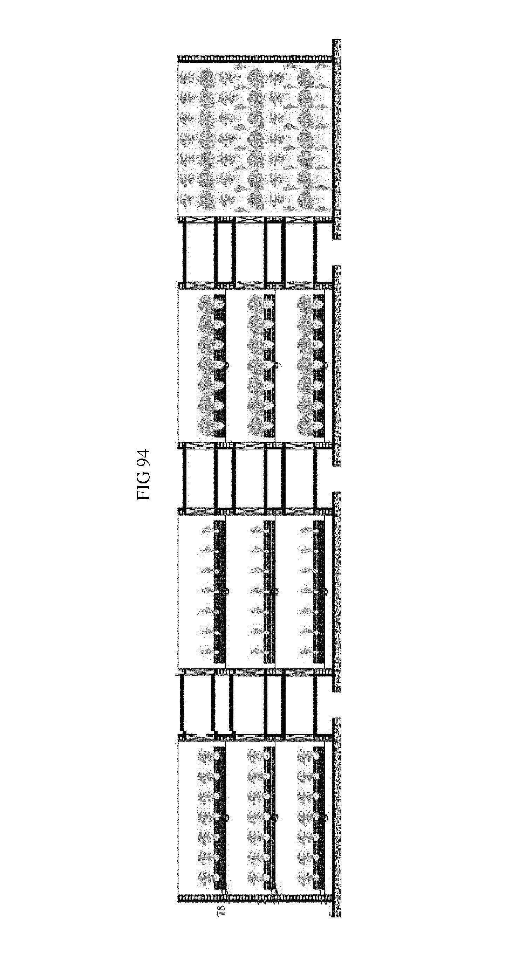

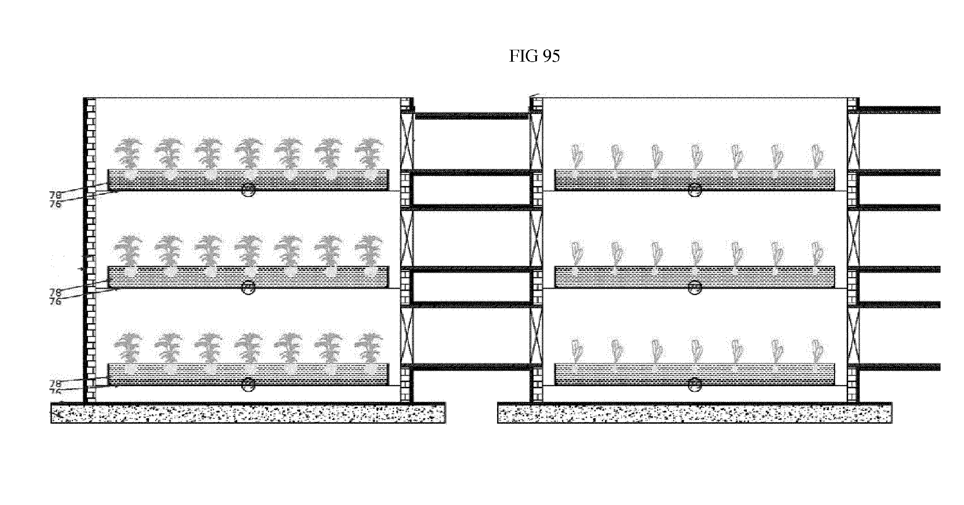

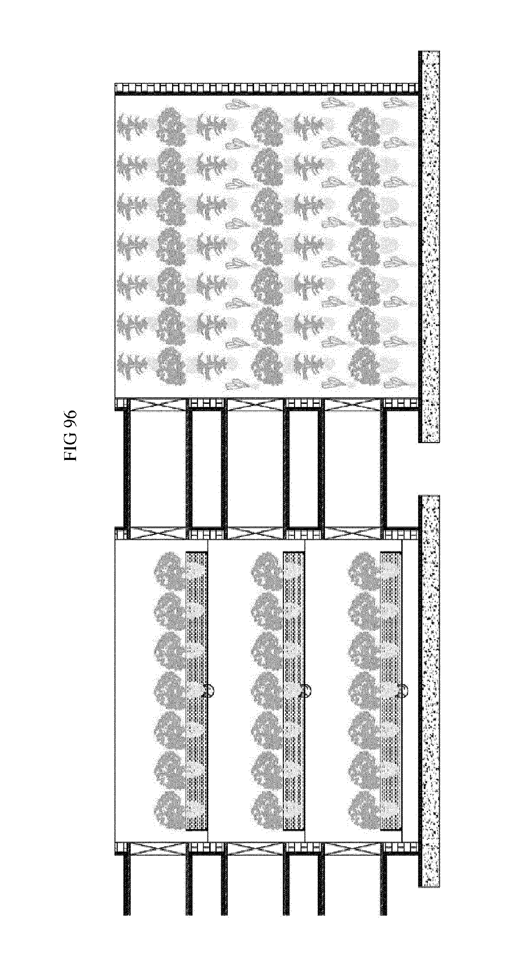

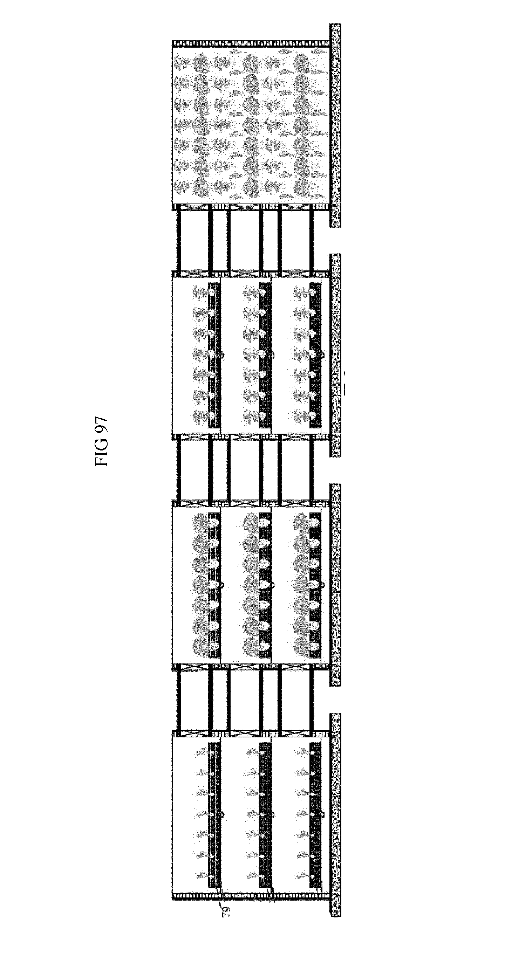

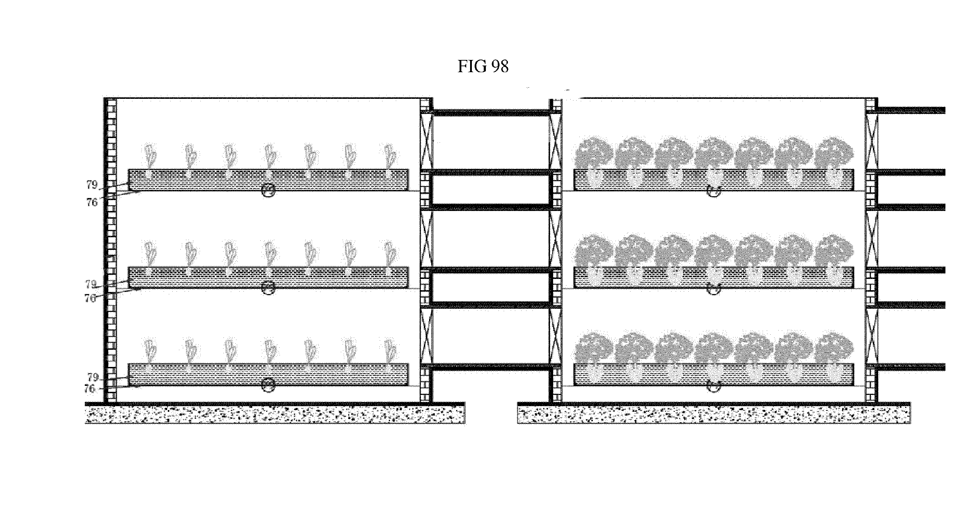

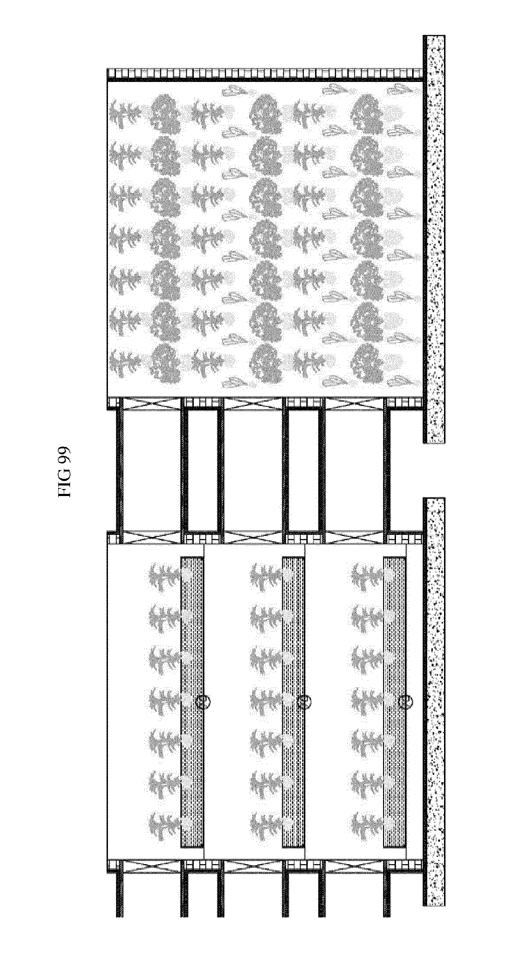

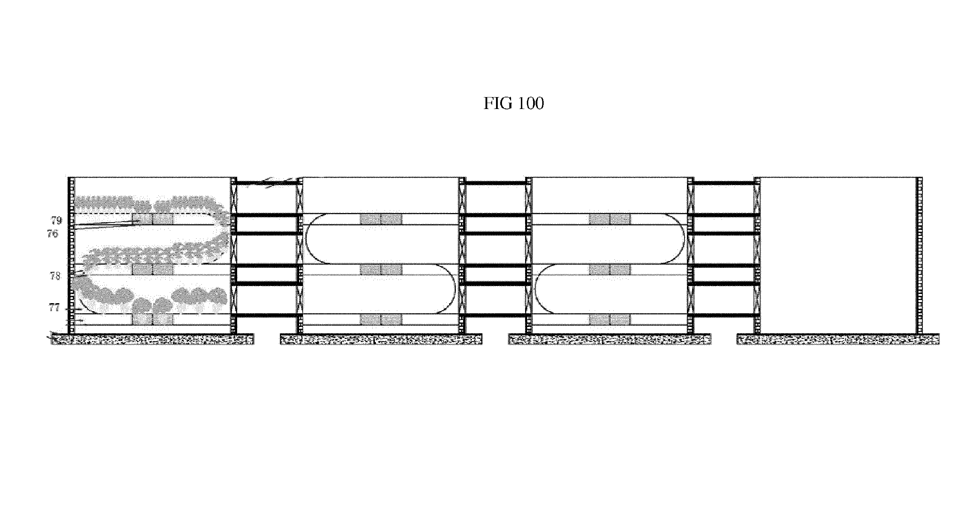

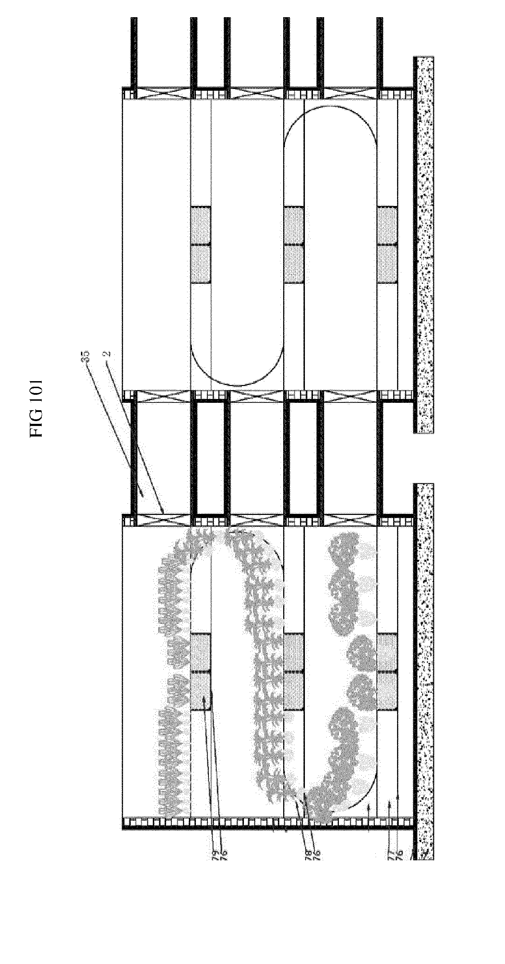

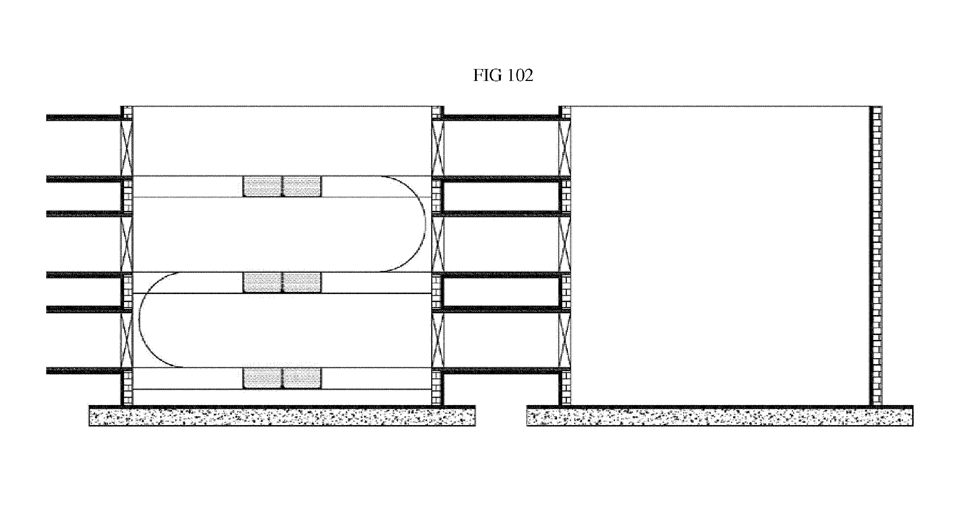

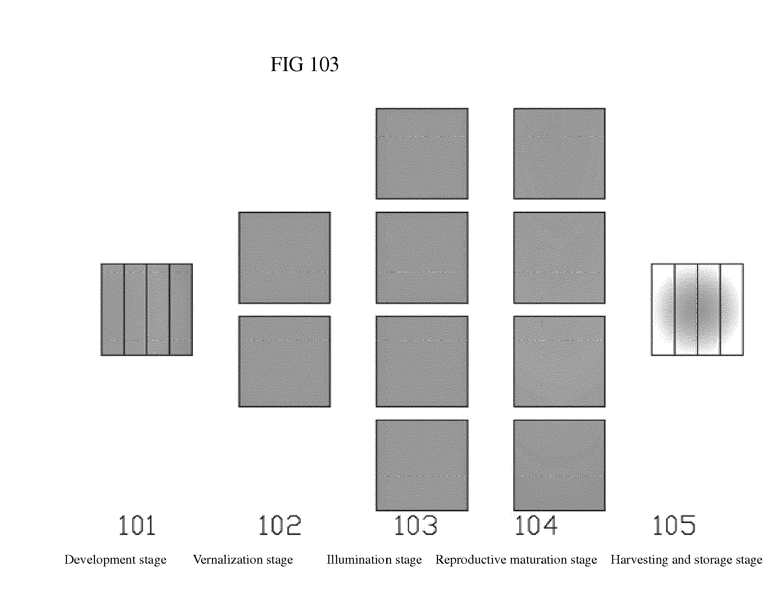

Referring to FIG. 14, the equipment according to this embodiment comprises: warehouse walls 25, a thermal insulating layer 22, a warehouse base layer 23, an in-warehouse environment 24, stems and leaves 70 of a crop group highly resistant to seawater, stems and roots 71 of a crop group highly resistant to seawater, stems and leaves 72 of a crop group moderately resistant to seawater, stems and roots 73 of a crop group moderately resistant to sea water, stems and leaves 74 of a normal freshwater crop group, roots 75 of a normal freshwater crop group, a water tank 76, seawater 77, seawater 78 processed by the crop group highly resistant to seawater (seawater having lower concentration), and seawater 79 processed by the crop group moderately resistant to seawater (similar to the freshwater). In FIG. 14, the suspension magnet, the control system, the conveyer device and the like are omitted. For details about these parts, reference may be made to Embodiment 3.

70 and 71 denote stems, leaves and roots of a crop group highly resistant to seawater, 72 and 73 denote stems, leaves and roots of a crop group moderately resistant to seawater, and 74 and 75 denote stems, leaves and roots of a normal freshwater crop group. Each category of crop may not definitely a single kind of crop, and may be a plurality of kinds of crops within a specific range of concentration resistance. The seawater tank 76 of the device on each layer is filled with seawater, and the environment on each layer is regulated to an optimal state desired by the crop by the environment control system. Then, the effective components of the seawater to be used for crop irrigation are tested, and the category of the crop is determined according to the test result, which prepares for the entry of the crop group highly resistance to seawater into the device. With respect to the crop group highly resistance to seawater, via the conveyer device, before the crop group highly resistant to seawater is placed into seawater, the crop may be in a normal physiological state (which is just transferred from another suitable environment) or may be in an abnormal stressed physiological state (the crop has not taken desired energy and nutritious substances for a period of time, or has been treated in a harsh environment for a period of time). Then, the stems and roots 71 of the crop group highly resistant to seawater are placed into the seawater 77 on the first layer, and treated for a period of time or after the crop group grows through a specific physiological stage. After a portion of solvents or nutritious substance in the seawater are absorbed and fixed by the crop group highly resistant to seawater, the concentration or salt concentration of the original seawater 77 is reduced. In this case, it is no longer suitable for the crop group highly resistant to seawater to grow and develop in the seawater 78 having low concentration. Then, the crop group highly resistant to seawater is exchanged to the seawater 77 on the second layer, and the above operations are continued to exchange the crop group to the third layer. After the crop group highly resistant to seawater is exchanged to the seawater 77 on the second layer, the seawater on the first layer is changed from the original seawater 77 to the seawater 78 having low concentration. In this case, the crop group moderately resistant to seawater is placed into the device via the conveyer device, and the same crop group, before being placed into the seawater 78 having low concentration, may be in a normal physiological state (which is just transferred from another suitable environment), or may be in an abnormal stressed physiological state (the crop has not taken desired energy and nutritious substances for a period of time, or has been treated in a harsh environment for a period of time). Then, the roots 73 of the crop group moderately resistant to seawater are placed into the seawater 78 having low concentration on the first layer, and are treated for a period of time or after the crop group grows through a specific physiological stage. After a portion of solvents or nutritious substance in the seawater 78 having low concentration are absorbed and fixed by the crop group moderately resistant to seawater, the concentration or salt concentration of the seawater 78 having low concentration is further reduced. In this case, it is no longer suitable for the crop group moderately resistant to seawater to grow and develop in the sub-freshwater 79 having even lower concentration and being similar to the freshwater seawater 78 having low concentration. Then, the crop group moderately resistant to seawater is exchanged to the seawater 78 having low concentration on the second layer, and the above operations are continued to exchange the crop group to the third layer. The original seawater 77 on the first layer of the device has changed to sub-freshwater 79 having low salt concentration. In this case, the crop in the normal freshwater needs to be placed into the device via the conveyer device. Like crop group, before being placed into the sub-freshwater 79, may be in a normal physiological state (which is just transferred from another suitable environment), or may be in an abnormal stressed physiological state (the crop has not taken desired energy and nutritious substances for a period of time, or has been treated in a harsh environment for a period of time). Then, the stems and roots 75 of the normal freshwater crop group are placed into the sub-freshwater 79 on the first layer, and are treated for a period of time or after the crop group grows through a specific physiological stage. After a portion of solvents or nutritious substance in the seawater 78 having low concentration are absorbed and fixed by the crop group moderately resistant to seawater, the concentration or salt concentration of the sub-freshwater 79 is further reduced. In this case, it is no longer suitable for the normal freshwater crop group to grow and develop in such an environment. Then, the normal freshwater crop group is exchanged to the sub-freshwater 79 having low concentration on the second layer, and the above operations are continued to exchange the crop group to the third layer. After the sub-freshwater 79 is subjected to the above treatments, the sub-freshwater 79 actually becomes freshwater applicable to various living organisms, which not only achieves the effect of irrigation, but also achieve the effect of purifying the seawater. In addition, during the entire process, a large amount of power energy is not needed, thereby finding a new way for utilizing and purifying seawater.

As illustrated in FIG. 14, after the crop group highly resistant to seawater is exchanged to the seawater 77, the seawater is changed from the original seawater 77 to the seawater 78 having low concentration. In this case, the crop group moderately resistant to seawater is placed into the device via the conveyer device, and the same crop group, before being placed into the seawater 78 having low concentration, may be in a normal physiological state (which is just transferred from another suitable environment), or may be in an abnormal stressed physiological state (the crop has not taken desired energy and nutritious substances for a period of time, or has been treated in a harsh environment for a period of time). Then, the roots 73 of the crop group moderately resistant to seawater are placed into the seawater 78 having low concentration, and are treated for a period of time or after the crop group grows through a specific physiological stage. After a portion of solvents or nutritious substance in the seawater 78 having low concentration are absorbed and fixed by the crop group moderately resistant to seawater, the concentration or salt concentration of the seawater 78 having low concentration is further reduced. In this case, it is no longer suitable for the crop group moderately resistant to seawater to grow and develop in the sub-freshwater 79 having even lower concentration and being similar to the freshwater seawater 78 having low concentration. Then, the crop group moderately resistant to seawater is exchanged to the seawater 78 having low concentration, and the above operations are continued.

The original seawater 77 of the device has changed to sub-freshwater 79 having low salt concentration. In this case, the crop in the normal freshwater needs to be placed into the device via the conveyer device. Like crop group, before being placed into the sub-freshwater 79, may be in a normal physiological state (which is just transferred from another suitable environment), or may be in an abnormal stressed physiological state (the crop has not taken desired energy and nutritious substances for a period of time, or has been treated in a harsh environment for a period of time). Then, the stems and roots 75 of the normal freshwater crop group are placed into the sub-freshwater 79, and are treated for a period of time or after the crop group grows through a specific physiological stage. After a portion of solvents or nutritious substance in the seawater 78 having low concentration are absorbed and fixed by the crop group moderately resistant to seawater, the concentration or salt concentration of the sub-freshwater 79 is further reduced. In this case, it is no longer suitable for the normal freshwater crop group to grow and develop in such an environment. Then, the normal freshwater crop group is exchanged to the sub-freshwater 79 having low concentration, and the above operations are continued to exchange the crop group to the third layer. After the sub-freshwater 79 is subjected to the above treatments, the sub-freshwater 79 actually becomes freshwater applicable to various living organisms, which not only achieves the effect of irrigation, but also achieve the effect of purifying the seawater.

According to this embodiment, with respect to the configuration on the layers, a plurality of chambers may be replaced by a configuration between a plurality of chambers.

### Embodiment 10 Application of the application equipment of suspension in plant cultivation in seawater treatment according to the present invention

### Selection of crops:

Crop highly resistance to seawater: salicornia bigelovii, mangrove
Crop moderately resistant to seawater: demeter fescue

### Normal freshwater crop: medicago sativa linn

The device employed herein is as illustrated in FIG. 9. The seawater tank of the device on each layer is filled with seawater, and the environment on each layer is regulated to an optimal state desired by the crop by the environment control system. Then, the effective components of the seawater to be used for crop irrigation are tested, and the category of the crop is determined according to the test result, which prepares for the entry of the crop group highly resistance to seawater into the device. With respect to the salicornia bigelovii, via the conveyer device, before the salicornia bigelovii is placed into seawater, the salicornia bigelovii may be in a normal physiological state (which is just transferred from another suitable environment) or may be in an abnormal stressed physiological state (the crop has not taken desired energy and nutritious substances for a period of time, or has been treated in a harsh environment for a period of time). Then, the stems and roots of the salicornia bigelovii are placed into the seawater on the first layer, and treated for a period of time or after the crop group grows through a specific physiological stage. After a portion of solvents or nutritious substance in the seawater are absorbed and fixed by the crop group highly resistant to seawater, the concentration or salt concentration of the original seawater is reduced. In this case, it is no longer suitable for the salicornia bigelovii to grow and develop in the seawater having low concentration. Then, the salicornia bigelovii is exchanged to the seawater on the second layer, and the above operations are continued to exchange the salicornia bigelovii to the third layer. After the salicornia bigelovii group is exchanged to the seawater on the second layer, the seawater on the first layer is changed from the original seawater to the seawater having low concentration. In this case, the demeter fescue is placed into the device via the conveyer device, and the same crop group, before being placed into the seawater having low concentration, may be in a normal physiological state (which is just transferred from another suitable environment), or may be in an abnormal stressed physiological state (the crop has not taken desired energy and nutritious substances for a period of time, or has been treated in a harsh environment for a period of time). Then, the roots 73 of the demeter fescue are placed into the seawater 78 having low concentration on the first layer, and are treated for a period of time or after the demeter fescue grows through a specific physiological stage. After a portion of solvents or nutritious substance in the seawater having low concentration are absorbed and fixed by the demeter fescue, the concentration or salt concentration of the seawater having low concentration is further reduced. In this case, it is no longer suitable for the demeter fescue to grow and develop in the sub-freshwater having even lower concentration and being similar to the freshwater seawater having low concentration. Then, the demeter fescue is exchanged to the seawater having low concentration on the second layer, and the above operations are continued to exchange the demeter fescue to the third layer. Through the continuous treatments, the original seawater on the first layer of the device has changed to sub-freshwater having low salt concentration. In this case, the medicago sativa linn needs to be placed into the device via the conveyer device. Like crop group, before being placed into the sub-freshwater, may be in a normal physiological state (which is just transplanted from another suitable environment), or may be in an abnormal stressed physiological state (the crop has not taken desired energy and nutritious substances for a period of time, or has been treated in a harsh environment for a period of time). Then, the stems and roots of the medicago sativa linn are placed into the sub-freshwater on the first layer, and are treated for a period of time or after the medicago sativa linn grows through a specific physiological stage. After a portion of solvents or nutritious substance in the seawater having low concentration are absorbed and fixed by the medicago sativa linn, the concentration or salt concentration of the sub-freshwater is further reduced. In this case, it is no longer suitable for the medicago sativa linn group to grow and develop in such an environment. Then, the medicago sativa linn is exchanged to the sub-freshwater having low concentration on the second layer, and the above operations are continued to exchange the medicago sativa linn to the third layer. After the sub-freshwater is subjected to the above treatments, the sub-freshwater actually becomes freshwater applicable to various living organisms, which not only achieves the effect of irrigation, but also achieve the effect of purifying the seawater. In addition, during the entire process, a large amount of power energy is not needed, thereby finding a new way for utilizing and purifying seawater.

The first layer, the second layer, and the third layer, as devices for various crops to achieve irrigation by turns and to purify seawater therein, may cause the crops to be in a normal physiological state (which is just transplanted from another suitable environment), or to be in an abnormal stressed physiological state (the crop has not taken desired energy and nutritious substances for a period of time, or has been treated in a harsh environment for a period of time).

The seawater tank of the device on each layer is filled with seawater, and the environment on each layer is regulated to an optimal state desired by the crop by the environment control system. Then, the effective components of the seawater to be used for crop irrigation are tested, and the category of the crop is determined according to the test result, which prepares for the entry of the salicornia bigelovii group into the device. With respect to the salicornia bigelovii, before the salicornia bigelovii is placed into seawater, the salicornia bigelovii may be in a normal physiological state (which is just transferred from another suitable environment) or may be in an abnormal stressed physiological state (the crop has not taken desired energy and nutritious substances for a period of time, or has been treated in a harsh environment for a period of time). Then, the stems and roots of the salicornia bigelovii group are placed into the seawater, and treated for a period of time or after the crop group grows through a specific physiological stage. After a portion of solvents or nutritious substance in the seawater are absorbed and fixed by the crop group highly resistant to seawater, the concentration or salt concentration of the original seawater is reduced. In this case, it is no longer suitable for the salicornia bigelovii group to grow and develop in the seawater having low concentration. Then, the salicornia bigelovii group is exchanged to the seawater, and the above operations are continued to exchange the salicornia bigelovii group to the third layer.

After the salicornia bigelovii group is exchanged to the seawater, the seawater is changed from the original seawater to the seawater having low concentration. In this case, the demeter fescue is placed into the device via the conveyer device, and the same crop group, before being placed into the seawater having low concentration, may be in a normal physiological state (which is just transferred from another suitable environment), or may be in an abnormal stressed physiological state (the crop has not taken desired energy and nutritious substances for a period of time, or has been treated in a harsh environment for a period of time). Then, the roots 73 of the demeter fescue group are placed into the seawater 78 having low concentration on the first layer, and are treated for a period of time or after the demeter fescue group grows through a specific physiological stage. After a portion of solvents or nutritious substance in the seawater having low concentration are absorbed and fixed by the demeter fescue group, the concentration or salt concentration of the seawater having low concentration is further reduced. In this case, it is no longer suitable for the demeter fescue group to grow and develop in the sub-freshwater having even lower concentration and being similar to the freshwater seawater having low concentration. Then, the demeter fescue group is exchanged to the seawater having low concentration on the second layer, and the above operations are continued to exchange the demeter fescue group to the third layer.

Through the continuous treatments, the original seawater of the device has changed to sub-freshwater having low salt concentration. In this case, the medicago sativa linn needs to be placed into the device via the conveyer device. Like crop group, before being placed into the sub-freshwater, may be in a normal in a normal physiological state (which is just transplanted from another suitable environment), or may be in an abnormal stressed physiological state (the crop has not taken desired energy and nutritious substances for a period of time, or has been treated in a harsh environment for a period of time). Then, the stems and roots of the medicago sativa linn are placed into the sub-freshwater, and are treated for a period of time or after the medicago sativa linn grows through a specific physiological stage. After a portion of solvents or nutritious substance in the seawater having low concentration are absorbed and fixed by the medicago sativa linn, the concentration or salt concentration of the sub-freshwater is further reduced. In this case, it is no longer suitable for the medicago sativa linn group to grow and develop in such an environment. Then, the medicago sativa linn is exchanged to the sub-freshwater having low concentration, and the above operations are continued to exchange the medicago sativa linn to the third layer. After the sub-freshwater is subjected to the above treatments, the sub-freshwater actually becomes freshwater applicable to various living organisms, which not only achieves the effect of irrigation, but also achieve the effect of purifying the seawater.

After the treatments, the original seawater is purified to freshwater applicable to the living organisms, and then the freshwater is pumped out from the seawater tank for other usages. Subsequently, the seawater is injected to the seawater tank for a next circle of seawater irrigation and seawater purification.

## Claims

1. Application of a drift and suspension device, comprising:
A: application in human bodies or animals, providing a "hypomagnetic state" environment for the human bodies or animals;
B: application in plants, providing a "hypomagnetic state" environment for the plants and converting a passive and relatively stationary growth of the plants into an initiative growth and development pattern; and
C: application in irrigation and/or purification of seawater or sewage water.

2. The application of the drift and suspension device according to claim 1, wherein the A: application in human bodies or animals comprises the following steps:
1) preparing for a magnetic suspension treatment of the human bodies or animals;
2) formulating an operational procedure of the corresponding magnetic suspension treatment according to vital signs of the human bodies or animals;
3) adjusting the drift and suspension device according to the operational procedure of the magnetic suspension treatment formulated in step 2) to achieve a "hypomagnetic state" environment most suitable for the human bodies or animals;
4) placing the human bodies or animals into the drift and suspension device to conduct the magnetic suspension treatment; and
5) monitoring various life and physiological data related to the human bodies or animals in a magnetic suspension device by using a dynamic information monitoring device, with the data transferred to a data processing center through a dynamic information transmission line, and further establishing the operational procedure of the magnetic suspension treatment upon analysis and research of the data to provide a "hypomagnetic state" environment more suitable for the human bodies or animals by a reality and operation system;
wherein the suspension refers to suspension of the human bodies or animals in the device, the suspension makes the human bodies or animals suspend in the device under the action of magnetic force or some other antigravities.

3. The application of the drift and suspension device according to claim 1, wherein the B: application to plants comprises the following steps:
1) selecting plants required;
2) formulating an operational procedure most suitable to growth and development of the plants and other lower living organisms according to different demands of the plants for substances and energy information in different periods and at different growth and development stages;
3) placing the plants into the device and running in accordance with the procedure formulated so that the plants initiatively absorbs and acquires desired substance, energy and information at the most suitable time and growth and development stage, unlike the traditional planting industry in which passive photosynthesis, metabolism, normal growth and development are unavailable unless plants are exposed to sunlight, proper temperature, and rainwater or artificial irrigation; and
4) the plants and other lower living organisms being drifted in the device in accordance with the procedure, growing and developing in the device under the state of drift in accordance with the procedure, and at the same time with the drift treatment, being dynamically monitored, with monitored information transmitted to a computer control center for analysis and research to formulate a "hypomagnetic state" environment more suitable for growth and development of the plants and other lower living organisms, thus controlling the whole device and actively acquiring the desired various substance, energy and information by means of demand information indicated by the plants and other lower living organisms;
wherein the drift refers to a situation that the plants and other lower living organisms are fixed to a supporting device, running in the manner of drift in accordance with a preset procedure under the action of the supporting device in the drift and suspension device; such a running manner of drift refers to a situation that the plants and other lower living organisms actively control the whole device in the running manner of drift in the drift and suspension device to actively acquire the desired various substance, energy and information, namely, from one layer of a chamber to another layer, and from one chamber to another chamber; at the same time of drift, a magnetic field with a desired strength and type is provided for the plants as well as different parts and organs of the plants according to demands of the plants for the magnetic field.

4. The application of the drift and suspension device according to claim 3, wherein the plants initiatively absorb and acquire the desired substance, energy and information at a most suitable time and growth and development stage; since plants and other lower living organisms have different demands for substance, energy and information at different growth and development stages, dynamic information data acquired during a running process of the plants in the device by means of the innovative device of the present invention based on base data related to existing scientific achievements in plants, the base data being substance, energy and information needed for growth and development of the plants, is average data on substance, energy and information demanded at a certain growth and development period, and the dynamic information data is instant data on the plants and other lower living organisms at a certain moment, both data is converted by the innovative device of the present invention into instructions of substance, energy and information desired by the plants and other lower living organisms at different growth and development stages, and becomes data information readable by human beings and computers, for example, data on substance, energy and information desired by plants at a certain growth and development stage is: temperature of a°C, humidity of b%, illumination of c lux, moisture of d grams, nitrogen phosphorus and potassium or the like of e grams, an organic fertilizer of f grams, light reaction of photosynthesis of g hours, dark reactions of photosynthesis of h hours, magnetic field intensity of i Tesla, CO2 concentration of j%, oxygen component k and the like; such data is substance, energy and information which the plants desire to absorb and acquire at this stage; and such data is sent to a control system of the device in real time for analysis and research, on the basis of which the control system formulates an operation scheme for the plants in the device, generates instructions for commanding various control systems, functional chambers and functional zones of the device, and provides substance and energy desired by a certain plant at this stage; and then the plant is placed, by means of a fixing system of the plant, into various functional chambers and functional zones in real time according to the operation scheme for absorbing and acquiring the desired energy and substance information, thereby forming a set of central systems of the whole device on the basis of demand instructions spontaneously sent by plants and other lower living organisms, control systems of respective parts, with dynamic information analysis and monitoring systems of respective parts of the device, lines between various systems and the computer control center for exchanging data, control and information, and the computer control center serving as accessory structures of the central system for commanding the whole device to serve for growth and development and reproduction of plants and other lower living organisms; in this way, an intelligent living body like higher animals is formed by combining plants and other lower living organisms with the device as a whole, which spontaneously acquires the substance, energy and information, spontaneously creates an ambient environment suitable for itself just like the human beings, and this combination converts the plants and other lower living organisms from relatively static growth, development and reproduction, and passive absorption and acquisition of substance, energy and information into dynamic and active living organisms.

5. The application of the drift and suspension device according to claim 5, wherein the plants achieves the whole process of growth and development and reproduction continuously in the device.

6. The application of the drift and suspension device according to claim 3, wherein the plants achieves a "pipelined" production continuously in the device.

7. The application of the drift and suspension device according to claim 1, wherein the C: application to irrigation and/or purification of seawater or sewage water comprises the following steps:
1) selecting plants and other lower living organisms (hereinafter referred to as plants) on the basis of detection of different seawater concentrations and effective constituents, for example, plants resistant to different seawater concentrations being selected in case of different seawater concentrations; and determining the type of crop according to the kind of sewage water, detection results of effective constituents and monitor results;
2) formulating an operational procedure most suitable for growth and development of plants selected according to different demands of plants for substance, energy and information as well as different degrees of tolerance to seawater concentration, and to different absorption and fixation of effective constituents and other factors in different periods and at different growth and development stages;
3) placing the plants into the device and running in accordance with the procedure formulated so that effective constituents in seawater or sewage water being absorbed and fixed by the plants under the action of growth and development and physiological activities of the plants, and seawater concentration is accordingly reduced, thus finally reaching the effect of desalination, purification and irrigation; and
4) plants being drifted in the device in accordance with the procedure for growth and development under the state of drift, effective constituents in seawater or sewage water being absorbed and fixed by the plants at the same time of drift treatment so that the concentration of effective constituents in seawater or sewage water are accordingly lowered or reduced, thus finally reaching the effect of desalination, purification and irrigation; simultaneously the plants being dynamically detected, with monitored information transmitted to the computer control center for analysis and research so as to formulate a hypomagnetic state environment more suitable for growth and development of the plants, thus exchanging the plants among different chambers in order to be more suitable for growth and development and physiological activities of the plants, and reaching the effect of desalination, purification and irrigation of seawater or sewage water;
wherein the drift refers to a situation that the plants and other lower living organisms are fixed to a supporting device and running in the drift and suspension device under the action of the supporting device in the manner of drift in accordance with a preset procedure; such a running manner of drift refers to a situation that the plants and other lower living organisms actively control the whole device to actively acquire the desired various substance, energy and information in the running manner of drift in the drift and suspension device, namely, from one layer of a chamber to another layer, and from one chamber to another chamber; at the same time of drift, a magnetic field with a desired strength and type is provided for the plants as well as different parts and organs of the plants according to demands of the plants for the magnetic field;
the drift and suspension device comprises: a controlled chamber wall, a controlled chamber wall insulation layer, a controlled chamber foundation, controlled environment, a gangway between a chamber and another chamber, a gangway door, leaves and stems of a crop group highly resistant to seawater, stems and roots of a crop group highly resistant to seawater, leaves and stems of a crop group moderately resistant to seawater, stems and roots of a crop group moderately resistant to seawater, leaves and stems of a normal freshwater crop group, stems and roots of a normal freshwater crop group, a seawater tank, seawater, seawater processed by the crop group highly resistant to seawater, and seawater processed by the crop group moderately resistant to seawater; wherein the gangway between a chamber and another chamber is a gangway for crop to enter from one chamber to another chamber, after a certain stage of growth and development and reproduction or a certain stage of physiological process of a crop is completed in one chamber, the crop is transplanted into another chamber for a next stage of growth and development and reproduction or another stage of physiological process; the gangway door is arranged on the gangway between a chamber and another chamber, the gangway door is opened first when the crop enters from one chamber into another chamber and the gangway door is closed after the crop enters from one chamber into another chamber, the gangway door maintains relatively independent environment between one chamber and another chamber, which contributes to completing growth and development of plants at different stages; in the leaves and stems and roots of the crop group highly resistant to seawater, leaves and stems and roots of the crop group moderately resistant to seawater, as well as leaves and stems and roots of the normal freshwater crop group, a crop group of each category is not a single crop but multiple plants within a certain range of tolerance concentrations, the seawater tank on each layer of the device is filled with seawater, the environment at each layer is adjusted by an environment control system into a state most suitable for the crop, then effective constituents of seawater used for irrigation of the crop are detected so as to determine the category of the crop according to detection results and get ready for entering of the crop group highly resistant to seawater which enters into the device through the gangway between a chamber and another chamber and the gangway door, the crop is either in a normal physiological state or be in an abnormal stressed physiological state before the crop group highly resistant to seawater is placed into seawater, then stems and roots of the crop group highly resistant to seawater are placed in seawater on a first layer; after treatment of a certain time or growth of a certain physiological stage, the pristine seawater concentration or salt concentration is reduced after a portion of solutes or nutrient substances in seawater is absorbed and fixed by the crop group highly resistant to seawater, now the seawater with lower concentration is unsuitable for normal growth and development of the crop group highly resistant to seawater, afterwards, the crop group highly resistant to seawater is placed in seawater on a second layer to continue the process and then placed in seawater on a third layer after the above process; seawater on the first layer is converted from the pristine seawater into seawater with lower concentration after the crop group highly resistant to seawater is placed in seawater on the second layer; now the crop group moderately resistant to seawater enters into the device through the gangway between a chamber and another chamber and the gangway door; similarly, the crop is either in a normal physiological state or in an abnormal stressed physiological state before the crop group moderately resistant to seawater is placed into seawater with lower concentration, then roots of the crop group moderately resistant to seawater are placed in seawater with lower concentration on the first layer; after treatment of a certain time or growth of a certain physiological stage, the concentration of the seawater with lower concentration or salt concentration is further reduced after a portion of solutes or nutrient substances in the seawater with lower concentration is absorbed and fixed by the crop group moderately resistant to seawater; now the seawater with even lower concentration, similar to freshwater, is unsuitable for normal growth and development of the crop group moderately resistant to seawater; afterwards, the crop group moderately resistant to seawater is placed in the seawater with lower concentration on the second layer to continue the process and then placed in seawater on the third layer.

8. The application of the drift and suspension device according to any one of claims 1 to 7, wherein the magnetic suspension treatment comprises a hypomagnetic suspension treatment and a magnetic suspension treatment, differentiated according to magnetic field intensity provided by the drift and suspension device for human bodies or animals or the like; the human bodies or animals will be completely suspended by the device when the magnetic field provided by the drift and suspension device reaches a certain intensity Bf, this treatment as well as various restorations, physical therapies and other therapies or the like based on such a magnetic field intensity pertain to magnetic suspension treatment; when the magnetic field intensity provided by the device is smaller than Bf, various restorations, physical therapies and other therapies or the like based on such a magnetic field intensity pertain to hypomagnetic suspension treatment; either an integrated treatment or a partial treatment is conducted on the human bodies or animals by a hypomagnetic suspension treatment or a magnetic suspension treatment.

9. The drift and suspension device applied to the human body or animal according to claim 2, wherein the drift and suspension device comprises: a suspension warehouse, a suspension warehouse wall, a suspension device shell, a suspension warehouse gate, an upper suspension magnet, a lower suspension magnet, a cushion pad, a preparation couch in for entry to warehouse, a device for supporting and fixing the preparation couch for entry to warehouse, a device for supporting and fixing the suspension warehouse, human for suspension regulation, a dynamic information monitoring device of various systems for human bodies, a dynamic information transmission line of various systems for human bodies, a data processing center, a reality and operation system, an upper suspension magnet energy supply, a lower suspension magnet energy supply, a system for monitoring and feedback information on suspension warehouse inside, an upper suspension magnet control system, a lower suspension magnet control system, an information transmission and feedback line, a human body energy regulation device and a human body nutrition regulation device; wherein
the cushion pad, the preparation couch for entry to warehouse and the device for supporting and fixing the preparation couch for entry to warehouse constitute a platform in preparation for a person subject to suspension regulation of the innovative device of the present invention; the cushion pad is placed on the preparation couch for entry to warehouse, after the person subject to suspension regulation gets ready, under the control of the reality and operation system, the person subject to suspension regulation is sent by the cushion pad to the suspension warehouse, the preparation couch for entry to warehouse is fixed by the device for supporting and fixing the preparation couch for entry to warehouse, one end of the preparation couch for entry to warehouse is connected to the suspension warehouse for bearing the weight of the person subject to suspension regulation and preparing for warehouse entry, the device for supporting and fixing the preparation couch for entry to warehouse is configured to fix and support the preparation couch for entry to warehouse, both the height and the length of the preparation couch for entry to warehouse are adjusted by the device for supporting and fixing the preparation couch for entry to warehouse as needed, and the bottom of the device for supporting and fixing the preparation couch for entry to warehouse is provided with wheels;
the suspension warehouse, the suspension warehouse wall, the suspension device shell, the suspension warehouse gate, the upper suspension magnet, the lower suspension magnet and the device for supporting and fixing the suspension warehouse constitute a suspension system in the innovative device of the present invention; the shape of the suspension warehouse is a circle, an oval, a rectangle or the like on the basis of human body structure and equipment installed; the suspension warehouse is configured to suspend the person subject to suspension regulation, with some other equipment installed, for example, the dynamic information monitoring device of various systems for human bodies or some other monitoring devices; the suspension warehouse wall is made from diamagnetic materials or non-magnetic materials, such as plastics, rubber, copper or the like; the upper part and the lower part of the suspension warehouse are provided with the upper suspension magnet and the lower suspension magnet respectively, both the upper suspension magnet and the lower suspension magnet are mounted between the suspension warehouse wall and the suspension device shell and generate a magnetic field in the suspension warehouse as needed, with magnetic induction intensity of the magnetic field regulated under the control of the reality and operation system; a superconducting magnet or a superconducting coil is selected and used as the upper suspension magnet and the lower suspension magnet both of which are composed of an electromagnet or a permanent magnet; according to different demands of human body for assistant regulation and restoration and dredging, under the control of the reality and operation system, a weak magnetic field, a strong magnetic field, an ultrastrong magnetic field, a uniform magnetic field, a gradient magnetic field, an alternating magnetic field and a pulsed magnet field or the like is generated in the suspension warehouse; the suspension warehouse gate is arranged at a joint connecting the preparation couch for entry to warehouse and the suspension warehouse, and the suspension warehouse gate is automatically opened under the control of the reality and operation system before the person subject to suspension regulation enters into the suspension warehouse, and is automatically closed under the control of the reality and operation system after the person subject to suspension regulation enters into the suspension warehouse; a sealed end of the suspension warehouse is opposite to one end of the suspension warehouse gate, it is not allowed to open the sealed end unless otherwise specified, for example, in case of maintenance or malfunction of the suspension warehouse gate; or both ends of the suspension warehouse gate are respectively provided with a suspension warehouse gate; the suspension device shell is fixed to the device for supporting and fixing the suspension warehouse, and various components and devices in the suspension system are directly or indirectly fixed to the suspension device shell;
the dynamic information monitoring device of various systems for human bodies, the dynamic information transmission line of various systems for human bodies, the data processing center and the reality and operation system constitute an information monitoring and control operation system in the innovative device of the present invention; the dynamic information monitoring device of various systems for human bodies is mounted in the suspension warehouse, and is connected to the person subject to suspension regulation after the person subject to suspension regulation is sent to the suspension warehouse; data related to human body life activities and various environmental data are monitored, and these data are transferred to the data processing center through the dynamic information transmission line of various systems for human bodies for analysis and treatment to make a corresponding judgment, afterwards, the reality and operation system sends out instructions to adjust and control the magnetic induction intensity of the suspension warehouse and contents of various substances in the environment and to communicate with the person subject to suspension regulation, thus finally unblocking internal magnetic state biomagnetic field pathway of an overall organism, strengthening the energy of the internal magnetic state of biomagnetic field pathway of the overall organism, and rejuvenating the vital activity of the organism;
under the control of the upper suspension magnet control system, the upper suspension magnet energy supply provides adjustable current for the upper suspension magnet and is connected to an outside power source; the upper suspension magnet control system is connected to the reality and operation system which issues a control instruction transmitted to the upper suspension magnet control system; under the control of the lower suspension magnet control system, the lower suspension magnet energy supply provides adjustable current for the lower suspension magnet and is connected to an outside power source; the lower suspension magnet control system is connected to the reality and operation system which issues a control instruction transmitted to the lower suspension magnet control system; both sides of the suspension warehouse are mounted with the upper suspension magnet energy supply, the lower suspension magnet energy supply, the system for monitoring and feedback information on suspension warehouse inside, the upper suspension magnet control system, the lower suspension magnet control system, the human body energy regulation device and the human body nutrition regulation device; the human energy regulation device is mounted on one side of the suspension warehouse and is controlled by the reality and operation system; the human body energy regulation device provides energy necessary for the person subject to suspension regulation in the suspension warehouse according to physiological needs of the person subject to suspension regulation for energy; first the human body energy regulation device detects the human body energy state, and then sends data to the data processing center to make a judgment through analysis and arrangement, and the reality and operation system issues an instruction to control the human body energy regulation device to provide a moderate amount of energy for human bodies, thus assisting organism in recuperation of internal magnetic state biomagnetic field; the human body nutrition regulation device is mounted on one side of the suspension warehouse and is controlled by the reality and operation system; supply of nutrient substances is based on physiological and pathological demands of the person subject to suspension regulation; first the human body nutrition regulation device detects the human body energy state, and then sends data to the data processing center to make a judgment through analysis and arrangement, and the reality and operation system issues an instruction to control the human body nutrition regulation device to provide an appropriate amount of nutrient substances for human bodies, thereby assisting organism in recuperation of internal magnetic state biomagnetic field; and
the drift and suspension device comprises a device of at least one set of suspension system; the position among all sets of suspension systems is an up-down structure, or front-back or left-right structures; the device exchanges human body or animals among various suspension systems for staged magnetic suspension treatment.

10. The drift and suspension device applied to plants according to claim 3, wherein the device comprises: a controlled chamber wall insulation layer, a controlled chamber foundation, a controlled environment, a nutrition trough, a gangway between a chamber and another chamber, a gangway door, a nutrient solution, a turbid solution, roots of a crop, leaves and stems of the crop, a crop fixing disk, a power system of the crop fixing disk, a control system of the crop fixing disk, a main bracket for fixing the crop, an auxiliary bracket for fixing the crop, a transverse bracket for fixing the crop, a transverse bracket for fixing the roots of the crop, a breeding matrix trough, breeding matrix, seeds, a permeable breeding tray, a nutrient solution feed pipeline, a nutrient solution recovery pipeline, a monitor probe for dynamic information of the nutrient solution, a transmission line for dynamic information of the nutrient solution, a temperature control system, a humidity control system, a CO2 concentration control system, an oxygen component control system, an illumination intensity uniformity control system, a microorganism control system, a magnetic field gradient control system, transmission control lines for various control systems, a temperature monitoring system, a humidity monitoring system, a CO2 concentration monitoring system, an oxygen component monitoring system, an illumination intensity uniformity monitoring system, a microorganism monitoring system, a magnetic field gradient monitoring system, an air quality monitoring system, transmission lines for various monitoring systems, a system for dynamically monitoring and analyzing items such as temperature, humidity, CO2, oxygen, illumination, air quality, microorganism and magnetic field gradient or the like, a central control system, a nutrient solution analysis and preparation system, a nutrient solution feed system, a system for dynamically monitoring and analyzing information on nutrient solution, a nutrient solution recovery system, a system for analyzing constituents of the nutrient solution recycled and extracting effective constituents, lines for exchanging data, control and information between various systems and a computer control center, and the computer control center;
the controlled chamber wall, the controlled chamber wall insulation layer and the controlled chamber foundation constitute a chamber which provides controlled environment for the innovative device of the present invention, in another word, a controlled environment chamber; the controlled chamber wall is a framework and a supporting part of the controlled environment chamber, serving as an installation foundation of thermal insulating material and other various equipment and facilities, and the controlled chamber wall is a brick structure, a steel structure, or structures of other materials; it is determined the length, width and height of the controlled chamber wall and material selected on the basis of planting needs; the controlled chamber wall insulation layer is mounted on the controlled chamber wall by means of exterior wall installation, interior wall installation or inwall installation, guaranteeing stable temperature inside the controlled environment chamber; materials with the minimum thermal conductivity are selected for the controlled chamber wall insulation layer which is installed in the controlled chamber foundation or a terrace besides on the controlled chamber wall so as to reduce the exchange of inside and outside heats and maintain a stable interior temperature as much as possible; the controlled chamber foundation serves as the foundation of the whole device, which determines the service life of the whole device and resistance to damage by various natural forces or human factors, and the like.; the controlled chamber foundation shall not leak any harmful or polluting substance to the outside environment; the controlled environment is a controllable environment required for growth and development of plants; interior environment includes temperature, humidity, CO2, oxygen, illumination, air composition, microorganism, magnetic field gradient and the like, and these factors are subject to monitoring, analysis, regulation and control by means of corresponding devices; the controlled environment chamber of the device is a layout structure of at least one layer in vertical direction, more layers are laid out in the vertical direction according to different categories of crops and planting needs; wherein in each layer crops are of the same category or interplanted with various crops, in the same layer the same nutrient solution or different nutrient solutions are designed on the basis of different demands, and crops between one layer and another layer are the same crop or different crops;
the nutrition trough is filed with nutrient solution to provide nutrient substances desired for growth and development of plants, roots of a crop are immersed into the nutrient solution to absorb various nutrient substances and moisture desired by the crop if necessary; the shape of the nutrition trough is unlimited and is a circle, a square, a rectangle or an irregular shape on the basis of demands of the crop; according to the quantity and category of the crop in the nutrition trough, the nutrition trough is a nutrition trough for a single-plant crop, a nutrition trough for a trough single-plant crop, or a nutrition trough for multiple mixed crops; the size of the nutrition trough is determined on the basis of situation of the crop; the nutrition trough is made from a plastic material, a resin material, a rubber material, a metal material, synthetic material and the like not reacting chemically with the nutrient solution, thus avoiding the nutrient solution from being polluted, avoiding the nutrition trough from being damaged structurally, avoiding the growth and development of the crop from being affected and avoiding unsafe factors; the nutrition trough is provided with a dynamic information monitor probe for the nutrient solution so as to know in real time the concentration and major constituents of the nutrient solution in the nutrition trough and variation of constituents; data information acquired is sent by the information monitor probe to the system for dynamically monitoring and analyzing information on the nutrient solution instantaneously through the dynamic information transmission line for the nutrient solution; after immediately processing the data information received, the system for dynamically monitoring and analyzing information on nutrient solution develops a corresponding regulation and control scheme, then send the regulation and control scheme to the computer control center through lines for exchanging data, control and information between various systems and the computer control center; afterwards, the computer control center makes an overall analysis on the basis of the whole and individual situations in the device, and send an instruction to the system for dynamically monitoring and analyzing information on the nutrient solution through lines for exchanging data, control and information between various systems and the computer control center; by means of the control instruction, the system for dynamically monitoring and analyzing information on the nutrient solution controls the nutrient solution feed pipeline mounted on the nutrition trough so as to maintain stable concentration and constituents of the nutrient solution, and transfer substances discharged or secreted by the crop to a recovery device through the nutrient solution recovery pipeline for extracting substances discharged or secreted by the crop; many constituents of the nutrient solution are soluble substances, but some substances are insoluble, which affect absorption and utilization of some substances of the crop, thus affecting health, yield and quality of the crop; this problem is solved by following solutions: (1) water for preparing the nutrient solution is processed to improve the solubility of the nutrient solution, thus improve the solubility, or temperature of the nutrient solution is properly increased without affecting growth and development of the crop; (2) some nutrient substances in soil whose main functions that fail to be determined at present are absorbed by the crop, water is mixed with corresponding soil to form a suspension liquid or a turbid solution, which facilitates absorption of certain special nutrient substances by the crop, thus maintaining special characteristics of the crop; in addition, after a magnetic field treatment of the water which constitute the nutrient solution, magnetic water formed increases the solubility of water, its main function is to serve as a hypomagnetic state for assisting an internal magnetic state of the crop and restoring and dredging the internal magnetic state of biomagnetic field pathway so as to maintain the internal magnetic state of biomagnetic field pathway of the crop unblocked;
wherein the crop fixing disk, the main bracket for fixing the crop, the auxiliary bracket for fixing the crop, the transverse bracket for fixing the crop and a transverse bracket for fixing roots of the crop constitute an innovative crop fixing system of the present invention; after being fixed by the fixing system, with the assistance of the fixing system and under the control of the computer control center, the crop spontaneously and selectively absorbs and uses various substances and energy on the basis of growth and development conditions of the crop and its demands for various substances and energy; the crop fixing disk serves as a fixing and supporting device of the whole fixing system, both the main bracket and the auxiliary bracket are fixed to the crop fixing disk, the crop is fixed to the fixing system by means of the main bracket and the auxiliary bracket, the crop moves in accordance with the procedure of the computer control center by means of the power system and the control system; the main bracket for fixing the crop is fixedly mounted on the crop fixing disk and is mainly used for fixing the main stem and main root of the crop and assisting other fixing brackets, the main bracket extends and warps, shaped like a rod, a spiral or an irregular curve and made from lighter materials such as plastic steel, plastics or other materials; the auxiliary bracket for fixing the crop is fixedly mounted on the main bracket for fixing the crop and is mainly used for fixing stems and fruits of the crop, the auxiliary bracket extends and warps, shaped like a rod, a spiral or an irregular curve; the transverse bracket for fixing the crop is fixedly mounted on the main bracket for fixing the crop and is mainly used for fixing stems and fruits of the crop, the transverse bracket extends and warps, shaped like a circular ring, a cruciform, a mesh structure or the like; brackets extend and warp so as to better fix the crop; the transverse bracket for fixing roots of the crop is fixedly mounted on the main bracket for fixing the crop and is mainly used for fixing roots of the crop and assisting roots of the crop to acquire moisture, nutrient substances and energy, shaped like a circular ring, a cruciform, a mesh structure or the like, the bracket extends and warps; after the crop is fixed to the fixing system, the crop moves under the action of the power system of the crop fixing disk, the power system of the crop fixing disk moves under the control of the control system of the crop fixing disk, the control system of the crop fixing disk receives an instruction from the computer control center, after the computer control center makes an overall coordination and analysis, an action instruction required for the crop is transferred to the control system of the crop fixing disk; under the control of the control system of the crop fixing disk, the power system of the crop fixing disk completes voluntary movement of the crop and acquire energy and substances desired by the crop, thus meeting design demands of the innovative device of the present invention;
the gangway between a chamber and another chamber is a gangway for entering from one chamber to another chamber, after a certain stage of growth and development and reproduction or a certain stage of physiological process of the crop is completed in one chamber, the crop is transplanted into another chamber for a next stage of growth and development and reproduction or another stage of physiological process; the gangway door is arranged on the gangway between a chamber and another chamber, the gangway door is opened first when the crop enters from one chamber into another chamber and the gangway door is closed after the crop enters from one chamber into another chamber, the gangway door maintains a relatively independent environment between one chamber and another chamber, which contributes to completing growth and development of the crop at different stages;
the temperature control system, the humidity control system, the CO2 concentration control system, the oxygen component control system, the illumination intensity uniformity control system, the microorganism control system, the magnetic field gradient control system constitute a control segment of the innovative device of the present invention;
the monitor probe for dynamic information of the nutrient solution, the temperature monitoring system, the humidity monitoring system, the CO2 concentration monitoring system, the oxygen component monitoring system, the illumination intensity uniformity monitoring system, the microorganism monitoring system, the magnetic field gradient monitoring system and the air quality monitoring system constitute a monitoring segment of the innovative device of the present invention;
the system for dynamically monitoring and analyzingthe temperature, humidity, CO2, oxygen, illumination, air quality, microorganism and magnetic field gradient or the like, the central control system, the nutrient solution analysis and preparation system, the nutrient solution feed system, the system for dynamically monitoring and analyzing information on the nutrient solution, the nutrient solution recovery system, the system for analyzing constituents of the nutrient solution recycled and extracting effective constituents and the computer control center constitute a central control system of the innovative device of the present invention;
the nutrient solution feed pipeline, the nutrient solution recovery pipeline, the dynamic information transmission line for the nutrient solution, transmission control lines for various control systems, transmission lines for various monitoring systems and lines for exchanging data, control and information between various systems and the computer control center constitute an accessory device of the innovative device of the present invention;
wherein in the temperature control system and the temperature monitoring system, temperature in the environment of the innovative device of the present invention, temperature in the nutrient solution and sensible temperature of the crop are sent by the temperature monitoring system to the temperature control system in real time, after the temperature control system analyzes and sorts temperature information acquired, the temperature information is sent to the computer control center for an overall analysis; according to different demands of plants for temperature at different growth and development stages and different physiological metabolism time, various factors and data are synthesized so as to give an instruction to regulate and control temperature, and the instruction is sent to the temperature control system for regulating and controlling temperature, thus reaching a temperature range most suitable for growth and development and reproduction of the crop;
wherein in the humidity control system and the humidity monitoring system, humidity in the environment of the innovative device of the present invention is sent by the humidity monitoring system to the humidity control system in real time, after the humidity control system analyzes and sorts humidity information acquired, the humidity information is sent to the computer control center for an overall analysis; according to different demands of plants for humidity at different growth and development stages and different physiological metabolism time, various factors, such as humidity, wind velocity, growth and physiological conditions of plants and other data are synthesized so as to give an instruction to regulate and control humidity, and the instruction is sent to the humidity control system for regulating humidity, thus maintaining environment humidity within a range most suitable for plants;
in the CO2 concentration control system and the CO2 concentration monitoring system, the concentration of CO2 in the environment of the innovative device of the present invention is sent by the CO2 concentration monitoring system to the CO2 concentration control system in real time, after the CO2 concentration control system analyzes and sorts CO2 concentration information acquired, the CO2 concentration information is sent to the computer control center for an overall analysis; according to different demands of plants for CO2 concentration at different growth and development stages and different physiological metabolism time, data of growth and development conditions of the crop and data according to the demands for CO2 concentration are synthesized so as to give an instruction to regulate and control CO2 supply, and the instruction is sent to the CO2 concentration control system for controlling CO2 supply from the environment, thus maintaining CO2 concentration in the environment within a range most suitable for plants;
in the oxygen component control system and the oxygen component monitoring system, the concentration of oxygen in the environment of the innovative device of the present invention is sent by the oxygen component monitoring system to the oxygen component control system in real time, after the oxygen component control system analyzes and sorts oxygen concentration information acquired, the oxygen concentration information is sent to the computer control center for an overall analysis; according to different demands of plants for oxygen concentration at different growth and development stages and different physiological metabolism time, data of growth and development conditions of the crop and data according to the demands for oxygen are synthesized so as to give an instruction to regulate and control oxygen concentration, and the instruction is sent to the oxygen component control system for controlling oxygen concentration in the environment, thus maintaining oxygen concentration in the environment within a range most suitable for plants; in the illumination intensity uniformity control system and the illumination intensity uniformity monitoring system, firstly, illumination intensity in the environment of the innovative device of the present invention is sent by the illumination intensity uniformity monitoring system to the illumination intensity uniformity control system in real time, after the illumination intensity uniformity control system analyzes and sorts illumination intensity information acquired, the illumination intensity information is sent to the computer control center for an overall analysis; according to different demands of plants for illumination intensity at different growth and development stages and different physiological metabolism time, data of photosynthesis of the crop and data according to the demands for illumination intensity are synthesized so as to give an instruction to regulate and control illumination intensity, and the instruction is sent to the illumination intensity uniformity control system for controlling illumination intensity in the environment, thus maintaining illumination intensity in the environment within a range most suitable for photosynthesis of plants;
wherein in the microorganism control system and the microorganism monitoring system, microorganisms and pests on the crop in the innovative device of the present invention are detected and damage of the crop resulted from these etiologies is evaluated by the innovative device of the present invention by means of the microorganism monitoring system, the information acquired is sent to the microorganism control system in real time; after the microorganism control system analyzes and sorts the information related to etiologies and to damage of the crop, the information is sent to the computer control center for an overall analysis, a scheme for prevention and treatment of diseases is formulated according to different growth and development stages and different physiological metabolism time of plant, then damage of the crop is targeted and repaired by means of the microorganism control system, thus maintaining healthy growth of the crop and guaranteeing high and stable yield of the crop;
wherein in the magnetic field gradient control system and the magnetic field gradient monitoring system, the internal magnetic state of biomagnetic field pathway of the crop in the innovative device of the present invention is detected whether to be orderly and unblocked by means of the magnetic field gradient monitoring system, also external magnetic state factors affecting the internal magnetic state are detected and analyzed, and then the information is sent to the magnetic field gradient control system in real time, after the magnetic field gradient control system analyzes and sorts the acquired information related to the internal magnetic state and the external magnetic state, the information is sent to the computer control center for an overall analysis, a corresponding restoration and recovery scheme is formulated according to different results of impact of the external magnetic state on the internal magnetic state biomagnetic field of plants, then the impact of the external magnetic state changed by means of the magnetic field gradient control system, and with the assistance of hypomagnetic state, restore and recover the impact on the internal magnetic state of biomagnetic field;
in the internal magnetic state, the external magnetic state and the hypomagnetic state, with regard to a complete life body as a living body such as human body, an animal or a plant and the like related to the health field, ambient environment where the life body exists is referred to as the external magnetic state of the life body, for example, cosmic magnetic field, geomagnetic field and magnetic fields generated by various electric appliances or human factors; a relatively stable magnetic field structure constituting a life body structure in accordance with a certain procedure and a relatively stable magnetic field pathway formed by interaction of magnetic fields are referred to as the internal magnetic state (namely, a complete life body, the structure of which comprises various molecules or atoms by means of interaction of respective magnetic fields in accordance with a certain procedure, the material basis of the life body comprises interaction of magnetic fields under the control of a certain procedure, a relatively stable magnetic field pathway is formed by interaction of magnetic fields; and energy, substances and information of the life body operate by means of the magnetic field pathway), each internal magnetic state of the life body forms a corresponding biomagnetic field respectively, and the biomagnetic field guarantees existence of effective control of the life body at a spirit level; as a relatively independent (or enclosed) biomagnetic field, in order to maintain a relatively stable internal magnetic state structure and a relatively stable unblocked internal magnetic state of magnetic field pathway, the internal magnetic state needs to acquire energy supply from the external magnetic state (external environment) and to exchange substances and information or the like with the external magnetic state (external environment), in addition, the biomagnetic field of the internal magnetic state is affected by the magnetic field of the external magnetic state, i.e., on the basis of maintaining a structure of an intrinsic magnetic field of the internal magnetic state; also action of magnetic field of the external magnetic state shall be complied with;
when the internal magnetic state biomagnetic field of living organisms such as animals and plants (biomagnetic field pathway) is subject to an irreversible (unrecoverable) affect, it is required to recover the affect by virtue of certain approaches or manners, for example, drug therapy, dietary therapy, physical or chemical method therapy; these approaches, manners and therapies that assist the internal magnetic state in self-repair are referred to as the hypomagnetic state, which not only assists some internal magnetic states affected by the magnetic field of the external magnetic state in repairing these internal magnetic states that cannot be repaired by themselves, but also facilitates some internal magnetic states capable of repairing by themselves in self-repair more simply.
